(19) Europäisches Patentamt
European Patent Office
Office européen des brevets

(11) EP 1 549 771 B1

# (12) EUROPEAN PATENT SPECIFICATION

(45) Date of publication and mention of the grant of the patent:
**31.12.2008 Bulletin 2009/01**

(21) Application number: **03799106.4**

(22) Date of filing: **17.09.2003**

(51) Int Cl.:
***C12Q 1/68*** *(2006.01)*

(86) International application number:
**PCT/JP2003/011817**

(87) International publication number:
**WO 2004/031412 (15.04.2004 Gazette 2004/16)**

## (54) METHOD FOR DIAGNOSING PANCREATIC CANCER

VERFAHREN ZUR DIAGNOSE VON BAUCHSPEICHELDRÜSENKREBS

METHODE DE DIAGNOSTIC DU CANCER DU PANCREAS

(84) Designated Contracting States:
**AT BE BG CH CY CZ DE DK EE ES FI FR GB GR HU IE IT LI LU MC NL PT RO SE SI SK TR**

(30) Priority: **30.09.2002 US 414872 P**
**28.02.2003 US 450889 P**

(43) Date of publication of application:
**06.07.2005 Bulletin 2005/27**

(60) Divisional application:
**08014535.2**

(73) Proprietor: **Oncotherapy Science, Inc.**
**Kawasaki-shi, Kanagawa 213-0012 (JP)**

(72) Inventors:
- **NAKAMURA, Yusuke**
**Yokohama-shi, Kanagawa 225-0011 (JP)**
- **KATAGIRI, Toyomasa**
**Tokyo 141-0022 (JP)**

(74) Representative: **Vossius & Partner**
**Siebertstrasse 4**
**81675 München (DE)**

(56) References cited:

| | |
|---|---|
| **EP-A- 1 241 269** | **WO-A-00/55350** |
| **WO-A-01/57270** | **WO-A-01/94629** |
| **WO-A-02/29103** | **WO-A-98/53319** |
| **WO-A-99/31274** | **WO-A-99/67386** |
| **WO-A-02/060317** | **WO-A-03/065873** |
| **WO-A-20/04005883** | **WO-A-20/07102526** |
| **US-B1- 6 335 170** | **US-B1- 6 429 302** |

- **GRESS TM ET AL: "A PANCREATIC CANCER-SPECIFIC EXPRESSION PROFILE" ONCOGENE, BASINGSTOKE, HANTS, GB, vol. 13, 1996, pages 1819-1830, XP002916550 ISSN: 0950-9232**
- **DATABASE BIOSIS [Online] BIOSCIENCES INFORMATION SERVICE, PHILADELPHIA, PA, US; 1990, SILVERMAN J A ET AL: "EXPRESSION OF C-MYC C-RAF-1 AND C-KI-RAS IN AZASERINE-INDUCED PANCREATIC CARCINOMAS AND GROWING PANCREAS IN RATS" XP002267298 Database accession no. PREV199191074872 & MOLECULAR CARCINOGENESIS, vol. 3, no. 6, 1990, pages 379-386, ISSN: 0899-1987**
- **CRNOGORAC-JURCEVIC TATJANA ET AL: "Expression profiling of microdissected pancreatic adenocarcinomas" ONCOGENE, vol. 21, no. 29, 4 July 2002 (2002-07-04), pages 4587-4594, XP002267283 ISSN: 0950-9232**
- **CRNOGORAC-JURCEVIC TATJANA ET AL: "Gene expression profiles of pancreatic cancer and stromal desmoplasia" ONCOGENE, vol. 20, no. 50, 1 November 2001 (2001-11-01), pages 7437-7446, XP002267284 ISSN: 0950-9232**
- **LACOBUZIO-DONAHUE C A ET AL: "DISCOVERY OF NOVEL TUMOR MARKERS OF PANCREATIC CANCER USING GLOBAL GENE EXPRESSION TECHNOLOGY" AMERICAN JOURNAL OF PATHOLOGY, PHILADELPHIA, PA, US, vol. 160, no. 4, April 2002 (2002-04), pages 1239-1249, XP008003747 ISSN: 0002-9440**
- **HAN HAIYONG ET AL: "Identification of differentially expressed genes in pancreatic cancer cells using cDNA microarray" CANCER RESEARCH, vol. 62, no. 10, 15 May 2002 (2002-05-15), pages 2890-2896, XP002267297 ISSN: 0008-5472**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 549 771 B1

- **ZHANG L ET AL: "Gene expression profiles in normal and cancer cells" SCIENCE, AMERICAN ASSOCIATION FOR THE ADVANCEMENT OF SCIENCE,, US, vol. 276, 23 May 1997 (1997-05-23), pages 1268-1272, XP002083785 ISSN: 0036-8075**
- **RYU BYUNGWOO ET AL: "Relationships and differentially expressed genes among pancreatic cancers examined by large-scale serial analysis of gene expression" CANCER RESEARCH, AMERICAN ASSOCIATION FOR CANCER RESEARCH, BALTIMORE, MD, US, vol. 62, no. 3, 1 February 2002 (2002-02-01), pages 819-826, XP002251739 ISSN: 0008-5472**
- **JORDAN M A ET AL: "MICROTUBULES AND ACTIN FILAMENTS: DYNAMIC TARGETS FOR CANCER CHEMOTHERAPY" CURRENT OPINION IN CELL BIOLOGY, CURRENT SCIENCE, LONDON, GB, vol. 10, no. 1, 1998, pages 123-130, XP000939151 ISSN: 0955-0674**
- **Patent application JP2007/065873**


Remarks:

The file contains technical information submitted after the application was filed and not included in this specification

## Description

### PRIORITY INFORMATION

This application claims priority to United States Provisional Application Serial No.60/414,872, filed September 30, 2002, and Serial No.60/450,889, filed February 28, 2003.

### TECHNICAL FIELD

The invention relates to methods of diagnosing pancreatic ductal adenocarcinoma.

### BACKGROUND OF THE INVENTION

Pancreatic cancer has one of the highest mortality rates of any malignancy, and the 5-year-survival rate of patients is 4%. 28000 patients with pancreatic cancer are diagnosed each year, and nearly all patients will die of their disease (1). The poor prognosis of this malignancy is a result of the difficulty of early diagnosis and poor response to current therapeutic methods (1, 2). In particular currently no tumor markers are identified that allow reliable screening at an early, potentially curative stage of the disease.

cDNA microarray technologies have enabled to obtain comprehensive profiles of gene expression in normal and malignant cells, and compare the gene expression in malignant and corresponding normal cells (Okabe et al., Cancer Res 61:2129-37 (2001); Kitahara et al., Cancer Res 61: 3544-9 (2001); Lin et al., Oncogene 21:4120-8 (2002); Hasegawa et al., Cancer Res 62:7412-7 (2002)): This approach enables to disclose the complex nature of cancer cells, and helps to understand the mechanism of carcinogenesis. Identification of genes that are deregulated in tumors can lead to more precise and accurate diagnosis of individual cancers, and to develop novel therapeutic targets (Bienz and Clevers, Cell 103:311-20 (2000)). To disclose mechanisms underlying tumors from a genome-wide point of view, and discover target molecules for diagnosis and development of novel therapeutic drugs, the present inventors have been analyzing the expression profiles of tumor cells using a cDNA microarray of 23040 genes (Okabe et al., Cancer Res 61: 2129-37 (2001); Kitahara et al., Cancer Res 61:3544-9 (2001); Lin et al., Oncogene 21:4120-8 (2002); Hasegawa et al., Cancer Res 62:7012-7 (2002)).

Studies designed to reveal mechanisms of carcinogenesis have already facilitated identification of molecular targets for anti-tumor agents. For example, inhibitors of farnexyltransferase (FTIs) which were originally developed to inhibit the growth-signaling pathway related to Ras, whose activation depends on posttranslational farnesylation, has been effective in treating Ras-dependent tumors in animal models (He et al., Cell 99:335-45 (1999)). Clinical trials on human using a combination or anti-cancer drugs and anti-HER2 monoclonal antibody, trastuzumab, have been conducted to antagonize the proto-oncogene receptor HER2/neu; and have been achieving improved clinical response and overall survival of breast-cancer patients (Lin et al., Cancer Res 61:6345-9 (2001)). A tyrosine kinase inhibitor, STI-571, which selectively inactivates bcr-abl fusion proteins, has been developed to treat chronic myelogenous leukemias wherein constitutive activation of bcr-abl tyrosine kinase plays a crucial role in the transformation of leukocytes. Agents of these kinds are designed to suppress oncogenic activity of specific gene products (Fujita et al., Cancer Res 61:7722-6 (2001)). Therefore, gene products commonly up-regulated in cancerous cells may serve as potential targets for developing novel anti-cancer agents.

It has been demonstrated that CD8+ cytotoxic T lymphocytes (CTLs) recognize epitope peptides derived from tumor-associated antigens (TAAs) presented on MHC Class I molecule, and lyse tumor cells. Since the discovery of MAGE family as the first example of TAAs, many other TAAs have been discovered using immunological approaches (Boon, Int J Cancer 54: 177-80 (1993); Boon and van der Bruggen, J Exp Med 183: 725-9 (1996); van der Bruggen et al., Science 254: 1643-7 (1991); Brichard et al., J Exp Med 178: 489-95 (1993); Kawakami et al., J Exp Med 180: 347-52 (1994)). Some of the discovered TAAs are now in the stage of clinical development as targets of immunotherapy. TAAs discovered so far include MAGE (van der Bruggen et al., Science 254: 1643-7 (1991)), gp100 (Kawakami et al., J Exp Med 180: 347-52 (1994)), SART (Shichijo et al., J Exp Med 187: 277-88 (1998)), and NY-ESO-1 (Chen et al., Proc Natl Acad Sci USA 94:1914-8 (1997)). On the other hand, gene products which had been demonstrated to be specifically overexpressed in tumor cells, have been shown to be recognized as targets inducing cellular immune responses. Such gene products include p53 (Umano et al., Brit J Cancer 84: 1052-7 (2001)), HER2/neu (Tanaka et al., Brit J Cancer 84: 94-9 (2001)), CEA (Nukaya et al., Int J Cancer 80: 92-7 (1999)), and so on.

In spite of significant progress in basic and clinical research concerning TAAs (Rosenbeg et al., Nature Med 4: 321-7 (1998); Mukherji et al., Proc Natl Acad Sci USA 92: 8078-82 (1995); Hu et al., Cancer Res 56: 2479-83 (1996)), only limited number of candidate TAAs for the treatment of adenocarcinomas, including colorectal cancer, are available. TAAs abundantly expressed in cancer cells, and at the same time which expression is restricted to cancer cells would be promising candidates as immunotherapeutic targets. Further, identification of new TAAs inducing potent and specific antitumor immune responses is expected to encourage clinical use of peptide vaccination strategy in various types of cancer (Boon and can der Bruggen, J Exp Med 183: 725-9 (1996); van der Bruggen et al., Science 254: 1643-7 (1991); Brichard et al., J Exp Med 178: 489-95 (1993); Kawakami et al.,J Exp Med 180: 347-52 (1994); Shichijo et al., J Exp Med 187: 277-88 (1998); Chen et al., Proc Natl Acad Sci USA 94: 1914-8 (1997); Harries, J Natl Cancer Inst 88: 1442-5 (1996); Butterfield et al., Cancer Res 59: 3134-42 (1999); Vissers et al., Cancer Res 59: 5554-9 (1999); van der Burg et al., J Immunol 156: 3308-14 (1996); Tanaka et al., Cancer Res 57: 4465-8 (1997); Fujie et al., Int J Cancer 80: 169-72 (1999); Kikuchi et al., Int J Cancer 81: 459-66 (1999); Oiso et al., Int J Cancer 81: 387-94 (1999)).

It has been repeatedly reported that peptide-stimulated peripheral blood mononuclear cells (PBMCs) from certain healthy donors produce significant levels of IFN-γ in response to the peptide, but rarely exert cytotoxicity against tumor cells in an HLA-A24 or A0201 restricted manner in $^{51}$Cr-release assays (Kawano et al., Cance Res 60: 3550-8 (2000); Nishizaka et al., Cancer Res 60: 4830-7 (2000); Tamura et al., Jpn J Cancer Res 92: 762-7 (2001)). However, both of HLA-A24 and HLA-A0201 are one of the popular HLA alleles in Japanese, as well as Caucasian (Date et al., Tissue Antigens 47: 93-101 (1996); Kondo et al., J Immunol 155: 4307-12 (1995); Kubo et al., J Immunol 152: 3913-24 (1994); Imanishi et al., Proceeding of the eleventh International Hictocompatibility Workshop and Conference Oxford University Press, Oxford, 1065 (1992); Williams et al., Tissue Antigen 49: 129 (1997)). Thus, antigenic peptides of carcinomas presented by these HLAs may be especially useful for the treatment of carcinomas among Japanese and Caucasian. Further, it is known that the induction of low-affinity CTL in vitro usually results from the use of peptide at a high concentration, generating a high level of specific peptide/MHC complexes on antigen presenting cells (APCs), which will effectively activate these CTL (Alexander-Miller et al., Proc Natl Acad Sci USA 93: 4102-7 (1996)).

EP1241269 describes methods of diagnosis or prognosis of cancer based on detecting the expression levels of PNC157, also known as RELP, and kits to be used in said methods (see paragraphs 9-36; Examples 4-5; claims 1-7). Pancreatic tumors are one of the preferred options (see paragraphs 24-25).

WO02/060317 describes methods for diagnosis of pancreatic cancer based on detecting expression levels of PNC157, as well as detection kits for use in said methods and pharmaceutical compositions (SEQ ID NOs: 3587, 3702, 3066, 2894, 3151, 3426, 2624; claims 1-17; pages 245-254; Example 6; pages 4, 194, 224).

## SUMMARY OF THE INVENTION

The invention is based on the discovery of a pattern of gene expression correlated with pancreatic cancer (PNC). Specifically, the invention is based on the finding of the gene expression pattern of the PNC-associated gene PNC 157, also known as REGIV, which is correlated with PNC. The genes that are differentially expressed in pancreatic cancer are collectively referred to herein as "PNC nucleic acids" or "PNC polynucleotides" and the corresponding encoded polypeptides are referred to as "PNC polypeptides" or "PNC proteins."

Accordingly, the invention features a method of diagnosing pancreatic ductal adenocarcinoma in a subject by determining the expression level of the PNC-associated gene PNC 157(REGIV) in a patient derived biological sample comprising a pancreatic ductal epithelial cell. By PNC-associated gene is meant a gene that is characterized by an expression level which differs in a cell obtained from a PNC cell compared to a normal cell. A normal cell is one obtained from pancreas tissue. A PNC-associated gene is one or more of PNC 1-605. The PNC-associated gene of the invention is PNC 157. An alteration, *e.g.*, increase or decrease of the level of expression of the gene compared to a normal control level of the gene indicates that the subject suffers from or is at risk of developing PNC. The expression of the PNC 157 gene of the present invention is increased compared to a normal control level of the gene.

By normal control level is meant a level of gene expression detected in a normal, healthy individual or in a population of individuals known not to be suffering from pancreatic cancer. A control level is a single expression pattern derived from a single reference population or from a plurality of expression patterns. For example, the control level can be a database of expression patterns from previously tested cells. A normal individual is one with no clinical symptoms of pancreatic cancer.

An increase in the level of PNC 1-259, in particular of PNC 157, detected in a test sample compared to a normal control level indicates the subject (from which the sample was obtained) suffers from or is at risk of developing PNC. In contrast, a decrease in the level of PNC 260-605, which are described herein, detected in a test sample compared to a normal control level indicates said subject suffers from or is at risk of developing PNC.

Alternatively, expression of a panel of PNC-associated genes in the sample is compared to a PNC control level of the same panel of genes. By PNC control level is meant the expression profile of the PNC-associated genes found in a population suffering from PNC.

Gene expression is increased or decreased 10%, 25%, 50% compared to the control level. Alternately, gene expression is increased or decreased 1, 2, 5 or more fold compared to the control level. Expression is determined by detecting hybridization, *e.g.*, on an array, of a PNC-associated gene probe to a gene transcript of the patient-derived tissue sample.

The patient derived tissue sample is any tissue from a test subject, e.g., a patient known to or suspected of having PNC., comprising a pancreatic ductal epithelial cell. For example, the tissue is an epithelial cell from a pancreatic ductal adenocarcinoma.

Herein disclosed is also a PNC reference expression profile of a gene expression level of two or more of PNC 1-605. Alternatively, herein described is a PNC reference expression profile of the levels of expression two or more of PNC 1-259 or PNC 260-605. Further described herein are methods of identifing an agent that inhibits or enhances the expression or activity of a PNC-associated gene, e.g. PNC 1-605 by contacting a test cell expressing a PNC-associated gene with a test agent and determining the expression level of the PNC associated gene. The test cell is a epithelial cell such as an epithelial cell from a pancreatic adenocarcinoma. A decrease of the level compared to a normal control level of the gene indicates that the test agent is an inhibitor of the PNC-associated gene and reduces a symptom of PNC, e.g. PNC 1-259, in particular of PNC 157. Alternatively, an increase of the level or activity compared to a normal control level or activity of the gene indicates that said test agent is an enhancer of expression or function of the PNC-associated gene and reduces a symptom of PNC, e.g, PNC 260-605.

Moreover, herein described is a kit with a detection reagent which binds to one or more PNC nucleic acids or which binds to a gene product encoded by the nucleic acid sequences. Also described is an array of nucleic acids that binds to one or more PNC nucleic acids. Therapeutic methods of the invention include a composition for use in a method of treating or preventing pancreatic ductal adenocarcinoma cancer in a subject by administering to the subject an antisense composition. The antisense composition reduces the expression of a specific target gene, e.g., the antisense composition contains a nucleotide, which is complementary to a PNC 157 sequence. Another composition for use in a method includes the steps of administering to a subject an short interfering RNA (siRNA) composition. The siRNA composition reduces the expression of a PNC 157 nucleic acid.

By another composition for use in a method, treatment or prevention of PNC in a subject is carried out by administering to a subject a ribozyme composition. The nucleic acid-specific ribozyme composition reduces the expression of a PNC 157 nucleic acid.

Herein described are also vaccines and vaccination methods. For example, a composition for use in a method of treating or preventing PNC in a subject is carried out by administering to the subject a vaccine containing a polypeptide encoded by a nucleic acid selected from the group consisting of PNC 1-259, in particular PNC 157, or an immunologically active fragment such a polypeptide. An immunologically active fragment is a polypeptide that is shorter in length than the full-length naturally-occurring protein and which induces an immune response. For example, an immunologically active fragment at least 8 residues in length and stimulates an immune cell such as a T cell or a B cell. Immune cell stimulation is measured by detecting cell proliferation, elaboration of cytokines (e.g., IL-2), or production of an antibody.

Also described herein are target molecules for treating or preventing malignant pancreatic cancer. 76 (PNC 606-681), 168 (PNC 682-849) and 84 (850-933) genes were identified as genes that showed unique altered expression patterns in pancreatic cancer cells with lymph-node metastasis, liver metastasis and early recurrence, respectively. Thus, malignant pancreatic cancer can be treated or prevented via the suppression of the expression or activity of up-regulated genes selected from the group consisting of PNC 606-640 and PNC 682-741. Furthermore, recurrence of pancreatic cancer can be treated or prevented via the suppression of the expression or activity of up-regulated genes selected from the group consisting of PNC 850-893. Moreover, malignant pancreatic cancer can also be treated or prevented through enhancing the expression or activity of down-regulating genes in cancerous cells.

Herein described are methods for predicting recurrence of pancreatic cancer. The method comprises the step of measuring the expression level of marker genes selected from the group consisting of PNC 850-879. The marker genes were identified as genes that show unique altered expression patterns in pancreatic cancer cells of patients with recurrence within 12 month after surgery. Therefore, recurrence of the pancreatic cancer in a subject can be predicted by determining whether the expression level detected in a sample derived from the subject is closer to the mean expression level of early-recurrent cases or late-recurrent cases in reference samples.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the present specification, including definitions, will control. In addition, the materials, methods, and examples are illustrative only and not intended to be limiting.

One advantage of the methods described herein is that the disease is identified prior to detection of overt clinical symptoms of pancreatic cancer. Other features and advantages of the invention will be apparent from the following detailed description, and from the claims.

## BRIEF DESCRIPTION OF THE FIGURES

Figure 1A is a photograph of a hematoxylin and eosin stained pancreatic cancer (well-differentiated type) before microdissection. 1A1 is the same sections after microdissection. 1A2 is a photograph of the microdissected cancer cells captured on the collecting cap.

Figure 1B is a photograph of a hematoxylin and eosin stained pancreatic cancer (scirrhous type) before microdissection. 1B1 is the same sections after microdissection. 1B2 is a photograph of the microdissected cancer cells captured on the collecting cap.

Figure 1C is a photograph of normal pancreas containing greater than 90% acinar cell.

Figure 1D is a photograph of microdissected normall pancreatic ductal epithial cells.

Figure 2 is a photograph of a DNA agarose gel showing expression of representative 12 genes and TUBA examined by semi-quantitative RT-PCR using cDNA prepared from amplified RNA. Lanes 1-12 each show the expression level of the genes in a different PNC patient: Gene symbols are noted for the genes. The last lane shows the expression level of each gene in a normal individual.

Figure 3 Dendrogram of two-dimensional hierarchical clustering analysis using 76 genes selected by a random-permutation test which compared expression profiles of 9 lymph-node positive cases with those of 4 lymph-node negative cases. In the vertical axis, 35 genes were clustered in the upper branch, indicating relatively high levels of expression in lymph-node positive cases.

Figure 4 Dendrogram of two-dimensional hierarchical clustering analysis using 168 genes selected by a random-permutation test which compared expression profiles of 5 liver-metastasis-positive cases with those of 6 negative cases. In the vertical axis, 60 genes were clustered in the upper branch which was more highly expressed in liver-metastasis-positive cases.

Figure 5(A) Result of a two-dimensional hierarchical clustering analysis using 84 genes selected by a random-permutation test which compared expression profiles of 7 early-recurrent cases (within 12months after surgery) with those of 6 late-recurrent cases (over 12 months after surgery). In the vertical axis, 84 genes were clustered in different branches according to similarity in relative expression ratios. (B) Optimization of the number of discriminating genes. The classification score (CS) was calculated by using the prediction score of early-recurrent case (PSr) and late-recurrent case (PSn) in each gene set, as follows. CS= $(\mu_{PSr} - \mu_{PSn}) / (\sigma_{PSr} + \sigma_{PSn})$. A larger value of CS indicates better separation of the two groups by the predictive-scoring system. (C) Different prediction scores appear when the number of discriminating genes is changed. Red diamonds represent early-recurrent cases; blue diamonds denote late-recurrent cases.

## DETAILED DESCRIPTION

Generally pancreatic ductal adenocarcinoma has a characteristic of highly desmoplastic stromal reaction, only a low percentage (about 30%) of cancer cells are contained in the tumor mass. Furthermore, normal pancreatic ductal epithelial cells, which recently considered to be the normal counterpart of the pancreatic adenocarcinoma, occupied only less than 5% of the total population of cells composing the organ 'pancreas' (7, 8). Hence, the gene-expression analysis of PNC compared to normal pancreas by using whole tissue is distorted by the contamination of needless cells such as fibroblast, inflammatory cells, acinar cells, etc., and results in "noisy data". Therefore Laser capture microdissection (LCM), or Laser microbeam microdissection (LMM), a method for isolating pure cell populations, was used to obtain specific cancer cells and normal epithelial cells (9,10).

The present invention is based in part on the discovery of changes in expression patterns of multiple nucleic acids, in particular of a PNC 157 nucleic acid, in epithelial cells from adenocarcinomas of patients with PNC. The differences in gene expression were identified by using a comprehensive cDNA microarray system.

The gene-expression profiles of cancer cells from 18 PNCs were analyzed using cDNA microarray representing 23,040 genes couples with laser microdissection. By comparing expression patterns between cancer cells from diagnostic PNC patients and normal ductal epithelial cells purely selected with Laser Microdisection, 259 genes were identified as commonly up-regulated in PNC cells, and 346 genes were identified as being commonly down-regulated in PNC cells. In addition, selection was made of candidate molecular markers with the potential of detecting cancer-related proteins in serum or sputum of patients, and discovered some potential targets for development of signal-suppressing strategies in human PNC.

The differentially expressed genes, in particular the PNC 157 gene, identified herein are used for diagnostic purposes as markers of PNC and as gene targets, the expression of which is altered to treat or alleviate a symptom of PNC.

The genes whose expression levels are modulated (*i.e.*, increased or decreased) in PNC patients are summarized in Tables 3-4 and are collectively referred to herein as " PNC-associated genes" , "PNC nucleic acids" or "PNC polynucleotides" and the corresponding encoded polypeptides are referred to as "PNC polypeptides" or "PNC proteins." Unless indicated otherwise, "PNC" is meant to refer to any of the sequences disclosed herein. (*e.g.*, PNC 1-605). The genes have been previously described and are presented along with a database accession number.

By measuring expression of the various genes in a sample of cells, PNC is diagnosed. Similarly, measuring the expression of these genes in response to various agents can identify agents for treating PNC.

The invention involves determining (*e.g.*, measuring) the expression of the PNC 157 sequence listed in Tables 3-4. Also described herein is the determination of the expression of the PNC sequences also listed in Tables 3-4. Using sequence information provided by the GeneBank™ database entries for the known sequences the PNC-associated genes are detected and measured using techniques well known to one of ordinary skill in the art. For example, sequences within the sequence database entries corresponding to PNC sequences, are used to construct probes for detecting PNC RNA sequences in, *e.g.*, northern blot hybridization analysis. Probes include at least 10, 20, 50, 100, 200 nucleotides of a reference sequence. As another example, the sequences can be used to construct primers for specifically amplifying the PNC nucleic acid in, *e.g*, amplification-based detection methods such as reverse-transcription based polymerase chain reaction.

Expression level of one or more of the PNC-associated genes, in particular of the PNC-associated gene PNC 157, in the test cell population, *e.g.*, a patient derived tissues sample, is then compared to expression levels of the some genes in a reference population. The reference cell population includes one or more cells for which the compared parameter is known, *i.e.*, pancreatic ductal adenocarcinoma cells or normal pancreatic ductal epithelial cells.

Whether or not a pattern of gene expression levels in the test cell population compared to the reference cell population indicates PNC or predisposition thereto depends upon the composition of the reference cell population. For example, if the reference cell population is composed of non-PNC cells, a similar gene expression pattern in the test cell population and reference cell population indicates the test cell population is non-PNC. Conversely, if the reference cell population is made up of PNC cells, a similar gene expression profile between the test cell population and the reference cell population indicates that the test cell population includes PNC cells.

A level of expression of a PNC marker gene, in particular of the PNC 157 gene, in a test cell population is considered altered in levels of expression if its expression level varies from the reference cell population by more than 1.0, 1.5, 2.0, 5.0, 10.0 or more fold from the expression level of the corresponding PNC marker gene in the reference cell population.

Differential gene expression between a test cell population and a reference cell population is normalized to a control nucleic acid, *e.g.* a housekeeping gene. For example, a control nucleic acid is one which is known not to differ depending on the cancerous or non-cancerous state of the cell. Expression levels of the control nucleic acid in the test and reference nucleic acid can be used to normalize signal levels in the compared populations. Control genes include, *e.g*,. β-actin, glyceraldehyde 3- phosphate dehydrogenase or ribosomal protein P1.

The test cell population is compared to multiple reference cell populations. Each of the multiple reference populations may differ in the known parameter. Thus, a test cell population may be compared to a second reference cell population known to contain, *e.g.*, PNC cells, as well as a second reference population known to contain, *e.g.*, non-PNC cells (normal cells). The test cell is included in a tissue type or cell sample from a subject known to contain, or to be suspected of containing, PNC cells.

The test cell is obtained from a bodily tissue or a bodily fluid, *e.g.*, biological fluid (such as blood or sputum). For example, the test cell is purified from pancreas tissue. Preferably, the test cell population comprises an epithelial cell. The epithelial cell is from tissue known to be or suspected to be a pancreatic ductal adenocarcinoma.

Cells in the reference cell population are derived from a tissue type as similar to test cell. Optionally, the reference cell population is a cell line, *e.g.* a PNC cell line (positive control) or a normal non-PNC cell line (negative control). Alternatively, the control cell population is derived from a database of molecular information derived from cells for which the assayed parameter or condition is known.

The subject is preferably a mammal. The mammal can be, *e.g.*, a human, non-human primate, mouse, rat, dog, cat, horse, or cow.

Expression of the genes, in particular of the PNC 157 gene, disclosed herein is determined at the protein or nucleic acid level using methods known in the art. For example, Northern hybridization analysis using probes which specifically recognize one or more of these nucleic acid sequences can be used to determine gene expression. Alternatively, expression is measured using reverse-transcription-based PCR assays, *e.g.*, using primers specific for the differentially expressed gene sequences. Expression is also determined at the protein level, *i.e.*, by measuring the levels of polypeptides encoded by the gene products described herein, or biological activity thereof. Such methods are well known in the art and include, e.g., immunoassays based on antibodies to proteins encoded by the genes. The biological activity of the proteins encoded by the genes are also well known.

*Diagnosing pancreatic cancer*

PNC is diagnosed by measuring the level of expression of one or more PNC nucleic acid sequences from a test population of cells, (*i.e.*, a patient derived biological sample), in particular by measuring the level of expression of the PNC 157 nucleic acid sequence. Preferably, the test cell population contains an epithelial cell, e.g., a cell obtained from pancreas tissue. Gene expression is also measured from blood or other bodily fluids such as urine. Other biological samples can be used for measuring the protein level. For example, the protein level in the blood, serum, or pancreatic juice derived from subject to be diagnosed can be measured by immunoassay or biological assay.

Expression of one or more PNC-associated genes, *e.g.*, PNC 1-605, in particular of PNC 157, is determined in the test cell or biological sample and compared to the expression of the normal control level. A normal control level is an expression profile of a PNC-associated gene typically found in a population known not to be suffering from PNC. An increase or a decrease of the level of expression in the patient derived tissue sample of the PNC-associated genes indicates that the subject is suffering from or is at risk of developing PNC. For example, an increase in expression of PNC 1-259, in particular of PNC 157, in the test population compared to the normal control level indicates that the subject is suffering from or is at risk of developing PNC. Conversely, a decrease in expression of PNC 260-605 in the test population compared to the normal control level indicates that the subject is suffering from or is at risk of developing PNC.

When one or more of the PNC-associated genes, in particular the PNC-associated gene PNC 157, are altered in the test population compared to the normal control level indicates that the subject suffers from or is at risk of developing PNC. For example, at least 1%, 5%, 25%, 50%, 60%, 80%, 90% or more of the panel of PNC-associated genes (PNC 1-259, PNC 260-605, or PNC 1-605, in particular PNC 157) are altered.

*Predicting prognosis of PNC*

Herein described is a method for predicting prognosis of PNC in a subject, the method comprising the steps of:

(a) detecting an expression level of one or more marker genes in a specimen collected from a subject to be predicted, wherein the one or more marker genes are selected from the group consisting of PNC 850-866, 894-906; and

(b) comparing the expression level of the one or more marker genes to that of a early recurrence cases and late recurrence cases; and

(c) when the expression level of one or marker genes is close to that of the early recurrence case, determining the subject to be at a risk of having recurrence of PNC and when the expression level of one or marker genes is close to that of the late recurrence case, determining the risk of the subject of having recurrence of PNC to be low.

Marker gene(s) for prediction of prognosis of PNC may be at least one gene selected from the group consisting of PNC 850-933 ; 84 genes shown in Table 8. The nucleotide sequences of the genes and amino acid sequences encoded thereby are known in the art. See Table 8 for the Accession Numbers of the genes.
Prediction of prognosis comprises prediction of probability for recurrence of PNC. When recurrence of PNC is observed within 12 month after surgery, the subject is determined to have poor prognosis. Accordingly, the expression levels of multiple marker genes selected from the group of PNC 850-866, 894-906 can be measured for the prediction. Preferably, the 30 genes consisting of top 17 genes (ARGBP2, CBARA1, EEF1G, LCAT, RPL23A, RPL17, ATP1A1, QARS, BZRP, TUFM, SERPINA4, SCAP, HK1, RPS11, SYNGR2, FLOT2, PSMB4) of up-regulated in late recurrence cases genes and top 13 genes of up-regulated in early recurrence cases genes (MTMR1, HT010, NPD002, YME1L1, CCT6A, HSPD1, TIMM9, GRB14, FLJ 10803, LAMP1, MLLT4, CTSB, RALY) of Table 8 are useful for the prediction. In the present method, the specimen is collected from a subject. Preferable specimen include pancreatic tissue derived from patient of pancreatic cancer. Methods for measuring the expression level of marker genes are well-known in the art. For example, DNA array is useful for measuring the expression level of multiple marker genes. Accordingly, first, the expression level of each marker genes in a specimen is measured and then compared to that of early recurrence cases and late recurrence cases. The expression level of the marker genes of each of the cases can be measured prior to the comparison of the expression level. Then, based on the above comparison, when the expression level of one or marker genes is close to that of the early recurrence case, determining the subject to be at a risk of having recurrence of PNC and when the expression level of one or marker genes is close to that of the late recurrence case, determining the risk of the subject of having recurrence of PNC to be low. The recurrence of PNC can be predicted using prediction score that may be calculated by statistical methods. Methods for calculating prediction score is well-known in the art (T.R. Golub et al., Science 286, 531-7, 1999; T.J. MacDonald et al., Nat. Genet, 29, 143-52, 2001). Furthermore, prediction of recurrence using prediction score in the present invention may be also performed according to the method disclosed in the Example.

*Identifying Agents that inhibit or enhance PNC-associated gene expression*

An agent that inhibits the expression or activity of a PNC-associated gene, in particular of the PNC-associated PNC 157 gene, is identified by contacting a test cell population expressing a PNC-associated up-regulated gene with a test agent and determining the expression level of the PNC-associated gene. A decrease in expression in the presence of the agent compared to the normal control level (or compared to the level in the absence of the test agent) indicates the agent is an inhibitor of a PNC-associated up-regulated gene and useful to inhibit PNC.

Alternatively, an agent that enhances the expression or activity of a PNC-associated down-regulated gene is identified by contacting a test cell population expressing a PNC-associated gene with a test agent and determining the expression level or activity of the PNC-associated down-regulated gene. An increase of expression or activity compared to a normal control expression level or activity of the PNC-associated gene indicates that the test agent augments expression or activity of the PNC-associated down-regulated gene.

The test cell population is any cell expressing the PNC-associated genes. For example, the test cell population contains an epithelial cell, such as a cell is or derived from pancreas tissue. For example, the test cell is an immortalized cell line derived from an adenocarcinoma cell. Alternatively, the test cell is a cell, which has been transfected with a PNC-associated gene or which has been transfected with a regulatory sequence (*e.g.* promoter sequence) from a PNC-associated gene operably linked to a reporter gene.

*Assessing efficacy of treatment of PNC in a subject*

The differentially expressed PNC-associated gene, in particular the PNC-associated PNC 157 gene, identified herein also allow for the course of treatment of PNC to be monitored. In this method, a test cell population is provided from a subject undergoing treatment for PNC. If desired, test cell populations are obtained from the subject at various time points before, during, or after treatment. Expression of one or more of the PNC-associated gene, in the cell population is then determined and compared to a reference cell population which includes cells whose PNC state is known. The reference cells have not been exposed to the treatment.

If the reference cell population contains no PNC cells, a similarity in expression between PNC-associated gene in the test cell population and the reference cell population indicates that the treatment is efficacious. However, a difference in expression between PNC-associated gene in the test population and a normal control reference cell population indicates a less favorable clinical outcome or prognosis.

By "efficacious" is meant that the treatment leads to a reduction in expression of a pathologically up-regulated gene, increase in expression of a pathologically down-regulated gene or a decrease in size, prevalence, or metastatic potential of pancreatic ductal adenocarcinoma in a subject. When treatment is applied prophylactically, "efficacious" means that the treatment retards or prevents a pancreatic tumor from forming or retards, prevents, or alleviates a symptom of clinical PNC. Assessment of pancreatic tumors is made using standard clinical protocols.
Efficaciousness is determined in association with any known method for diagnosing or treating PNC. PNC is diagnosed for example, by identifying symptomatic anomalies, *e.g.*, weight loss, abdominal pain, back pain, anorexia, nausea, vomiting and generalized malaise, weakness, and jaundice.

*Selecting a therapeutic agent for treating PNC that is appropriate for a particular individual*

Differences in the genetic makeup of individuals can result in differences in their relative abilities to metabolize various drugs. An agent that is metabolized in a subject to act as an anti-PNC agent can manifest itself by inducing a change in gene expression pattern in the subject's cells from that characteristic of a cancerous state to a gene expression pattern characteristic of a non-cancerous state. Accordingly, the differentially expressed PNC-associated gene, in particular the PNC-associated PNC 157 gene, disclosed herein allow for a putative therapeutic or prophylactic inhibitor of PNC to be tested in a test cell population from a selected subject in order to determine if the agent is a suitable inhibitor of PNC in the subject.
To identify an inhibitor of PNC, that is appropriate for a specific subject, a test cell population from the subject is exposed to a therapeutic agent, and the expression of one or more of PNC 1-605 genes, specifically of the PNC 157 gene, is determined.
The test cell population contains a PNC cell expressing a PNC-associated gene. Preferably, the test cell is an epithelial cell. For example a test cell population is incubated in the presence of a candidate agent and the pattern of gene expression of the test sample is measured and compared to one or more reference profiles, *e.g.*, a PNC reference expression profile or a non-PNC reference expression profile.
A decrease in expression of one or more of PNC 1-259, specifically of the PNC 157 gene, or an increase in expression of one or more of PNC 260-605 described herein in a test cell population relative to a reference cell population containing PNC is indicative that the agent is therapeutic.
The test agent can be any compound or composition. For example, the test agents are immunomodulatory agents.

*Screening assays for identifying therapeutic agents*

The differentially expressed genes, in particular the PNC 157 gene, disclosed herein can also be used to identify candidate therapeutic agents for treating PNC. The method is based on screening a candidate therapeutic agent to determine if it converts an expression profile of PNC 1-605, specifically of PNC 157, characteristic of a PNC state to a pattern indicative of a non-PNC state.

In the method, a cell is exposed to a test agent or a combination of test agents (sequentially or consequentially) and the expression of one or more PNC 1-605, specifically of PNC 157, in the cell is measured. The expression profile of the PNC-associated gene in the test population is compared to expression level of the PNC-associated gene in a reference cell population that is not exposed to the test agent.

An agent effective in stimulating expression of under-expressed genes, or in suppressing expression of over-expressed genes is deemed to lead to a clinical benefit such compounds are further tested for the ability to prevent pancreatic ductal adenocarcinomal growth in animals or test subjects.

In a further embodiment, the present invention provides methods for screening candidate agents which are potential targets in the treatment of PNC. As discussed in detail above, by controlling the expression levels or activities of marker genes, one can control the onset and progression of PNC. Thus, candidate agents, which are potential targets in the treatment of PNC, can be identified through screenings that use the expression levels and activities of marker genes as indices. In the context of the present invention, such screening may comprise, for example, the following steps:

a) contacting a test compound with a polypeptide encoded by PNC 157;
b) detecting the binding activity between the polypeptide and the test compound; and
c) selecting a compound that binds to the polypeptide.

Alternatively, the screening method of the present invention may comprise the following steps:

a) contacting a candidate compound with a cell expressing one or more marker genes, wherein the marker gene is PNC 157; and
b) selecting a compound that reduces the expression level of the PNC 157 marker gene.

Cells expressing a marker gene include, for example, cell lines established from PNC; such cells can be used for the above screening of the present invention.

A protein required for the screening can be obtained as a recombinant protein using the nucleotide sequence of the marker gene. Based on the information of the marker gene, one skilled in the art can select any biological activity of the protein as an index for screening and a measurement method based on the selected biological activity.

Alternatively, the screening method may comprise the following steps:

a) contacting a candidate compound with a cell into which a vector comprising the transcriptional regulatory region of one or more marker genes and a reporter gene that is expressed under the control of the transcriptional regulatory region has been introduced, wherein the marker gene is PNC 157;
b) measuring the activity of said reporter gene; and
c) selecting a compound that reduces the expression level of said reporter gene when said marker gene is an up-regulated marker gene, in particular PNC 157, as compoared to a control. If the marker gene is down-regulated, it is selected from the group consisting of PNC 260-605, as compared to a control.

Suitable reporter genes and host cells are well known in the art. The reporter construct required for the screening can be prepared by using the transcriptional regulatory region of a marker gene. When the transcriptional regulatory region of a marker gene has been known to those skilled in the art, a reporter construct can be prepared by using the previous sequence information. When the transcriptional regulatory region of a marker gene remains unidentified a nucleotide segment containing the transcriptional regulatory region can be isolated from a genome library based on the nucleotide sequence information of the marker gene.

The compound isolated by the screening is a candidate for drugs that inhibit the activity of the protein encoded by marker genes and can be applied to the treatment or prevention of pancreatic cancer.

Moreover, compound in which a part of the structure of the compound inhibiting the activity of proteins encoded by marker genes is converted by addition, deletion and/or replacement are also included in the compounds obtainable by the screening method of the present invention.

When administrating the compound isolated by the method of the invention as a pharmaceutical for humans and other mammals, such as mice, rats, guinea-pigs, rabbits, cats, dogs, sheep, pigs, cattle, monkeys, baboons, and chimpanzees, the isolated compound can be directly administered or can be formulated into a dosage form using known pharmaceutical preparation methods. For example, according to the need, the drugs can be taken orally, as sugar-coated tablets, capsules, elixirs and microcapsules, or non-orally, in the form of injections of sterile solutions or suspensions with water or any other pharmaceutically acceptable liquid. For example, the compounds can be mixed with pharmaceutically acceptable carriers or media, specifically, sterilized water, physiological saline, plant-oils, emulsifiers, suspending agents, surfactants, stabilizers, flavoring agents, excipients, vehicles, preservatives, binders, and such, in a unit dose form required for generally accepted drug implementation. The amount of active ingredients in these preparations makes a suitable dosage within the indicated range acquirable.

Examples of additives that can be mixed to tablets and capsules are, binders such as gelatin, corn starch, tragacanth gum and arabic gum; excipients such as crystalline cellulose; swelling agents such as corn starch, gelatin and alginic acid; lubricants such as magnesium stearate; sweeteners such as sucrose, lactose or saccharin; and flavoring agents such as peppermint, Gaultheria adenothrix oil and cherry. When the unit-dose form is a capsule, a liquid carrier, such as an oil, can also be further included in the above ingredients. Sterile composites for injections can be formulated following normal drug implementations using vehicles such as distilled water used for injections.

Physiological saline, glucose, and other isotonic liquids including adjuvants, such as D-sorbitol, D-mannnose, D-mannitol, and sodium chloride, can be used as aqueous solutions for injections. These can be used in conjunction with suitable solubilizers, such as alcohol, specifically ethanol, polyalcohols such as propylene glycol and polyethylene glycol, non-ionic surfactants, such as Polysorbate 80 (TM) and HCO-50.

Sesame oil or Soy-bean oil can be used as a oleaginous liquid and may be used in conjunction with benzyl benzoate or benzyl alcohol as a solubilizer and may be formulated with a buffer, such as phosphate buffer and sodium acetate buffer; a pain-killer, such as procaine hydrochloride; a stabilizer, such as benzyl alcohol andphenol; and an anti-oxidant. The prepared injection may be filled into a suitable ampule.

Methods well known to one skilled in the art may be used to administer the pharmaceutical composition of the present inevntion to patients, for example as intraarterial, intravenous, or percutaneous injections and also as intranasal, transbronchial, intramuscular or oral administrations. The dosage and method of administration vary according to the body-weight and age of a patient and the administration method; however, one skilled in the art can routinely select a suitable metod of administration. If said compound is encodable by a DNA, the DNA can be inserted into a vector for gene therapy and the vector administered to a patient to perform the therapy. The dosage and method of administration vary according to the body-weight, age, and symptoms of the patient but one skilled in the art can suitably select them.

For example, although the dose of a compound that binds to the protein of the present invention and regulates its activity depends on the symptoms, the dose is about 0.1 mg to about 100 mg per day, preferably about 1.0 mg to about 50 mg per day and more preferably about 1.0 mg to about 20 mg per day, when administered orally to a normal adult (weight 60 kg).

When administering parenterally, in the form of an injection to a normal adult (weight 60 kg), although there are some differences according to the patient, target organ, symptoms and method of administration, it is convenient to intravenously inject a dose of about 0.01 mg to about 30 mg per day, preferably about 0.1 to about 20 mg per day and more preferably about 0.1 to about 10 mg per day. Also, in the case of other animals too, it is possible to administer an amount converted to 60 kgs of body-weight.

*Screening assays for identifying therapeutic agents for malignant pancreatic cancer*

The present invention provides target molecules for treating or preventing malignant pancreatic cancer. In the present invention, malignant cancer includes cancers having properties such as follows:

- local invasion;
- aggressive proliferation ; and
- metastasis.

Therefore, according to the present invention, malignant pancreatic cancer includes pancreatic cancer with metastasis. Screening assay for malignant PNC of the present invention can be performed according to the mehtod for PNC described above using marker genes for malignant pancreatic cancer.

It is described herein that marker genes selected from the group consisting of PNC 606-681, and 682-849 are useful for the screening. 76 genes shown in Table 6 (PNC 606-681) were associated with lymph node metastasis. Among the genes, 35 genes (PNC 606-640) were relatively up-regulated and 41 genes (PNC 641-681) were down-regulated in node-positive tumors (Figure 3). In addition, 168 genes (PNC 682-849) showed unique altered expression patterns in pancreatic cells with liver metastasis (Table 7) wherein 60 of the genes(PNC 682-741) were relatively up-regulated (Figure 4). An agent suppressing the activity or expression of these up-regulated genes obtained by the present invention are useful for treating or preventing malignant pancreatic cancer with lymph-node metastasis or liver metastasis. Alternatively, an agent enhancing the activity or expression of the down-regulated genes obtained by the present invention are also useful for treating or preventing malignant pancreatic cancer.

Furthermore, described herein are target molecules for treating or preventing recurrence of pancreatic cancer. Herein, recurrence of pancreatic cancer indicates recurrence of cancer in pancreas after surgery. For example, the recurrence of cancer within 12 month after surgery can be predicted by the invention. According to the present invention, early recurrence includes the recurrence within 12 month after surgery, and when no recurrence can be observed within 12 month after surgery in a case, the case is considered to be a pancreatic cancer with "late recurrence". 84 genes (PNC 850-933) shown in Table 8 are useful as the marker genes for screening. Among them, the genes shown in Figure 5A-1 are up-regulated in early recurrence cases(PNC 894-933), and the genes shown in Figure 5A-2 are up-regulated in late recurrence cases(PNC 850-893). Therefore, an agent suppressing the up-regulated genes in early recurrence cases is useful for treating or preventing recurrence. Alternatively, an agent enhancing the up-regulated genes in late recurrence cases is also useful for treating or preventing recurrence.

*Assessing the prognosis of a subject with pancreatic cancer*

Also described is a method of assessing the prognosis of a subject with PNC by comparing the expression of one or more PNC-associated gene in a test cell population to the expression of the genes in a reference cell population derived from patients over a spectrum of disease stages. By comparing gene expression of one or more PNC-associated gene in the test cell population and the reference cell population(s), or by comparing the pattern of gene expression over time in test cell populations derived from the subject, the prognosis of the subject can be assessed.

A decrease in expression of one or more of PNC 260-605 compared to a normal control or an increase of expression of one or more of PNC 1-259, specifically PNC 157, compared to a normal control indicates less favorable prognosis. A similar expression of one or more of PNC 1-605, specifically, PNC 157, indicates a more favorable prognosis compared to nomal control indicates a more favorable prognosis for the subject. Preferably, the prognosis of a subject can be assessed by comparing the expression profile of PNC 1-605, specifically PNC 157. The classification score (CS) may be use for the comparing the expression profile.

*Kits*

Also described herein is a PNC-detection reagent, e.g., a nucleic acid that specifically binds to or identifies one or more PNC nucleic acids such as oligonucleotide sequences, which are complementary to a portion of a PNC nucleic acid or antibodies which bind to proteins encoded by a PNC nucleic acid. The reagents are packaged together in the form of a kit. The reagents are packaged in separate containers, e.g., a nucleic acid or antibody (either bound to a solid matrix or packaged separately with reagents for binding them to the matrix), a control reagent (positive and/or negative), and/or a detectable label. Instructions (e.g., written, tape, VCR, CD-ROM, etc.) for carrying out the assay are included in the kit. The assay format of the kit is a Northern hybridization or a sandwich ELISA known in the art.

For example, PNC detection reagent is immobilized on a solid matrix such as a porous strip to form at least one PNC detection site. The measurement or detection region of the porous strip may include a plurality of sites containing a nucleic acid. A test strip may also contain sites for negative and/or positive controls. Alternatively, control sites are located on a separate strip from the test strip. Optionally, the different detection sites may contain different amounts of immobilized nucleic acids, *i.e.*, a higher amount in the first detection site and lesser amounts in subsequent sites. Upon the addition of test sample, the number of sites displaying a detectable signal provides a quantitative indication of the amount of PNC present in the sample. The detection sites may be configured in any suitably detectable shape and are typically in the shape of a bar or dot spanning the width of a teststrip.

Alternatively, the kit contains a nucleic acid substrate array comprising one or more nucleic acids. The nucleic acids on the array specifically identify one or more nucleic acid sequences represented by PNC 1-605. The expression of 2,3,4, 5, 6, 7, 8, 9, 10, 15, 20, 25, 40 or 50 or more of the nucleic acids represented by PNC 1-605 are identified by virtue of the level of binding to an array test strip or chip. The substrate array can be on, *e.g.*, a solid substrate, *e.g.*, a "chip" as described in U.S. Patent No.5,744,305.

*Arrays and pluralities*

Herein described is a nucleic acid substrate array comprising one or more nucleic acids. The nucleic acids on the array specifically correspond to one or more nucleic acid sequences represented by PNC 1-605, in particular PNC 157. The level of expression of 2, 3, 4, 5, 6, 7, 8, 9, 10, 15, 20, 25, 40 or 50 or more of the nucleic acids represented by PNC 1-605 are identified by detecting nucleic acid binding to the array.

Further described herein is an isolated plurality (*i.e.*, a mixture if two or more nucleic acids) of nucleic acids. The nucleic acids are in a liquid phase or a solid phase, *e.g.*, immobilized on a solid support such as a nitrocellulose membrane. The plurality includes one or more of the nucleic acids represented by PNC 1-605, in particular PNC 157. In various embodiments, the plurality includes 2, 3, 4, 5, 6, 7, 8, 9, 10,15, 20, 25,40 or 50 or more of the nucleic acids represented by PNC 1-605, in particular PNC 157.

*Methods of inhibiting pancreatic cancer*

The invention provides a composition for use in a method for treating or alleviating a symptom of PNC in a subject by decreasing expression or activity of PNC 157. Therapeutic compounds are administered prophylactically or therapeutically to subject suffering from or at risk of (or susceptible to) developing PNC. Such subjects are identified using standard clinical methods or by detecting an aberrant level of expression or activity of PNC 157. Therapeutic agents include inhibitors of cell cycle regulation, cell proliferation, and protein kinase activity.

The method includes decreasing the expression, or function, or both, of one or more gene products of genes, in particular of the PNC 157 gene, whose expression is aberrantly increased ("over-expressed gene") in pancreas cells. Expression is inhibited in any of several ways known in the art. For example, expression is inhibited by administering to the subject a nucleic acid that inhibits, or antagonizes, the expression of the over-expressed gene or genes, e.g., an antisense oligonucleotide of small interfering RNA which disrupts expression of the over-expressed gene or genes.

As noted above, antisense nucleic acids corresponding to the nucleotide sequence of PNC 157 can be used to reduce the expression level of the PNC 157. Antisense nucleic acids corresponding to PNC 157 that is up-regulated in pancreatic cancer are useful for the treatment of pancreatic cancer. Specifically, the antisense nucleic acids of the present invention may act by binding to PNC 157 or mRNAs corresponding thereto, thereby inhibiting the transcription or translation of the genes, promoting the degradation of the mRNAs, and/or inhibiting the expression of proteins encoded by PNC 157, finally inhibiting the function of the proteins. The term "antisense nucleic acids" as used herein encompasses both nucleotides that are entirely complementary to the target sequence and those having a mismatch of one or more nucleotides, so long as the antisense nucleic acids can specifically hybridize to the target sequences. For example, the antisense nucleic acids of the present invention include polynucleotides that have a homology of at least 70% or higher, preferably at 80% or higher, more preferably 90% or higher, even more preferably 95% or higher over a span of at least 15 continuous nucleotides. Algorithms known in the art can be used to determine the homology.

The antisense nucleic acid derivatives of the present invention act on cells producing the proteins encoded by marker genes by binding to the DNAs or mRNAs encoding the proteins, inhibiting their transcription or translation, promoting the degradation of the mRNAs, and inhibiting the expression of the proteins, thereby resulting in the inhibition of the protein function.

An antisense nucleic acid derivative of the present invention can be made into an external preparation, such as a liniment or a poultice, by mixing with a suitable base material which is inactive against the derivative.

Also, as needed, the derivatives can be formulated into tablets, powders, granules, capsules, liposome capsules, injections, solutions, nose-drops and freeze-drying agents by adding excipients, isotonic agents, solubilizers, stabilizers, preservatives, pain-killers, and such. These can be prepared by following known methods.

The antisense nucleic acids derivative is given to the patient by directly applying onto the ailing site or by injecting into a blood vessel so that it will reach the site of ailment. An antisense-mounting medium can also be used to increase durability and membrane-permeability. Examples are, liposomes, poly-L-lysine, lipids, cholesterol, lipofectin or derivatives of these.

The dosage of the antisense nucleic acid derivative of the present invention can be adjusted suitably according to the patient's condition and used in desired amounts. For example, a dose range of 0.1 to 100 mg/kg, preferably 0.1 to 50 mg/kg can be administered.

The antisense nucleic acids of the invention inhibit the expression of the PNC 157 protein of the invention and is thereby useful for suppressing the biological activity of a protein of the invention. Also, expression-inhibitors, comprising the antisense nucleic acids of the invention, are useful since they can inhibit the biological activity of a protein of the invention.

The antisense nucleic acids of present invention include modified oligonucleotides. For example, thioated nucleotides may be used to confer nuclease resistance to an oligonucleotide. Also, a siRNA against marker gene can be used to reduce the expression level of the PNC 157 marker gene. By the term "siRNA" is meant a double stranded RNA molecule which prevents translation of a target mRNA. Standard techniques of introducing siRNA into the cell are used, including those in which DNA is a template from which RNA is transcribed. In the context of the present invention, the siRNA comprises a sense nucleic acid sequence and an anti-sense nucleic acid sequence against an up-regulated marker gene, in particular PNC 157. The siRNA is constructed such that a single transcript has both the sense and complementary antisense sequences from the target gene, e.g., a hairpin.

The method is used to alter the expression in a cell of an up-regulated, e.g., as a result of malignant transformation of the cells. Binding of the siRNA to a transcript corresponding to PNC 157 in the target cell results in a reduction in the protein production by the cell. The length of the oligonucleotide is at least 10 nucleotides and may be as long as the naturally-occurring transcript. Preferably, the oligonucleotide is 19-25 nucleotides in length. Most preferably, the oligonucleotide is less than 75, 50, 25 nucleotides in length.

The nucleotide sequence of the siRNAs were designed using an siRNA design computer program available from the Ambion website (http://www.ambion.com/techlib/ misc/siRNA_finder.html). The computer program selects nucleotide sequences for siRNA synthesis based on the following protocol.

Selection of siRNA Target Sites:

1. Beginning with the AUG start codon of the object transcript, scan downstream for AA dinucleotide sequences.

Record the occurrence of each AA and the 3' adjacent 19 nucleotides as potential siRNA target sites. Tuschl, et al. recommend against designing siRNA to the 5' and 3' untranslated regions (UTRs) and regions near the start codon (within 75 bases) as these may be richer in regulatory protein binding sites. UTR-binding proteins and/or translation initiation complexes may interfere with binding of the siRNA endonuclease complex.

2. Compare the potential target sites to the human genome database and eliminate from consideration any target sequences with significant homology to other coding sequences. The homology search can be performed using BLAST, which can be found on the NCBI server at: www.ncbi.nlm.nih.gov/BLAST/

3. Select qualifying target sequences for synthesis. At Ambion, preferably several target sequences can be selected along the length of the gene to evaluate. The antisense oligonucleotide or siRNA of the invention inhibit the expression of the PNC 157 polypeptide of the invention and is thereby useful for suppressing the biological activity of the polypeptide of the invention. Also, expression-inhibitors, comprising the antisense oligonucleotide or siRNA of the invention, are useful in the point that they can inhibit the biological activity of the polypeptide of the invention. Therefore, a composition comprising the antisense oligonucleotide or siRNA of the present invention is useful in treating a pancreatic cancer.

Alternatively, function of the PNC 157 gene product is inhibited by administering a compound that binds to or otherwise inhibits the function of the gene products. For example, the compound is an antibody which binds to the over-expressed gene product or gene products.

The present invention refers to the use of antibodies, particularly antibodies against a PNC 157 protein encoded by the up-regulated PNC 157 marker gene, or a fragment of the antibody. As used herein, the term "antibody" refers to an immunoglobulin molecule having a specific structure, that interacts (i.e., binds) only with the antigen that was used for synthesizing the antibody (i.e., the up-regulated marker gene product) or with an antigen closely related to it. Furthermore, an antibody may be a fragment of an antibody or a modified antibody, so long as it binds to one or more of the proteins encoded by the marker genes. For instance, the antibody fragment may be Fab, $F(ab')_2$, Fv, or single chain Fv (scFv), in which Fv fragments from H and L chains are ligated by an appropriate linker (Huston J. S. et al. Proc. Natl. Acad. Sci. U.S.A. 85:5879-5883 (1988)). More specifically, an antibody fragment may be generated by treating an antibody with an enzyme, such as papain or pepsin. Alternatively, a gene encoding the antibody fragment may be constructed, inserted into an expression vector, and expressed in an appropriate host cell (see, for example, Co M. S. et al. J. Immunol. 152:2968-2976 (1994); Better M. and Horwitz A. H. Methods Enzymol. 178:476-496 (1989); Pluckthun A. and Skerra A. Methods Enzymol. 178:497-515 (1989); Lamoyi E. Methods Enzymol. 121:652-663 (1986); Rousseaux J. et al. Methods Enzymol. 121:663-669 (1986); Bird R. E. and Walker B. W. Trends Biotechnol. 9:132-137 (1991)).

An antibody may be modified by conjugation with a variety of molecules, such as polyethylene glycol (PEG). The present invention provides such modified antibodies. The modified antibody can be obtained by chemically modifying an antibody. These modification methods are conventional in the field.

Alternatively, an antibody may be obtained as a chimeric antibody, between a variable region derived from a nonhuman antibody and a constant region derived from a human antibody, or as a humanized antibody, comprising the complementarity determining region (CDR) derived from a nonhuman antibody, the frame work region (FR) derived from a human antibody, and the constant region. Such antibodies can be prepared by using known technologies.

Cancer therapies directed at specific molecular alterations that occur in cancer cells have been validated through clinical development and regulatory approval of anti-cancer drugs such as trastuzumab (Herceptin) for the treatment of advanced breast cancer, imatinib methylate (Gleevec) for chronic myeloid leukemia, gefitinib (Iressa) for non-small cell lung cancer (NSCLC), and rituximab (anti-CD20 mAb) for B-cell lymphoma and mantle cell lymphoma (Ciardiello F, Tortora G. A novel approach in the treatment of cancer: targeting the epidermal growth factor receptor. Clin Cancer Res. 2001 Oct;7(10):2958-70. Review.; Slamon DJ, Leyland-Jones B, Shak S, Fuchs H, Paton V, Bajamonde A, Fleming T, Eiermann W, Wolter J, Pegram M, Baselga J, Norton L. Use of chemotherapy plus a monoclonal antibody against HER2 for metastatic breast cancer that overexpresses HER2. N Engl J Med. 2001 Mar 15;344(11):783-92.; Rehwald U, Schulz H, Reiser M, Sieber M, Staak JO, Morschhauser F, Driessen C, Rudiger T, Muller-Hermelink K, Diehl V, Engert A. Treatment of relapsed CD20+ Hodgkin lymphoma with the monoclonal antibody rituximab is effective and well tolerated: results of a phase 2 trial of the German Hodgkin Lymphoma Study Group. Blood. 2003 Jan 15;101 (2):420-424.; Fang G, Kim CN, Perkins CL, Ramadevi N, Winton E, Wittmann S and Bhalla KN. (2000). Blood, 96, 2246-2253.). These drugs are clinically effective and better tolerated than traditional anti-cancer agents because they target only transformed cells. Hence, such drugs not only improve survival and quality of life for cancer patients, but also validate the concept of molecularly targeted cancer therapy. Furthermore, targeted drugs can enhance the efficacy of standard chemotherapy when used in combination with it (Gianni L. (2002). Oncology, 63 Suppl 1, 47-56.; Klejman A, Rushen L, Morrione A, Slupianek A and Skorski T. (2002). Oncogene, 21, 5868-5876.). Therefore, future cancer treatments will probably involve combining conventional drugs with target-specific agents aimed at different characteristics of tumor cells such as angiogenesis and invasiveness.

These modulatory methods are performed ex vivo or in vitro (e.g., by culturing the cell with the agent) or, alternatively, in vivo (e.g., by administering the agent to a subject). The method involves administering a protein or combination of proteins or a nucleic acid molecule or combination of nucleic acid, molecules as therapy to counteract aberrant expression or activity of the differentially expressed genes.

Diseases and disorders that are characterized by increased (relative to a subject not suffering from the disease or disorder) levels or biological activity of the genes may be treated with therapeutics that antagonize (i.e., reduce or inhibit) activity of the over-expressed gene or genes. Therapeutics that antagonized activity are administered therapeutically or prophylactically.

Therapeutics that may be utilized include, e.g., (i) a polypeptide, or analogs, derivatives, fragments or homologs thereof of the over-expressed or under-expressed gene or genes; (ii) antibodies to the over-expressed or under-expressed gene or genes; (iii) nucleic acids encoding the over-expressed or under-expressed gene or genes; (iv) antisense nucleic acids or nucleic acids that are "dysfunctional" (i.e., due to a heterologous insertion within the nucleic acids of one or more over-expressed or under-expressed gene or genes); (v) small interfering RNA (siRNA); or (vi) modulators (i.e., inhibitors, agonists and antagonists that alter the interaction between an over/under-expressed polypeptide and its binding partner). The dysfunctional antisense molecules are utilized to "knockout" endogenous function of a polypeptide by homologous recombination (see, e.g., Capecchi, Science 244: 1288-1292 1989). 259

Diseases and disorders that are characterized by decreased (relative to a subject not suffering from the disease or disorder) levels or biological activity may be treated with therapeutics that increase (i.e., are agonists to) activity. Therapeutics that up-regulate activity may be administered in a therapeutic or prophylactic manner. Therapeutics that may be utilized include, but are not limited to, a polypeptide (or analogs, derivatives, fragments or homologs thereof) or an agonist that increases bioavailability.

Increased or decreased levels can be readily detected by quantifying peptide and/or RNA, by obtaining a patient tissue sample (e.g., from biopsy tissue) and assaying it in vitro for RNA or peptide levels, structure and/or activity of the expressed peptides (or mRNAs of a gene whose expression is altered). Methods that are well-known within the art include, but are not limited to, immunoassays (e.g., by Western blot analysis, immunoprecipitation followed by sodium dodecyl sulfate (SDS) polyacrylamide gel electrophoresis, immunocytochemistry, etc.) and/or hybridization assays to detect expression of mRNAs (e.g., Northern assays, dot blots, in situ hybridization, etc.).

Prophylactic administration occurs prior to the manifestation of overt clinical symptoms of disease, such that a disease or disorder is prevented or, alternatively, delayed in its progression.

Therapeutic methods include contacting a cell with an agent that modulates one or more of the activities of the gene products of the differentially expressed genes. An agent that modulates protein activity includes a nucleic acid or a protein, a naturally-occurring cognate ligand of these proteins, a peptide, a peptidomimetic, or other small molecule. For example, the agent stimulates one or more protein activities of one or more of a differentially under-expressed gene.

Herein described are compositions for use in a method of treating or preventing pancreatic cancer in a subject comprising administering to said subject a vaccine comprising a polypeptide encoded by a nucleic acid selected from the group consisting of PNC 1-259, in particular PNC 157, or an immunologically active fragment of said polypeptide, or a polynucleotide encoding the polypeptide or the fragment thereof. An administration of the polypeptide induces an anti-tumor immunity in a subject. To inducing anti-tumor immunity, a polypeptide encoded by a nucleic acid selected from the group consisting of PNC 1-259, in particular PNC 157, or an immunologically active fragment of said polypeptide, or a polynucleotide encoding the polypeptide is administered. The polypeptide or the immunologically active fragments thereof are useful as vaccines against PNC. In some cases the proteins or fragments thereof may be administered in a form bound to the T cell recepor (TCR) or presented by an antigen presenting cell (APC), such as macrophage, dendritic cell (DC), or B-cells. Due to the strong antigen presenting ability of DC, the use of DC is most preferable among the APCs.

A vaccine against PNC refers to a substance that has the function to induce anti-tumor immunity upon inoculation into animals. Accordingly, polypeptides encoded by PNC 1-259 or fragments thereof were suggested to be HLA-A24 or HLA-A*0201 restricted epitopes peptides that may induce potent and specific immune response against PNC cells expressing PNC 1-259. Thus, herein described is a composition for use in a method of inducing anti-tumor immunity using the polypeptides. In general, anti-tumor immunity includes immune responses such as follows:

- induction of cytotoxic lymphocytes against tumors,
- induction of antibodies that recognize tumors, and
- induction of anti-tumor cytokine production.

Therefore, when a certain protein induces any one of these immune responses upon inoculation into an animal, the protein is decided to have anti-tumor immunity inducing effect. The induction of the anti-tumor immunity by a protein can be detected by observing in vivo or in vitro the response of the immune system in the host against the protein.

For example, a method for detecting the induction of cytotoxic T lymphocytes is well known. A foreign substance that enters the living body is presented to T cells and B cells by the action of antigen presenting cells (APCs). T cells that respond to the antigen presented by APC in antigen specific manner differentiate into cytotoxic T cells (or cytotoxic T lymphocytes; CTLs) due to stimulation by the antigen, and then proliferate (this is referred to as activation of T cells). Therefore, CTL induction by a certain peptide can be evaluated by presenting the peptide to T cell by APC, and detecting the induction of CTL. Furthermore, APC has the effect of activating CD4+ T cells, CD8+ T cells, macrophages, eosinophils, and NK cells. Since CD4+ T cells and CD8+ T cells are also important in anti-tumor immunity, the anti-tumor immunity inducing action of the peptide can be evaluated using the activation effect of these cells as indicators.

A method for evaluating the inducing action of CTL using dendritic cells (DCs) as APC is well known in the art. DC is a representative APC having the strongest CTL inducing action among APCs. In this method, the test polypeptide is initially contacted with DC, and then this DC is contacted with T cells. Detection of T cells having cytotoxic effects against the cells of interest after the contact with DC shows that the test polypeptide has an activity of inducing the cytotoxic T cells. Activity of CTL against tumors can be detected, for example, using the lysis of $^{51}$Cr-labeled tumor cells as the indicator. Alternatively, the method of evaluating the degree of tumor cell damage using $^{3}$H-thymidine uptake activity or LDH (lactose dehydrogenase)-release as the indicator is also well known.

Apart from DC, peripheral blood mononuclear cells (PBMCs) may also be used as the APC. The induction of CTL is reported that it can be enhanced by culturing PBMC in the presence of GM-CSF and IL-4. Similarly, CTL has been shown to be induced by culturing PBMC in the presence of keyhole limpet hemocyanin (KLH) and IL-7.

The test polypeptides confirmed to possess CTL inducing activity by these methods are polypeptides having DC activation effect and subsequent CTL inducing activity. Therefore, polypeptides that induce CTL against tumor cells are useful as vaccines against tumors. Furthermore, APC that acquired the ability to induce CTL against tumors by contacting with the polypeptides are useful as vaccines against tumors. Furthermore, CTL that acquired cytotoxicity due to presentation of the polypeptide antigens by APC can be also used as vaccines against tumors. Such therapeutic methods for tumors using anti-tumor immunity due to APC and CTL are referred to as cellular immunotherapy.

Generally, when using a polypeptide for cellular immunotherapy, efficiency of the CTL-induction is known to increase by combining a plurality of polypeptides having different structures and contacting them with DC. Therefore, when stimulating DC with protein fragments, it is advantageous to use a mixture of multiple types of fragment.

Alternatively, the induction of anti-tumor immunity by a polypeptide can be confirmed by observing the induction of antibody production against tumors. For example, when antibodies against a polypeptide are induced in a laboratory animal immunized with the polypeptide, and when growth of tumor cells is suppressed by those antibodies, the polypeptide can be determined to have an ability to induce anti-tumor immunity.

Anti-tumor immunity is induced by administering the vaccine of this invention, and the induction of anti-tumor immunity enables treatment and prevention of PNC. Therapy against cancer or prevention of the onset of cancer includes any of the steps, such as inhibition of the growth of cancerous cells, involution of cancer, and suppression of occurrence of cancer. Decrease in mortality of individuals having cancer, decrease of tumor markers in the blood, alleviation of detectable symptoms accompanying cancer, and such are also included in the therapy or prevention of cancer. Such therapeutic and preventive effects are preferably statistically significant. For example, in observation, at a significance level of 5% or less, wherein the therapeutic or preventive effect of a vaccine against cell proliferative diseases is compared to a control without vaccine administration. For example, Student's t-test, the Mann-Whitney U-test, or ANOVA may be used for statistical analyses.

The above-mentioned protein having immunological activity or a vector encoding the protein may be combined with an adjuvant. An adjuvant refers to a compound that enhances the immune response against the protein when administered together (or successively) with the protein having immunological activity. Examples of adjuvants include cholera toxin, salmonella toxin, alum, and such, but are not limited thereto. Furthermore, the vaccine described herein may be combined appropriately with a pharmaceutically acceptable carrier. Examples of such carriers are sterilized water, physiological saline, phosphate buffer, culture fluid, and such. Furthermore, the vaccine may contain as necessary, stabilizers, suspensions, preservatives, surfactants, and such. The vaccine is administered systemically or locally. Vaccine administration may be performed by single administration, or boosted by multiple administrations.

When using APC or CTL as the vaccine of this invention, tumors can be treated or prevented, for example, by the ex vivo method. More specifically, PBMCs of the subject receiving treatment or prevention are collected, the cells are contacted with the polypeptide ex vivo, and following the induction of APC or CTL, the-cells may be administered to the subject. APC can be also induced by introducing a vector encoding the polypeptide into PBMCs ex vivo. APC or CTL induced in vitro can be cloned prior to administration. By cloning and growing cells having high activity of damaging target cells, cellular immunotherapy can be performed more effectively. Furthermore, APC and CTL isolated in this manner may be used for cellular immunotherapy not only against individuals from whom the cells are derived, but also against similar types of tumors from other individuals.

*Methods for inhibiting development or recurrence of malignant pancreatic cancer*

Further described herein is a composition for use in a method for treating or preventing malignant pancreatic cancer, or recurrence of pancreatic cancer by increasing or decreasing the expression or activity of marker genes. The marker genes that can be used for the treatment or prevention of malignant pancreatic cancer are PNC 606-681 (Table 6) and PNC 682-849 (Table 7). Alternatively, the marker genes for treating or preventing the recurrence are PNC 850-933 (Table 8). 35 genes of the PNC 606-640 (Figure 3) and 60 genes of PNC 682-741 (Figure 4) are up-regulated in the malignant cancer cells and 40 genes of PNC 894-933 are up-regulated in the early recurrence cases. Antisense-nucleotides and siRNAs against any one of the up-regulated marker genes are useful for suppressing the expression of the up-regulated genes. Alternatively, the activity of a protein encoded by any one of the up-regulated marker genes can be inhibited by administering an antibody that binds to the protein. Furthermore, a vaccine against the protein encoded by any one of the up-regulated marker genes is useful for inducing anti tumor immunity. Moreover, administration of the down regulated genes or proteins encoded thereby is also effective for treating or preventing malignant pancreatic cancer or the recurrence.

*Pharmaceutical compositions for inhibiting PNC, malignant PNC, or recurrence of PNC.*

Pharmaceutical formulations include those suitable for oral, rectal, nasal, topical (including buccal and sublingual), vaginal or parenteral (including intramuscular, subcutaneous and intravenous) administration, or for administration by inhalation or insufflation. Preferably, administration is intravenous. The formulations are optionally packaged in discrete dosage units.

Pharmaceutical formulations suitable for oral administration include capsules, cachets or tablets, each containing a predetermined amount of the active ingredient. Formulations also include powders, granules or solutions, suspensions or emulsions. The active ingredient is optionally administered as a bolus electuary or paste. Tablets and capsules for oral administration may contain conventional excipients such as binding agents, fillers, lubricants, disintegrant or wetting agents. A tablet may be made by compression or molding, optionally with one or more formulational ingredients. Compressed tablets may be prepared by compressing in a suitable machine the active ingredients in a free-flowing form such as a powder or granules, optionally mixed with a binder, lubricant, inert diluent, lubricating, surface active or dispersing agent. Molded tablets may be made by molding in a suitable machine a mixture of the powdered compound moistened with an inert liquid diluent. The tablets may be coated according to methods well known in the art. Oral fluid preparations may be in the form of, for example, aqueous or oily suspensions, solutions, emulsions, syrups or elixirs, or may be presented as a dry product for constitution with water or other suitable vehicle before use. Such liquid preparations may contain conventional additives such as suspending agents, emulsifying agents, non-aqueous vehicles (which may include edible oils), or preservatives. The tablets may optionally be formulated so as to provide slow or controlled release of the active ingredient therein. A package of tablets may contain one tablet to be taken on each of the month.

Formulations for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit dose or multi-dose containers, for example sealed ampoules and vials, and may be stored in a freeze-dried (lyophilized) condition requiring only the addition of the sterile liquid carrier, for example, saline, water-for-injection, immediately prior to use. Alternatively, the formulations may be presented for continuous infusion. Extemporaneous injection solutions and suspensions may be prepared from sterile powders, granules and tablets of the kind previously described.

Formulations for rectal administration include suppositories with standard carriers such as cocoa butter or polyethylene glycol. Formulations for topical administration in the mouth, for example buccally or sublingually, include lozenges, which contain the active ingredient in a flavored base such as sucrose and acacia or tragacanth, and pastilles comprising the active ingredient in a base such as gelatin and glycerin or sucrose and acacia. For intra-nasal administration the compounds of the invention may be used as a liquid spray or dispersible powder or in the form of drops. Drops may be formulated with an aqueous or non-aqueous base also comprising one or more dispersing agents, solubilizing agents or suspending agents.

For administration by inhalation the compounds are conveniently delivered from an insufflator, nebulizer, pressurized packs or other convenient means of delivering an aerosol spray. Pressurized packs may comprise a suitable propellant such as dichlorodifluoromethane, trichlorofluoromethane, dichiorotetrafluoroethane, carbon dioxide or other suitable gas. In the case of a pressurized aerosol, the dosage unit may be determined by providing a valve to deliver a metered amount.

Alternatively, for administration by inhalation or insufflation, the compounds may take the form of a dry powder composition, for example a powder mix of the compound and a suitable powder base such as lactose or starch. The powder composition may be presented in unit dosage form, in for example, capsules, cartridges, gelatin or blister packs from which the powder may be administered with the aid of an inhalator or insufflators.

Other formulations include implantable devices and adhesive patches; which release a therapeutic agent.

When desired, the above described formulations, adapted to give sustained release of the active ingredient, may be employed. The pharmaceutical compositions may also contain other active ingredients such as antimicrobial agents, immunosuppressants or preservatives.

It should be understood that in addition to the ingredients particularly mentioned above, the formulations of this invention may include other agents conventional in the art having regard to the type of formulation in question, for example, those suitable for oral administration may include flavoring agents.

Preferred unit dosage formulations are those containing an effective dose, as recited below, or an appropriate fraction thereof, of the active ingredient.

For each of the aforementioned conditions, the compositions, e.g., polypeptides and organic compounds are administered orally or via injection at a dose of from about 0.1 to about 250 mg/kg per day. The dose range for adult humans is generally from about 5 mg to about 17.5 g/day, preferably about 5 mg to about 10 g/day, and most preferably about 100 mg to about 3 g/day. Tablets or other unit dosage forms of presentation provided in discrete units may conveniently contain an amount which is effective at such dosage or as a multiple of the same, for instance, units containing about 5 mg to about 500 mg, usually from about 100 mg to about 500 mg.

The dose employed will depend upon a number of factors, including the age and sex of the subject, the precise disorder being treated, and its severity. Also the route of administration may vary depending upon the condition and its severity.

The invention will be further described in the following examples, which do not limit the scope of the invention described in the claims. The following examples illustrate the identification and characterization of genes differentially expressed in PNC cells.

*Genome-wide cDNA Microarray Analysis of Gene-Expression Profiles of Pancreatic Cancer Using Cancer and Normal Ductal Epithelial Cells Purely Selected by Laser Microdissection*

Tumor markers and targets for therapeutic intervention were identified by analyzing gene-expression profiles using a cDNA microarray representing 23,040 genes. Pancreatic ductal adenocarcinoma that has a characteristic of highly desmoplastic stromal reaction contained a low proportion of cancer cells in the tumor mass. Furthermore, normal duct epithelial cells from which the pancreatic carcinoma originates correspond to a few percent of the pancreas tissue. Therefore, cancer cells were purified from 18 pancreatic cancers by means of laser microbeam microdissection (LMM). Gene expression profiles were examined and compared with those of normal purified pancreatic ductal epithelial cells. These cell populations had been rendered homogenous (more than 95% purified cells). As a result, 259 genes were identified to be commonly up-regulated in pancreatic cancer cells; among them, the disease correlation and/or function of 64 (including 30 ESTs) genes were not known prior to the invention. The up-regulated genes included ones that were previously reported to be over-expressed in pancreatic cancer, such as interferon-induced transmembrane protein 1 (IFITM1), plasminogen activator, urokinase (PLAU), prostate stem cell antigen (PSCA), S100 calcium binding protein P (S100P), and baculoviral IAP repeat-containing 5 (BIRC5). 346 genes were identified as being commonly down-regulated in pancreatic cancer cells. Of them, 211 genes were functionally characterized and included some tumor suppressor genes such as AXIN1 up-regulated 1 (AXUD1), deleted in liver cancer 1 (DLC1), growth arrest and DNA-damage-inducible, beta (GADD45B), p53-inducible p53DINP1 (p53DINP1).

The present gene expression profile represents a highly accurate cancer reference; because a number of limitations of earlier methods were overcome. First, a microarray analysis using clinical samples has been difficult, because of various cellular components are present in the normal as well as cancer tissues. In particular, pancreatic ductal adenocarcinoma that has a characteristic of highly desmoplastic stromal reaction contained a low proportion of cancer cells in the tumor mass. Furthermore, the normal pancreas is mostly constituted from acinar cells and islets that accounted for more than 95% of whole pancreas, and normal duct epithelial cells from which the pancreatic carcinoma originates correspond to a few % of the pancreas. Therefore, the analysis of gene-expression profiles using bulk pancreatic cancer and normal whole pancreatic tissues is significantly influenced by the proportions of cells mixed in the tissues examined; proportional differences of acinar cells, islet cells, fibroblasts, and inflammatory cells may mask the significant increase or decrease of genes that are involved in pancreatic carcinogenesis. Hence, in this study, LMM systems were used to purify cancer and normal epithelial cells from surgical specimens to a high degree of purity (95% or higher). Because it is possible to microdissect even a single cell with LMM, this technology is critical for an accurate microarray analysis of pancreatic cancer specimens. To evaluate the purifity of micordissected pancreatic cancer and normal ductal cells, the expression profile of AMY1A gene which is known to be expressed specifically in acinar cells were analyzed. As a result, the proportion of contaminating acinar cells in the dissected normal pancreatic ductal epithelial cells was estimated to be smaller than 0.29%. In addition to AMY1A, expression levels of other genes that were highly expressed in acinar cells like elastase 1, trypsin 1, and pancreatic lipase were examined. Similar results were obtained, indicating that the purifity of cell populations by the LMM technique was as high as 99.2%-99.7%.

Second, the quality of extracted RNA from clinical tissue, particularly from pancreas, is one of the most important factors. Pancreas is known to be RNase-rich organ and degradation of RNA occurs very rapidly. In this study, the quality of the extracted RNA from the specimen was examined by visualization of 28S and 18S ribosomal RNAs using denaturing agarose gel electrophoresis. Following electrophoretic analysis, samples in which bands corresponding to two ribosomal RNAs were clearly observed were selected. For example, 18 cases (32%) were selected from the 56 surgically-ressected cases, i.e., many were not included in the analysis due to the poor quality of RNA.

Careful purification of cancer cells as well as normal epitherial ductal cells, subsequent RNA isolation, and cDNA microarray analysis identified 259 genes whose expression was commonly up-regulated (genes which were able to obtain expression data in more than 50% cancer cases and whose expression ratio(Cy5/Cy3 intensity ratio) was more than 5.0 and the genes which were able to calculate in 33 to 50% cases and which expressed the expression ratio of more than 5.0 in all of that cases were also evaluated)

Over 90% of the gene expression profile of pancreatic cancer was different from previous pancreatic cancer expression profiles, because the expression data was obtained by testing highly purified cell populations obtained from patient tissues using laser dissection techniques.

The profiles obtained and described herein represent an improvement over earlier profiles, because they were obtained by analyzing highly purified populations of cancerous cells (pancreatic ductal adenocarcinoma) and compared to a highly purified population of the most relevant normal control, i.e., normal duct epithelial cells. Earlier methods and profiles where hampered by a high percentage of contaminating cells, which reduced the accuracy and reliability of earlier profiles. This present profile is the first one of precise and genome-wide gene expression profiles in large-scale pancreatic cancer. These data identify molecular targets for therapeutic modulation for the treatment of pancreatic cancer and specific novel tumor markers for early and accurate diagnosis of the cancer or a precancerous condition.

**EXAMPLE 1: PREPARATION OF TEST SAMPLES**

Tissue obtained from diseased tissue (e.g., epithelial cells from PNC) and normal tissues was evaluated to identify genes which are differently expressed or a disease state, e.g., PNC. The assays were carried out as follows.

Patients, tissue samples, and laser microdissection

Tissue samples of pancreatic cancer (n =18) and normal pancreas (n = 7) were obtained from surgical specimens from patients with informed consent. All pancreatic cancer tissues had histologically confirmed invasive ductal carcinoma. Clinicopathological features of the patients we used in this study are summarized in Table 1. Since almost all pancreatic ductal cells from corresponding normal tissue blocks showed dysplastic changes mostly because of downstream ductal obstruction, ductal cells for only 4 of the 18 pancreatic cancer tissues were suitable to use as normal controls. Hence, additional control ductal cells were obtained from 3 normal pancreas tissues from patients who were operated by cholangiocarcinoma, duodenal leiomyosarcoma, or ampullary tumor. In each case, the specimens were harvested immediately after surgical resection and were embedded in TissueTek OCT medium (Sakura, Tokyo, Japan) before storage at -80˚C. The frozen tissues were cut to 8-μm sections using a cryostat (Sakura, Tokyo, Japan) and then stained with Hematoxylin and Eosin, and check the histological state. Pancreatic carcinoma cells and normal pancreatic ductal epithelial cells were isolated selectively using the EZ cut system with pulsed ultraviolet narrow beam focus laser (SL Microtest GmbH, Germany) in accordance with the manufacturer's protocols. After microdissection, 7 normal cases were mixed to make a "universal control of normal pancreatic ductal epithelial cells ", that was used as a control for all 18 cancer samples.

Table1. Clinocopathological features of the pancreatic cancer patients

| Patient No. | Age | Sex | location | pT | pN | M | Stage | Histopathology | Status |
|---|---|---|---|---|---|---|---|---|---|
| 1 | 74 | M | pb | 1 | 0 | 0 | I | por | alive |
| 2 | 56 | F | ph | 2 | 0 | 0 | I | pap | alive |
| 3 | 61 | M | ph | 1 | 0 | 0 | I | mod | alive |
| 4 | 75 | M | ph | 2 | 0 | 0 | I | pap | alive |
| 5 | unknown | M | ph | 3 | 1 | 0 | III | mod | alive |
| 6 | unknown | M | pb | 4 | 0 | 0 | IVA | well | alive |
| 7 | 77 | M | phpb | 4 | 0 | 0 | IVA | mod | dead |
| 8 | 73 | F | ph | 4 | 1 | 0 | IVA | mod | dead |
| 9 | 75 | M | pb | 4 | 1 | 0 | IVA | adenoscc | alive |

(continued)

| Table1. Clinocopathological features of the pancreatic cancer patients | | | | | | | | | |
|---|---|---|---|---|---|---|---|---|---|
| Patient No. | Age | Sex | location | pT | pN | M | Stage | Histopathology | Status |
| 10 | 61 | M | ph | 4 | 0 | 0 | IVA | mod | alive |
| 11 | 64 | M | ph | 4 | 1 | 0 | IVA | well | dead |
| 12 | 61 | M | ph | 4 | 1 | 0 | IVA | mod | dead |
| 13 | 65 | M | ph | 4 | 1 | 0 | IVA | mod | dead |
| 14 | 46 | M | ph | 4 | 1 | 0 | IVA | mod | dead |
| 15 | 59 | M | ph | 4 | 0 | 0 | IVA | mod=por | alive |
| 16 | 58 | M | ph | 4 | 1 | 0 | IVA | mod | dead |
| 17 | 74 | F | pb | 4 | 1 | 1 | IVB | mod | dead |
| 18 | 69 | F | ph | 4 | 1 | 1 | IVB | mod | dead |
| Clinical stage was judged according to the UICC TNM classification<br>location: Tumor location, ph: pancreas head, pb: pancreas body<br>All patients were Invasive ductal adenocarcinomas, well: Tubular adenocarcinoma well differentiated type<br>mod: Tubular adenocarcinoma mderately type, por: Tubular adenocarcinoma poorly differentiated type, pap: Papillary adenocarcinoma, adenoscc: Adenosquamous carcinoma | | | | | | | | | |

<u>Isolation of pancreatic cancer cells and normal pancreatic ductal epithelial cells by using LMM</u>

To obtain precise expression profiles of pancreatic cancer cells, LMM was used to purify cancer cells and avoid contamination of non-cancerous cells. In addition, since pancreatic cancer originates from pancreatic ductal cells, pancreatic ductal epithelial cells were used as controls. The great majority of cells in pancreas are acinar cells, it was determined that the use of the entire pancreas was inappropriate for screening genes associated with pancreatic carcinogenesis. As shown in Fig. 1, representative cancer cases (Fig. 1A and 1B), and normal pancreatic duct (Fig. 1C and 1D) were microdissected. Fig. 1A and 1B showed a well-differentiated type and a scirrhous type of invasive ductal adenocarcinoma, and the proportion of cancer cell was about 30% and 10%, respectively. After isolation of pancreatic cancer cells by LMM, we estimated that the proportion of pancreatic cancer cells used in this study was at least 95%.

The proportion of acinar cells contaminated was examined in the microdissected normal pancreatic ductal epithelial cells which used as universal control (Fig 1C and 1D). The signal intensity of AMY1A gene was examined that is known to be expressed exclusively in normal acinar cells. The signal intensity of whole pancreatic tissue was investigated in which >90% of the cells are acinar cells, the ratio of the average signal intensity of the pancreatic amylase gene to that of ACTB was approximately 96.7, whereas the ratio of that in microdissected normal pancreatic ductal epithelial cells in this study was calculated approximately 0.28. This result showed the proportion of contaminating acinar cells in the microdissected normal pancreatic ductal epithelial cells was estimated to be 0.29% in average (Fig. 1). Furthermore, the extent of contamination of acinar cells was determined in the microdissected normal pancreatic ductal epithelial cells. Pancreatic amylase gene (AMY1A) that is expressed exclusively in pancreatic acinar cells was used to evaluate the proportion of the acinar cells in microdissected normal pancreatic ductal epithelial cells. Each intensity was normalized by intensity of β-actin gene (ACTB) as follows;

(Ratio A) the AMY1A /ACTB intensity ratio in whole pancreas (most of the cells correspond to acinar cells) = 96.74

(Ratio B) the AMY1A/ACTB intensity ratio in microdissected normal ductal epithelial cells = 0.28

$$\text{Contamination percentage (\%)} ;(\text{Ratio B}) /(\text{Ratio A}) \times 100 = 0.29\%$$

Extraction of RNA and T7-based RNA amplification

Total RNAs were extracted from each sample of laser-microdissected cells into 350 μl of RLT lysis buffer (QIAGEN, Hilden, Germany). The extracted RNAs were treated for 30 minutes at room temperature with 30 units of DNase I (Roche, Basel, Switzerland) in the presence of 1 unit of RNase inhibitor (TOYOBO, Osaka, Japan) to remove any contaminating genomic DNA. After inactivation at 70° C for 10 min, the RNAs were purified with an RNeasy Mini Kit (QIAGEN) according to the manufacturer's recommendations. All of the DNase 1-treated RNAs were subjected to T7-based RNA amplification as described previously. Two rounds of amplification yielded 50-100μg of aRNA from each sample. A 2.5μg aliquot of aRNA from cancer and normal pancreatic duct epithelial cells was labeled with Cy5-dCTP or Cy3-dCTP, respectively, by a protocol described elsewhere. The hybridization, washing, and scanning were carried out according to the methods described previously (11).

Preparation of the cDNA microarray

A genome-wide cDNA microarray with 23,040 cDNAs selected from the UniGene database (build # 131) of the National Center for Biotechnology Information (NCBI) was constructed. Briefly, the cDNAs were amplified by RT-PCR using poly(A)$^+$ RNA isolated from various human organs as templates; the lengths of the amplicons ranged from 200 to 1,100 bp that did not contain repetitive or poly(A) sequences. The cDNA microarray system was constructed essentially as described previously (11).

Acquisition of data

Signal intensities of Cy3 and Cy5 from the 23,040 spots were quantified and analyzed by substituting backgrounds, using Array Vision software (Imaging Research, Inc., St. Catharines, Ontario, Canada). Subsequently, the fluorescent intensities of Cy5 (tumor) and Cy3 (control) for each target spot were adjusted so that the mean Cy3/Cy5 ratio of the 52 housekeeping genes was equal to one. Because the data derived from low signal intensities are less reliable, a cut-off value for signal intensities was determined on each slide and excluded genes from further analysis when both Cy3 and Cy5 dyes provided signal intensities lower than the cut-off as described previously (12). For other genes we calculated the Cy5/Cy3 ratio using raw data of each sample.

Semi-Quantitative RT-PCR

The 12 highly up-regulated genes were selected and examined their expression levels by applying the semi-quantitative RT-PCR experiments. A 3-μg aliquot of aRNA from each sample was reversely-transcribed for single-stranded cDNAs using random primer (Roche) and Superscript II (Life Technologies, Inc.). Each cDNA mixture was diluted for subsequent PCR amplification with the same primer sets that were prepared for the target DNA or tubulin, alpha-specific reactions. The primer sequences are listed in Table 2. Expression of tubulin-alpha served as an internal control. PCR reactions were optimized for the number of cycles to ensure product intensity within the linear phase of amplification.

Table 2 Primer sequences for semi-quantitarive RT-PCR experiments

| PNC Assign ment | Acce ssion No. | Sym bol | Forward primer | SE Q ID NO | Reverse primer | SE Q ID NO |
|---|---|---|---|---|---|---|
| 12 | AA9 1682 6 | *APP* | 5'-CTGCTGGTCTTCAATTACCAAG-3' | No. I | 5'-CTCATCCCCTTATATTGCCACTT-3' | No. 2 |
| 13 | L206 88 | *ARH GDI B* | 5'-CTCCCTCTGATCCTCCATCAG-3' | No. 3 | 5'-TCTTGTTCTCTTGTGTCGTTTACAG-3' | No. 4 |
| 15 | L242 03 | *ATD C* | 5'-CATTCTCTCTGGCGATGGAGTG-3' | No. 5 | 5'-ACCAATGGTTTATTCCAAAGGG-3' | No. 6 |
| 16 | U514 78 | *ATP1 B3* | 5'-CAGTGTACAGTCGCCAGATAG-3' | No. 7 | 5'-TCCTCACATACAGAACTTCTCCAC-3' | No. 8 |
| 19 | U752 85 | *BIRC 5* | 5'-CTCCCTCAGAAAAAGGCAGTG-3' | No. 9 | 5'-GAAGCTGTAACAATCCACCCTG-3' | No. 10 |
| 22 | AF06 8760 | *BUB 1B* | 5'-AGCTAGGCAATCAAGTCTCAC-3' | No. 11 | 5'-AGGGAAAAGTAGAGACAAATGGG-3' | No. 12 |
| 33 | ABO 1153 6 | *CEL SR3* | 5'-AAGCAGCTTCCTGGGAGATT-3' | No. 13 | 5'-ACGGAACAATTTACACAGACAGG-3' | No. 14 |
| 35 | X549 41 | *CKS 1* | 5'-ACTATTCGGACAAATACGACGAC-3' | No. 15 | 5'-CACTGTTTGAATGTGCTGGTAAC-3' | No. 16 |
| 36 | X549 42 | *CKS* 2 | 5'-CAAGCAGATCTACTACTCGGACAA-3' | No. 17 | 5'-CAGTAACCTACTTGCAGTTGCATT-3' | No. 18 |
| 48 | AA5 7995 9 | *CYP 2S1* | 5'-CACCCTGATTCTACCAAATGC-3' | No. 19 | 5'-CCTTAAGTCACAAGGAACGTCAG-3' | No. 20 |

(continued)

| PNC Assign ment | Acce ssion No. | Sym bol | Forward primer | SE Q ID NO | Reverse primer | SE Q ID NO |
|---|---|---|---|---|---|---|
| 54 | M91 670 | *E2-EPF* | **5'-TCTGCTCACAGAGATCCACG-3'** | No. 21 | **5'-TTAGAGACAGAGTTGGAGGGAGG-3'** | No. 22 |
| 56 | U326 45 | *ELF4* | **5'-AGAAATGTCAGCCACGGAAAC-3'** | No. 23 | **5'-AAAGGCACTTTAATGCCAACTG-3'** | No. 24 |
| 57 | AF01 0314 | *ENC 1* | **5'-CGATATAGGCATTTGGTCTCAC-3'** | No. 25 | **5'-TTTCTCTTCATTAGACTTGGCCTCT-3'** | No. 26 |
| 59 | L366 45 | *EPH A4* | **5'-GAAGGCGTGGTCACTAAATGTAA-3'** | No. 27 | **5'-CTTTAATTTCAGAGGGCGAAGAC-3'** | No. 28 |
| 61 | AI62 7919 | *Evi-1* | **5'-GCAAGCTTGTGCGATGTTATGT-3'** | No. 29 | **5'-CTCCTCCCATAGTAATGCACTGA-3'** | No. 30 |
| 63 | L167 83 | *FOX M1* | **5'-GATGGATGCAACTGAAGCAGAG-3'** | No. 31 | **5'-GTCCACCTTCGCTTTTATTGAGT-3'** | No. 32 |
| 73 | AA6 5219 7 | *GW1 12* | **5'-GAAAATCTGATGGCAGTGACAA-3'** | No. 33 | **5'-AAGGTTTCCAACTACTGCACTGA-3'** | No. 34 |
| 74 | J045 01 | *GYS1* | **5'-TGCCCACTGTGAAACCACTAG-3'** | No. 35 | **5'-CATCTCATCTCCGGACACACT-3'** | No. 36 |
| 77 | D164 31 | *HDG F* | **5'-TATCCCAGCTGCCTAGATTC-3'** | No. 37 | **5'-GAGTCTTCCCAAGCATCCTATTT-3'** | No. 38 |
| 83 | M16 937 | *HOX B7* | **5'-GTACCTATAGGAAAGTCTGTC-3'** | No. 39 | **5'-AACACGCGAGTGGTAGGTTTT-3'** | No. 40 |

(continued)

| PNC Assign ment | Acce ssion No. | Sym bol | Forward primer | SE Q ID NO | Reverse primer | SE Q ID NO |
|---|---|---|---|---|---|---|
| 84 | AA4 9586 8 | *hPA D-colon y10* | 5'-CACTGAGCCAACTACTGTCACTG-3' | No. 41 | 5'-CTTCCTACCCACAGCTCTTTCTC-3' | No. 42 |
| 102 | U637 43 | *KNS L6* | 5'-ACTCTAGGACTTGCATGATTGCC-3' | No. 43 | 5'-TCCTCTAGGACTCTAGGGAGACA-3' | No. 44 |
| 103 | U703 22 | *KPN B2* | 5'-TCTTGGAGACTATAAGGGAGCC-3' | No. 45 | 5'-TTTTGCTTCTTCACATCCACTG-3' | No. 46 |
| 115 | X577 66 | *MMP 11* | 5'-GCACTGAAGCAAGGGTGCTG-3' | No. 47 | 5'-GACAGGATTGAGGTATGTTGCAG-3' | No. 48 |
| 120 | X132 93 | *MYB L2* | 5'-TCCTGAGGTGTTGAGGGTGTC-3' | No. 49 | 5'-ATCCTAAGCAGGGTCTGAGATG-3' | No. 50 |
| 125 | X043 71 | *OAS 1* | 5'-TTTCAGGATCAGTTAAATCGCC-3' | No. 51 | 5'-GGCCTGGCTGAATTACCCATG-3' | No. 52 |
| 127 | U657 85 | *ORP 150* | 5'-GTTCTGCTCCTCCCAGACAG-3' | No. 53 | 5'-GCCCTAGCTCCTGCTACAGA-3' | No. 54 |
| 132 | D385 54 | *PCO LN3* | 5'-GCTCACTGCGTTTGGTTTTC-3' | No. 55 | 5'-CAGCATTCTAGGAGAAAGGTGAA-3' | No. 56 |
| 141 | AA9 3198 1 | *PPM 1B* | 5'-CTGTAACGTTTTCCTGAAGCTGT-3' | No. 57 | 5'-TCAGTACAGGGTTGGATCAGAGT-3' | No. 58 |
| 143 | AF04, 4588 | *PRC 1* | 5'-GTGCCTACTTTGCCTGAGTTC-3' | No. 59 | 5'-CAGGACACGTACTGTATGAGGTAAA-3' | No. 60 |

(continued)

| PNC Assign ment | Acce ssion No. | Sym bol | Forward primer | SE Q ID NO | Reverse primer | SE Q ID NO |
|---|---|---|---|---|---|---|
| 149 | AF04 3498 | *PSC A* | 5'-GACCATGTATGTTTGCACCC-3' | No. 61 | 5'-AACTCACGTCAACTCTTGTCCTC-3' | No. 62 |
| 152 | M77 836 | *PYC R1* | 5'-ATCCCAAGTCCAGCGTGAAG-3' | No. 63 | 5'-TCCACTATTCCACCCACAGTAAC-3' | No. 64 |
| 155 | X646 52 | *RBM S1* | 5'-CTGTCGAGACGTCTAATGACC-3' | No. 65 | 5'-TTACTAAAATAAACCTGTTCGGGGG-3' | No. 66 |
| 157 | AA3 1652 5 | *REG1 V* | 5'-CCAGTAGTGGCTTCTAGCTC-3' | No. 67 | 5'-GAAAAACAAGCAGGAGTTGAGTG-3' | No. 68 |
| 164 | AA3 0806 2 | *S100 P* | 5'-GCATGATCATAGACGTCTTTTCC-3' | No. 69 | 5'-GATGAACTCACTGAAGTCCACCT-3' | No. 70 |
| 169 | AF02 9082 | *SFN* | 5'-GAGCGCACCTAACCACTGGTC-3' | No. 71 | 5'-TGAGTGTCACAGGGGAACTTTAT-3' | No. 72 |
| 170 | AA6 3959 9 | *SLC1 2A2* | 5'-AACCGAAGTCTCCATACACG-3' | No. 73 | 5'-GTTCGTGGGAATCATCAGAG-3' | No. 74 |
| 173 | K031 95 | *SLC2 A1* | 5'-AACCGAAGTCTCCATACACG-3' | No. 75 | 5'-GTTCGTGGGAATCATCAGAG-3' | No. 76 |
| 178 | M32 313 | *SRD 5A1* | 5'-TCTGTAACAATAACAAGACC-3' | No. 77 | 5'-CCAGATGAGATGATAAGGCAAAG-3' | No. 78 |
| 180 | M81 601 | *TCE A1* | 5'-TGTCCCAAGTCTTATTTGCTGA-3' | No. 79 | 5'-GCAACAGTGGCCTTTAAAGTATG-3' | No. 80 |
| 184 | K025 81 | *TK1* | 5'-GTAATTGTGGCTGCACTGGAT-3' | No. 81 | 5'-ATTTCATAAGCTACAGCAGAGGC-3' | No. 82 |

(continued)

| PNC Assign ment | Acce ssion No. | Sym bol | Forward primer | SE Q ID NO | Reverse primer | SE Q ID NO |
|---|---|---|---|---|---|---|
| 188 | U733 79 | *UBC H10* | 5'-ACACACATGCTGCCGAGCTC-3' | No. 83 | 5'-TAATATACAAGGGCTCAACCGAG-3' | No. 84 |
| 196 | AA5 8194 0 | *WHS C1* | 5'-CCTATGAGTGTAGTTGATGAC-3' | No. 85 | 5'-CAACTGGCAAGTCTCAACTCTCT-3' | No. 86 |
| 198 | AA7 | *FLJ1* | 5'- | No. | 5'- | No. |
| | 0915 5 | *0134* | TCCAGATGGATTTGTCCTGTATC-3' | 87 | TAGTAGCAAGCCCAGTAACCTTG-3' | 88 |
| 199 | AA8 0663 0 | *FLJ1 0540* | 5'-GCTTACCATTGAAACTTAACCCC-3' | No. 89 | 5'-CTCATTTACAGTAGCCCAGTGGT-3' | No. 90 |
| 203 | AA9 1881 1 | *FLJ2 0225* | 5'-GACTTCCACAATGAACAGGGTAA-3' | No. 91 | 5'-ATTGGAATAAGAGGAACAGGAGC-3' | No. 92 |
| 208 | D146 57 | *KIAA 0101* | 5'-CCAATTAGCTTTGTTGAACAGGC-3' | No. 93 | 5'-GGCAGCAGTACAACAATCTAAGC-3' | No. 94 |
| 217 | R397 94 | *KIAA 1624* | 5'-CAGTGCTACACCCACTTCTTG-3' | No. 95 | 5'-ATACCACCAATGGTTCTGCTATG-3' | No. 96 |
| 218 | AA4 3404 5 | *KIAA 1808* | 5'-CTCATCTTTGAAGCCAGCAG-3' | No. 97 | 5'-GACTCACAGGCAGGAACATC-3' | No. 98 |
| 225 | AA5 2311 7 | *FLJ2 1504* | 5'-GGATAGCTGGGGCATTTGTCTAG-3' | No. 99 | 5'-TCCATAAAAGAGTTTGGCAGTC-3' | No. 100 |
| 231 | AA7 8933 2 | *VAN GL1* | 5'-GAGTTGTATTATGAAGAGGCCGA-3' | No. 101 | 5'-ATGTCTCAGACTGTAAGCGAAGG-3' | No. 102 |

(continued)

| PNC Assign ment | Acce ssion No. | Sym bol | Forward primer | SE Q ID NO | Reverse primer | SE Q ID NO |
|---|---|---|---|---|---|---|
| 234 | AI34 9804 | *EST* | 5'-GTAGATGTGGGGACAACAGAGAG-3' | No. 103 | 5'-TTTAAAGTCACCTTAGGTTGGGG-3' | No. 104 |
| 239 | AA8 0611 4 | *EST* | 5'-CACCTATCCCTATTACCTGACCC-3' | No. 105 | 5'-TCTGAGGGTTTACATTGACGACT-3' | No. 106 |
| 242 | AA4 1956 8 | *EST* | 5'-GAGTCCAGGTAAGTGAATCTGTCC-3' | No. 107 | 5'-ATTTCCACCGAGACCTCTCATC-3' | No. 108 |
| 245 | AA5 7018 6 | *EST* | 5'-GTCTATCTGTGCTGGAACCTGAG-3' | No. 109 | 5'-GTGTAGGTGAGTGCTTTCTCCA-3' | No. 110 |
| 253 | AA8 3032 6 | *EST* | 5'-ACTCCCGAGTAAATCATAGAGCC-3' | No. 111 | 5'-GACTGTTTCTACTCCAGAGGGGT-3' | No. 112 |
| 254 | AI24 0520 | *FXY D3* | 5'-AAAGCTGATGAGGACAGACCAG-3' | No. 113 | 5'-GGCAGAGGCACAATCATTTTAG-3' | No. 114 |
| 259 | AI02 7791 | *EST* | 5'-TGGTGTCTTTCTACCATTCAAGG-3' | No. 115 | 5'-AAAAGGCTAGTCCCCTTCTACCT-3' | No. 116 |
| | AF14 1347 | *TUB A* | 5'-CTTGGGTCTGTAACAAAGCATTC-3' | No. 117 | 5'-AAGGATTATGAGGAGGTTGGTGT-3' | No. 118 |

Accession numbers and gene symbols were retrieved from the Unigene Databases (build#131).

**EXAMPLE 2: IDENTIFICATION OF PNC — ASSOCIATED GENES**

The up- or down-regulated genes were identified common to pancreatic cancer using following criteria; 1) genes which were able to obtain expression data in more than 50% cancer cases, and 2) genes whose expression ratio was more than 5.0 in pancreatic cancer cells (defined as up-regulated genes) or genes whose expression ratio was under 0.2 (defined as down-regulated genes) in more than 50% of informative cases. Moreover, 3) the genes which were able to calculate in 33 to 50% cases and which expressed the expression ratio of more than 5.0 in all of that cases were also evaluated as up-regulated genes.

**IDENTIFICATION OF GENES WITH CLINICALLY RELEVANT EXPRESSION PATTERNS IN PNC CELLS**

The expression of approximately 23,000 genes in in 18 pancreatic cancer patients was examined using cDNA microarray. Individual data were excluded when both Cy5 and Cy3 signals were under cut-off values. Two hundred fifty-nine up-regulated genes were identified whose expression ratio was more than 5.0 in PNC cells (*see* Table 3). 167 of them were expressed greater than 10-fold comparing to the normal ductal cells. Three hundred forty-six down-regulated genes whose expression ratio was less than 0.2 were identified (*see* Table 4).

Among the up-regulated genes, interferon induced transmembrane protein 1 (IFITM1), plasminogen activator, urokinase (PLAU), prostate stem cell antigen (PSCA), S 100 calcium binding protein P (S100P), RNA binding-motif single-stranded interacting protein 1 (RBMS1), and baculoviral IAP repeat-containing 5 (BIRC5), have been reported to be overexpressed in pancreatic cancer (5, 6). Furthermore, these up-regulated genes included ones encoding proteins involved in the signal transduction pathway, transcriptional factors, cell cycle, and cell adhesion (Table 5).

Significantly overexpressed genes have diagnostic potential, and of them which were critical for tumor growth have also therapeutic potential. Specifically, genes such as regenerating gene type IV (REGIV), ephrin type-A receptor 4 precursor (EphA4), and vang (van gogh, Drosophila)-like 1 (VANGL1), are useful as a potential molecular target for new therapeutic agents.

REGIV was over-expressed in all informative pancreatic cancer cases, and the overexpression was confirmed in 7 of the 12 pancreatic cancer cases by semi-quantitative RT-PCR. Since REGIV protein was thought to be a secreted protein from the amino-acid sequences and in fact its secretion was detected in the culture medium of HT29-5M12 cells (22), it is a candidate as tumor marker.

EphA4 was indicated to be overexpressed in 12 of the 14 informative pancreatic cancer cases in the microarray, and confined in 9 of the 12 cases were examined by semi-quantitative RT-PCR. EphA4 is known to be a membrane receptor belonging to the ephrin family, which contains an intracellular tyrosine kinase catalytic domain (23). Involvement of EphA4 in any human cancer has not been reported. However, its nature of the cytoplasmic membrane receptor protein with possible tyrosine kinase activity as well as high level expression in cancer cells suggest that EphA4 is a candidate gene for therapeutic agents.

VANGL1 was over-expressed if all of the informative pancreatic cancer cases in the microarray data, and its high expression was also confirmed in 9 of the 12 cases by semi-quantitative RT-PCR. VANGL1, which contained four putative transmembrane domains, was expressed specifically in testis and ovary among 29 normal tissues examined (4). This gene was also highly and frequency transactivated in hepatocellular carcinoma. Since the enforced reduction of this gene expression in hepatocellular carcinomas induced apoptosis (4), this gene product is a good candidate for development of novel anti-cancer drugs. Among the genes that were functionally highly overexpressed in pancreatic cancer such as the above mentioned genes, those whose products are putative membranous or secreted are of interest for potential as novel anti-cancer drugs or as serological diagnostic markers for early detection.

To confirm the reliability of the expression profiles indicated by microarray analysis, semi-quantitative RT-PCR experiments were performed. Other 55 genes whose cancer/normal ratios were highest among the informative genes, APP, ARHGDIB, ATDC, ATP1B3, BIRC5, BUB1B, CELSR3, CKS1, CKS2, CYP2S1, E2-EPF, ELF4, ENC1, Evi-1, FOXM1, GW112, GYS1, HDGF, HOXB7, hPAD-colony10, KNSL6, KPNB2, MMP11, MYBL2, OAS1, ORP150, PCOLN3, PPM1B, PRC1, PSCA, PYCR1, RBMS1, S100P, SFN, SLC12A2, SLC2A1, SRD5A1, TCEA1, TK1, UBCH10, WHSC1, FLJ10134, FLJ10540, FLJ20225, KIAA0101, KIAA1624, KIAA1808, FLJ21504, FXYD3, and 6 ESTs (Accession No.AI349804, AA806114, AA419568, AA570186, AA830326, AI027791) were PCR-amplified and compared with the microarray data. As shown in Fig.2, the results of the cDNA microarray werehighly similar to those of the RT-PCR analysis in the great majority of the tested cases.

APP was confirmed whose over-expression in 10 of the 12 cases,
ARHGDIB was confirmed whose over-expression in 12 cases,
ATDC was confirmed whose over-expression in 10 of the 12 cases,
ATP1B3 was confirmed whose over-expression in 12 cases,
BIRC5 was confirmed whose over-expression in 12 cases,
BUB1B was confirmed whose over-expression in 12 cases,

CELSR3 was confirmed whose over-expression in 9 of the 12 cases,
CKS1 was confirmed whose over-expression in 7 of the 12 cases,
CKS2 was conformed whose over-expression in 11 of the 12 cases,
CYP2S1 was confirmed whose over-expression in 8 of the 12 cases,
E2-EPF was confirmed whose over-expression in 8 of the 12 cases,
ELF4 was confirmed whose over-expression in 11 of the 12 cases,
ENC1 was confirmed whose over-expression in 7 of the 12 cases,
Evi-1 was confirmed whose over-expression in 11 of the 12 cases,
FOXM1 was confirmed whose over-expression in 11 of the 12 cases,
GW112 was confirmed whose over-expression in 7 of the 12 cases,
GYS1 was confirmed whose over-expression in 10 of the 12 cases,
HDGF was confirmed whose over-expression in 10 of the 12 cases,
HOXB7 was confirmed whose over-expression in 6 of the 12 cases, hPAD-colony10 was confirmed whose over-expression in 6 of the 12 cases,
KNSL6 was confirmed whose over-expression in 12 cases,
KPNB2 was confirmed whose over-expression in 10 of the 12 cases,
MMP11 was confirmed whose over-expression in 10 of the 12 cases,
MYBL2 was confirmed whose over-expression in 11 of the 12 cases,
OAS1 was confirmed whose over-expression in 10 of the 12 cases,
ORP150 was confirmed whose over-expression in 8 of the 12 cases,
PCOLN3 was confirmed whose over-expression in 4 of the 12 cases,
PPM1B was confirmed whose over-expression in 3 of the 12 cases,
PRC1 was confirmed whose over-expression in 12 cases,
PSCA was confirmed whose over-expression in 6 of the 12 cases,
PYCR1 was confirmed whose over-expression in 9 of the 12 cases,
RBMS 1 was confirmed whose over-expression in 12 cases,
S100P was confirmed whose over-expression in 10 of the 12 cases,
SFN was confirmed whose over-expression in 9 of the 12 cases,
SLC12A2 was confirmed whose over-expression in 5 of the 12 cases,
SLC2A1 was confirmed whose over-expression in 11 of the 12 cases,
SRD5A1 was confirmed whose over-expression in 8 of the 12 cases,
TCEA1 was confirmed whose over-expression in 8 of the 12 cases,
TK1 was confirmed whose over-expression in 10 of the 12 cases,
UBCH10 was confirmed whose over-expression in 10 of the 12 cases,
WHSC1 was confirmed whose over-expression in 8 of the 12 cases,
FLJ10134 was confirmed whose over-expression in 8 of the 12 cases,
FLJ10540 was confirmed whose over-expression in 11 of the 12 cases,
FLJ20225 was confirmed whose over-expression in 5 of the 12 cases,
KIAA0101 was confirmed whose over-expression in 12 cases,
KIAA1624 was confirmed whose over-expression in 9 of the 12 cases,
KIAA1808 was confirmed whose over-expression in 8 of the 12 cases,
FLJ21504 was confirmed whose over-expression in 11 of the 12 cases,
FXYD3 was confirmed whose over-expression in 9 of the 12 cases, and.
Accession No.AI349804 was confirmed whose over-expression in 11 of the 12 cases,
AA806114 was confirmed whose over-expression in 8 of the 12 cases,
AA419568 was confirmed whose over-expression in 9 of the 12 cases,
AA570186 was confirmed whose over-expression in 6 of the 12 cases,
AA830326 was confirmed whose over-expression in 12 cases,
AI027791 was confirmed whose over-expression in 6 of the 12 cases.

These data verified the reliability of our strategy to identify commonly up-regulated genes in PNC cells.

Among the 346 down-regulated genes in pancreatic cancer cells, functions of 211 genes are characterized. These included genes that have been reported to be invoved in growth suppression (24,27,28,29), such as AXIN1 up-regulated 1 (AXUD 1), Deleted in liver cancer 1 (DLC1), growth arrest and DNA-damage-inducible, beta (GADD45B), and P53-inducible p53DINP1 (p53DINP1).

The down-regulated genes are likely to have a tumor suppressive function. Although the representative tumor suppressor genes for pancreatic cancer such as SMAD4, TP53, INK4A, and BRCA2 (24, 25) were not observed in down-regulated gene list, other genes that were reported to be involved in tumor suppression or apoptosis, such as, AXIN1 up-regulated 1(AXUD1), deleted in liver cancer 1 (DLC1), growth arrest and DNA-damage-inducible, beta (GADD45B), p53-inducible p53DINP1 (p53DINP1) were included in these data.

AXUD1, a nuclear protein, is induced in response to elevation of axin that is a key mediator of the Wnt-signalling pathway and is important in axis formation in early development. Dysfunction or down-regulation of the Wnt-signaling pathway is observed in human tumors, suggesting that this gene product has a tumor suppressor function (26, 27). Hence, these data imply that down-regulation of AXUD1 might lead to down-regulation of this signaling pathway and then lead to pancreatic carcinogenesis. Deleted in liver cancer 1 (DLC1) was suggested to be a candidate tumor suppressor gene for human liver cancer, as well as for prostate, lung, colorectal, and breast cancers. DLC1 shares high sequence similarity with the rat p122 RhoGap that negatively regulates the Rho GTPases. Hence, down-regulation of DLC 1 is considered to result in the constitutive activation of the Rho-Rho-kinase pathway and subsequent oncogenic malignant transformation (28, 29).

Table3. A list of up-regulated genes

| PNC Assign ment | Accession No. | Symbol | Gene Name |
|---|---|---|---|
| 1 | V00478 | *ACTB* | actin, beta |
| 2 | D26579 | *ADAM8* | a disintegrin and metalloproteinase domain 8 |
| 3 | D14874 | *ADM* | adrenomedullin |
| 4 | H78430 | *AHSG* | alpha-2-HS-glycoprotein |
| 5 | W92633 | *AIB3* | thyroid hormone receptor binding protein |
| 6 | AF024714 | *AIM2* | absent in melanoma 2 |
| 7 | X60673 | *AK3* | adenylate kinase 3 |
| 8 | AF047002 | *ALY* | transcriptional coactivator |
| 9 | AI341261 | *ANLN* | anillin (Drosophila Scraps homolog), actin binding protein |
| 10 | J03578 | *ANXA6* | annexin A6 |
| 11 | U81504 | *AP3B1* | adaptor-related protein complex 3, beta 1 subunit |
| 12 | AA916826 | *APP* | amyloid beta (A4) precursor protein (protease nexin-II, Alzheimer disease) |
| 13 | L20688 | *ARHGDIB* | Rho GDP dissociation inhibitor (GDI) beta |
| 14 | AF006086 | *ARPC3* | actin related protein 2/3 complex, subunit 3 (21 kD) |
| 15 | L24203 | *ATDC* | ataxia-telangiectasia group D-associated protein |
| 16 | U51478 | *ATP1B3* | ATPase, Na+/K+ transporting, beta 3 polypeptide |
| 17 | AA148566 | *ATP2B4* | ATPase, Ca++ transporting, plasma membrane 4 |
| 18 | W27948 | *ATP6S1* | ATPase, H+ transporting, lysosomal (vacuolar proton pump), subunit 1 |
| 19 | U75285 | *BIRC5* | baculoviral LAP repeat-containing 5 (survivin) |
| 20 | L13689 | *BMI1* | murine leukemia viral (bmi) oncogene homolog |
| 21 | W91908 | *BRAG* | B cell RAG associated protein |
| 22 | AF068760 | *BUB1B* | budding uninhibited by benzimidazoles 1 (yeast homolog), beta |
| 23 | AF028824 | *C19ORF3* | chromosome 19 open reading frame 3 |
| 24 | J04080 | *C1S* | complement component 1,s s subcomponent |
| 25 | M15082 | C2 | complement component 2 |
| 26 | AA600048 | *CALD1* | caldesmon 1 |
| 27 | AA621719 | *CAP-C* | chromosome-associated polypeptide C |
| 28 | AA557142 | *CD2AP* | CD2-associated protein |
| 29 | Z11697 | *CD83* | CD83 antigen (activated B lymphocytes, immunoglobulin superfamily) |
| 30 | H52870 | *CDC10* | CDC10 (cell division cycle 10, S. cerevisiae, homolog) |
| 31 | AA421724 | *CDC20* | CDC20 (cell division cycle 20, S. cerevisiae, homolog) |
| 32 | X63629 | *CDH3* | cadherin 3, type 1, P-cadherin (placental) |
| 33 | AB011536 | *CELSR3* | cadherin, EGF LAG seven-pass G-type receptor 3, flamingo (Drosophila) homolog |
| 34 | X95404 | *CFL1* | cofilin 1 (non-muscle) |
| 35 | X54941 | *CKS1* | CDC28 protein kinase 1 |

(continued)

| PNC Assign ment | Accession No. | Symbol | Gene Name |
|---|---|---|---|
| 36 | X54942 | *CKS2* | CDC28 protein kinase 2 |
| 37 | AA001074 | *CNNM4* | Cyclin M4 |
| 38 | AA977821 | *COL1A1* | collagen, type I, alpha 1 |
| 39 | J03464 | *COL1C2* | collagen, type I, alpha 2 |
| 40 | X14420 | *COL3A2* | collagen, type III, alpha 1 (Ehlers-Danlos syndrome type IV, autosomal dominant) |
| 41 | AI140851 | *COL6A1* | collagen, type VI, alpha 1 |
| 42 | J04823 | *COX8* | cytochrome c oxidase subunit VIII |
| 43 | AA523543 | *CRABP1* | cellular retinoic acid-binding protein 1 |
| 44 | AA905901 | *CRSP3* | cofactor required for Sp1 transcriptional activation, subunit 3 (130kD) |
| 45 | X16312 | *CSNK2B* | casein kinase 2, beta polypeptide |
| 46 | U16306 | *CSPG2* | chondroitin sulfate proteoglycan 2 (versican) |
| 47 | U40763 | *CYP* | Clk-associating RS-cyclophilin |
| 48 | AA579959 | *CYP2S1* | cytochrome P540 family member predicted from ESTs |
| 49 | AA863145 | *DAO* | D-amino-acid oxidase |
| 50 | AI287670 | *DDEF1* | Development and differentiation enhancing factor 1 |
| 51 | AI159886 | *DDX21* | DEAD/H (Asp-Glu-Ala-Asp/His) box polypeptide 21 |
| 52 | U90426 | *DDXL* | nuclear RNA helicase, DECD variant of DEAD box family |
| 53 | AA921756 | *DLA4* | diaphorase (NADH/NADPH) (cytochrome b-5 reductase) |
| 54 | M91670 | *E2-EPF* | ubiquitin carrier protein |
| 55 | AA457022 | *E2IG5* | hypothetical protein, estradiol-induced |
| 56 | U32645 | *ELF4* | E74-like factor 4 (ets domain transcription factor) |
| 57 | AF010314 | *ENC1* | ectodermal-neural cortex (with BTB-like domain) |
| 58 | AF027299 | *EPB41L2* | erythrocyte membrane protein band 4.1-like 2 |
| 59 | L36645 | *EPHA4* | EphA4 |
| 60 | AA983304 | *ERH* | enhancer of rudimentary (Drosophila) homolog |
| 61 | AI627919 | *Evi-1* | ecotropic viral integration site 1 |
| 62 | X02761 | *FN1* | fibronectin 1 |
| 63 | L16783 . | *FOXM1* | forkhead box M1 |
| 64 | M14333 | *FYN* | FYN oncogene related to SRC, FGR, YES UDP-N-acetyl- |
| 65 | N36998 | *GALNT2* | alpha-D-galactosamine:polypeptide N-acetylgalactosaminyltransferase 2 |
| 66 | AA418167 | *GATA3* | GATA-binding protein 3 |
| 67 | U78027 | *GLA* | galactosidase, alpha |
| 68 | AF040260 | *GMDS* | GDP-mannose 4,6-dehydratase |
| 69 | J03260 | *GNAZ* | guanine nucleotide binding protein (G protein), alpha z polypeptide |
| 70 | D63997 | *GOLGA3* | golgi autoantigen, golgin subfamily a, 3 |
| 71 | X62320 | *GRN* | granulin |
| 72 | D87119 | *GS3955* | GS3955 protein |
| 73 | AA652197 | *GW112* | differentially expressed in hematopoietic lineages |
| 74 | J04501 | *GYS1* | glycogen synthase 1 (muscle) |
| 75 | M60756 | *H2BFQ* | H2B histone family, member Q |
| 76 | AA608605 | *HCS* | cytochrome c |
| 77 | D16431 | *HDGF* | hepatoma-derived growth factor (high-mobility group protein 1-like) |
| 78 | X63187 | *HE4* | epididymis-specific, whey-acidic protein type, four-disulfide core |

(continued)

| PNC Assign ment | Accession No. | Symbol | Gene Name |
|---|---|---|---|
| 79 | AA714394 | *HMG2* | high-mobility group (nonhistone chromosomal) protein 2 |
| 80 | X92518 | *HMGIC* | high-mobility group (nonhistone chromosomal) protein isoform I-C |
| 81 | X06985 | *HMOX1* | heme oxygenase (decycling) 1 |
| 82 | N92060 | *HNRPL* | Heterogeneous nuclear ribonucleoprotein L |
| 83 | M16937 | *HOXB7* | homeo box B7 |
| 84 | AA495868 | *hPAD-colony10* | peptidylarginine deiminase type I |
| 85 | AF070616 | *HPCAL1* | hippocalcin-like 1 |
| 86 | AF064084 | *ICMT* | isoprenylcysteine carboxyl methyltransferase |
| 87 | AA328385 | *ICSBP1* | interferon consensus sequence binding protein 1 |
| 88 | AA573936 | *IDH2* | isocitrate dehydrogenase 2 (NADP+), mitochondrial |
| 89 | AI341760 | *IFI27* | interferon, alpha-inducible protein 27 |
| 90 | AI081175 | *IFITMI* | interferon induced transmembrane protein 1 (9-27) |
| 91 | X16302 | *IGFBP2* | insulin-like growth factor binding protein 2 (36kD) |
| 92 | M87789 | *IGHG3* | immunoglobulin heavy constant gamma 3 (G3m marker) |
| 93 | M87790 | *Iglλ* | immunoglobulin lambda locus |
| 94 | S74221 | *IK* | IK cytokine, down-regulator of HLA II |
| 95 | X59770 | *IL1R2* | interleukin 1 receptor, type II |
| 96 | J05272 | *IMPDH1* | IMP (inosine monophosphate) dehydrogenase 1 |
| 97 | AB003184 | *ISLR* | immunoglobulin superfamily containing leucine-rich repeat |
| 98 | M15395 | *ITGB2* | integrin, beta 2 |
| 99 | L38961 | *ITM1* | integral membrane protein 1 |
| 100 | AA574178 | *KAI1* | Kangai 1 |
| 101 | M55513 | *KCNA5* | potassium voltage-gated channel, shaker-related subfamily, member 5 |
| 102 | U63743 | *KNSL6* | kinesin-like 6 (mitotic centromere-associated kinesin) |
| 103 | U70322 | *KPNB2* | karyopherin (importin) beta 2 |
| 104 | J00269 | *KRT6A* | keratin 6A |
| 105 | X53305 | *LAP18* | leukemia-associated phosphoprotein p18 (stathmin) |
| 106 | AA742701 | *LCPI* | lymphocyte cytosolic protein 1 (L-plastin) |
| 107 | AA826336 | *LHFPL2* | lipoma HMGIC fusion partner-like 2 |
| 108 | U24576 | *LMO4* | LIM domain only 4 |
| 109 | AA555023 | *LOC51191* | cyclin-E binding protein 1 |
| 110 | AI299952 | *LOC51765* | serine/threonine protein kinase MASK |
| 111 | U89942 | *LOXL2* | lysyl oxidase-like 2 mannosyl (alpha,6-)-glycoprotein beta, |
| 112 | U15128 | *MGAT2* | 2-N-acetylglucosaminyltransferase |
| 113 | J03746 | *MGSTI* | microsomal glutathione S-transferase 1 myeloid/lymphoid |
| 114 | AA531437 | *MLLT4* | or mixed-lineage leukemia (trithorax (Drosophila) homolog); translocated to, 4 |
| 115 | X57766 | *MMP 11* | matrix metalloproteinase 11 (stromelysin 3) matrix |
| 116 | J05070 | *MMP9* | metalloproteinase 9 (gelatinase B, 92kD gelatinase, 92kD type IV collagenase) |
| 117 | AF034374 | *MOCS1* | molybdenum cofactor biosynthesis protein A; molybdenum cofactor biosynthesis protein C |
| 118 | M74905 | *MPG* | N-methylpurine-DNA glycosylase |
| 119 | AA458825 | *MTIF2* | mitochondrial translational initiation factor 2 |
| 120 | X13293 | *MYBL2* | v-myb avian myeloblastosis viral oncogene homolog-like 2 |
| 121 | D32002 | *NCBP1* | nuclear cap binding protein subunit 1, 80kD |
| 122 | AA729022 | *NCOA3* | nuclear receptor coactivator 3 |
| 123 | AF047434 | *NDUFS5* | NADH dehydrogenase (ubiquinone) Fe-S protein 5 (15kD) (NADH-coenzyme Q reductase) |

(continued)

| PNC Assign ment | Accession No. | Symbol | Gene Name |
|---|---|---|---|
| 124 | AA602490 | *NOP5/NOP58* | nucleolar protein NOP5/NOP58 |
| 25 | X04371 | *OAS1* | 2',5'-oligoadenylate synthetase 1 (40-46 kD) |
| 126 | M23204 | *OAT* | ornithine aminotransferase (gyrate atrophy) |
| 127 | U65785 | *ORP150* | oxygen regulated protein (150kD) |
| 128 | AI223298 | *P125* | Sec23-interacting protein p125 |
| 129 | M24486 | *P4HA1* | procollagen-proline, 2-oxoglutarate 4-dioxygenase (proline 4-hydroxylase), alpha polypeptide I |
| 130 | M80482 | *PACE4* | paired basic amino acid cleaving system 4 |
| 131 | L11370 | *PCDHI* | protocadherin 1 (cadherin-like 1) |
| 132 | D38554 | *PCOLN3* | procollagen (type III) N-endopeptidase |
| 133 | AA034069 | *PDK1* | pyruvate dehydrogenase kinase, isoenzyme 1 |
| 134 | AA586974 | *PI3* | protease inhibitor 3, skin-derived (SKALP) |
| 135 | M16750 | *PIM1* | pim oncogene |
| 136 | AA234962 | *PKP3* | plakophilin 3 |
| 137 | X02419 | *PLAU* | plasminogen activator, urokinase |
| 138 | AA308562 | *PLEK2* | pleckstrin 2 (mouse) homolog |
| 139 | U97519 | *PODXL* | podocalyxin-like |
| 140 | AI185998 | *PPIC* | peptidylprolyl isomerase C (cyclophilin C) |
| 141 | AA931981 | *PPM1B* | protein phosphatase 1B (formerly 2C), magnesium-dependent, beta isoform |
| 142 | L42373 | *PPP2R5A* | protein phosphatase 2, regulatory subunit B (B56), alpha isoform |
| 143 | AF044588 | *PRC1* | protein regulator of cytokinesis 1 |
| 144 | X74496 | *PREP* | prolyl endopeptidase |
| 145 | M65066 | *PRK4R1B* | protein kinase, cAMP-dependent, regulatory, type I, beta |
| 146 | AA972414 | *PRO2975* | hypothetical protein PRO2975 |
| 147 | D00860 | *PRPS1* | phosphoribosyl pyrophosphate synthetase 1 |
| 148 | D87258 | *PRSS11* | protease, serine, 11 (IGF binding) |
| 149 | AF043498 | *PSCA* | prostate stem cell antigen |
| 150 | D26598 | *PSMB3* | proteasome (prosome, macropain) subunit, beta type, 3 |
| 151 | X62006 | *PTB* | polypyrimidine tract binding protein (heterogeneous nuclear ribonucleoprotein I) |
| 152 | M77836 | *PYCR1* | pyrroline-5-carboxylate reductase 1 |
| 153 | X12953 | *RAB2* | RAB2, member RAS oncogene family |
| 154 | AA346311 | *RAI3* | retinoic acid induced 3 |
| 155 | X64652 | *RBMS1* | RNA binding motif, single stranded interacting protein 1 |
| 156 | S45545 | *RCV1* | recoverin |
| 157 | AA316525 | *REGIV* | Regenerating gene type IV |
| 158 | AB008109 | *RGS5* | regulator of G-protein signalling 5 |
| 159 | AA778308 | *RNASE1* | ribonuclease, RNase A family, 1 (pancreatic) |
| 160 | AA811043 | *RNASE6PL* | ribonuclease 6 precursor |
| 161 | L05096 | *RPL39* | Homo sapiens ribosomal protein L39 mRNA, complete cds |
| 162 | X76302 | *RY1* | putative nucleic acid binding protein RY |
| 163 | D38583 | *S100A11* | S100 calcium-binding protein A11 (calgizzarin) |
| 164 | AA308062 | *S100P* | S100 calcium-binding protein P |
| 165 | AA452018 | *SCD* | stearoyl-CoA desaturase (delta-9-desaturase) |
| 166 | D49737 | *SDHC* | succinate dehydrogenase complex, subunit C, integral membrane protein, 15kD |
| 167 | AA579861 | *SEC23A* | Sec23 (S. cerevisiae) homolog A |
| 168 | AA430643 | *SEPWI* | selenoprotein W, 1 |

(continued)

| PNC Assign ment | Accession No. | Symbol | Gene Name |
|---|---|---|---|
| 169 | AF029082 | *SFN* | stratifin solute carrier family 12 (sodium/potassium/ |
| 170 | AA639599 | *SLC12A2* | chloride transporters), member 2 |
| 171 | L20859 | *SLC20A1* | solute carrier family 20 (phosphate transporter), member 1 |
| 172 | L02785 | *SLC26A3* | solute carrier family 26, member 3 |
| 173 | K03195 | *SLC2A1* | solute carrier family 2 (facilitated glucose transporter), member 1 |
| 174 | U09873 | *SNL* | singed (Drosophila)-like (sea urchin fascin homolog like) |
| 175 | X13482 | *SNRPA1* | small nuclear ribonucleoprotein polypeptide A' |
| 176 | M37716 | *SNRPE* | small nuclear ribonucleoprotein polypeptide E |
| 177 | J03040 | *SPARC* | secreted protein, acidic, cysteine-rich (osteonectin) |
| 178 | M32313 | *SRD5A1* | steroid-5-alpha-reductase, alpha polypeptide 1 |
| 179 | M95787 | *TAGLN* | transgelin |
| 180 | M81601 | *TCEA1* | transcription elongation factor A (SII), 1 |
| 181 | A033095 | *TEGT* | testis enhanced gene transcript (BAX inhibitor 1) |
| 182 | L12350 | *THBS2* | thrombospondin 2 |
| 183 | M77142 | *TI41* | TIA1 cytotoxic granule-associated RNA-binding protein |
| 184 | K02581 | *TK1* | thymidine kinase 1, soluble |
| 185 | AA429631 | *TK2* | thymidine kinase 2, mitochondrial |
| 186 | U09087 | *TMPO* | thymopoietin |
| 187 | AF065388 | *TSPAN* | tetraspan 1 |
| 188 | U73379 | *UBCH10* | ubiquitin carrier protein E2-C |
| 189 | AA977545 | *UBE2D2* | ubiquitin-conjugating enzyme E2D 2 (homologous to yeast UBC4/5) |
| 190 | U45328 | *UBE2I* | ubiquitin-conjugating enzyme E2I (homologous to yeast UBC9) |
| 191 | M57899 | *UGTIA1* | UDP glycosyltransferase 1 family, polypeptide A1 |
| 192 | AA315189 | *UQCRB* | ubiquinol-cytoclrome c reductase binding protein |
| 193 | AB000450 | *VRK2* | vaccinia related kinase 2 |
| 194 | AA079060 | *WFDC2* | WAP four-disulfide core domain 2 |
| 195 | AA043277 | *WFS1* | Wolfram syndrome 1 (wolfram in) |
| 196 | AA581940 | *WHSC1* | Wolf-Hirschhorn syndrome candidate 1 |
| 197 | AI185056 | *ZNF134* | zinc finger protein 134 (clone pHZ5) |
| 198 | AA709155 | *FLJ10134* | hypothetical protein FLJ10134 |
| 199 | AA806630 | *FLJ10540* | hypothetical protein FLJ10540 |
| 200 | AA115015 | *FLJ10633* | hypothetical protein FLJ10633 |
| 201 | AA394229 | *FLJ0637* | hypothetical protein FLJ10637 |
| 202 | AA633302 | *FL120063* | hypothetical protein FLJ20063 |
| 203 | AA918811 | *FLJ20225* | hypothetical protein |
| 204 | R09189 | *FL120281* | hypothetical protein FLJ20281 |
| 205 | AA112198 | *FLJ20296* | hypothetical protein FLJ20296 |
| 206 | AI033837 | *FLJ20406* | hypothetical protein FLJ20406 |
| 207 | AA974462 | *FLJ23053* | hypothetical protein FLJ23053 |
| 208 | D14657 | *KIAAO1O1* | KIAA0101 gene product |
| 209 | D61862 | *KIAA0332* | KIAA0332 protein |
| 210 | AB014566 | *KIAA0666* | KIAA0666 protein |
| 211 | AB014570 | *KIAA0670* | KIAA0670 protein/acinus |
| 212 | AA665890 | *KIAA0729* | KIAA0729 protein |
| 213 | W80765 | *KIAA0731* | KIAA0731 protein |
| 214 | AF052170 | *KIAA0750* | KIAA0750 gene product |
| 215 | D20853 | *KIAA0776* | KIAA0776 protein |

(continued)

| PNC Assign ment | Accession No. | Symbol | Gene Name |
|---|---|---|---|
| 216 | AA031775 | *KIAA0990* | KIAA0990 protein |
| 217 | R39794 | *KIAA1624* | KIAA1624 protein |
| 218 | AA434045 | *KIAA1808* | ESTs |
| 219 | AI074410 | *KIAA1863* | Homo sapiens cDNA FLJ13996 fis, clone Y79AA1002211 |
| 220 | AF070638 | *CGI-57* | hypothetical protein |
| 221 | N38882 | | H.sapiens gene from PAC 106H8 |
| 222 | AI142828 | | Homo sapiens adlican mRNA, complete cds |
| 223 | AA028961 | | Homo sapiens cDNA FLJ12150 fis, clone MAMMA1000422 |
| 224 | AA933635 | | Homo sapiens cDNA FLJ13154 fis, clone NT2RP3003427 |
| 225 | AA523117 | *FLJ21504* | Homo sapiens cDNA: FLJ21504 fis, clone COL05662 |
| 226 | AA555187 | | Homo sapiens cDNA: FLJ22277 fis, clone HRC03740 |
| 227 | AF035315 | | Homo sapiens clone 23664 and 23905 mRNA sequence |
| 228 | AA968840 | | Homo sapiens HSPC285 mRNA, partial cds |
| 229 | R55322 | | Homo sapiens mRNA; cDNA DKFZp547K204 (from clone DKFZp547K204) |
| 230 | W55876 | | Homo sapiens mRNA; cDNA DKFZp586A0424 (from clone DKFZp586A0424) |
| 231 | AA789332 | *VANGL1* | ESTs, Moderately similar to KIAA1215 protein [H.sapiens] |
| 232 | AI310156 | | ESTs, Weakly similar to A4P_HUMAN INTESTINAL MEMBRANE A4 PROTEIN [H.sapiens] |
| 233 | C01335 | | ESTs, Weakly similar to FLDED [H.sapiens] |
| 234 | AI349804 | | ESTs, Weakly similar to IQGA_HUMAN RAS GTPASE-ACTIVATING-LIKE PROTEIN IQGAP 1 |
| 235 | AA683373 | | ESTs |
| 236 | H28960 | | ESTs |
| 237 | AA429665 | | ESTs |
| 238 | R17093 | | ESTs |
| 239 | AA806114 | | ESTs |
| 240 | AA707966 | | ESTs |
| 241 | D85376 | | ESTs |
| 242 | AA419568 | | ESTs |
| 243 | AA251355 | | ESTs |
| 244 | W63676 | | ESTs |
| 245 | AA570186 | | ESTs |
| 246 | AI239432 | | ESTs |
| 247 | AI264318 | | ESTs |
| 248 | AA553741 | | ESTs |
| 249 | N70804 | | ESTs |
| 250 | R61891 | | ESTs |
| 251 | W01507 | | ESTs |
| 252 | AA587884 | | ESTs |
| 253 | AA830326 | | ESTs |
| 254 | AI240520 | | ESTs |
| 255 | AA453716 | | ESTs |
| 256 | AI199761 | | ESTs |
| 257 | AI271678 | | ESTs |
| 258 | AA242941 | | ESTs |
| 259 | AI027791 | | ESTs |

Table4 A list of down-regulated genes

| PNC Assign ment | Accession No. | Symbol | Gene Name |
|---|---|---|---|
| 260 | D16294 | *ACAA2* | acetyl-Coenzyme A acyltransferase 2 (mitochondrial 3-oxoacyl-Coenzyme A thiolase) |
| 261 | M12963 | *ADH1* | alcohol dehydrogenase 1 (class I), alpha polypeptide |
| 262 | X04299 | *ADH3* | alcohol dehydrogenase 3 (class I), gamma polypeptide |
| 263 | L22214 | *ADORA1* | adenosine A 1 receptor |
| 264 | U04241 | *AES* | amino-terminal enhancer of split |
| 265 | AF044961 | *AKR1B11* | aldo-keto reductase family 1, member B11 |
| 266 | U05861 | *AKR1C1* | aldo-keto reductase family 1, member C1 |
| 267 | D26125 | *AKR1C4* | aldo-keto reductase family 1, member C4 |
| 268 | AI765873 | *ALDH10* | aldehyde dehydrogenase 10 (fatty aldehyde dehydrogenase) |
| 269 | X02747 | *ALDOB* | aldolase B, fructose-bisphosphate |
| 270 | M18786 | *AMY1A* | amylase, alpha 1A; salivary |
| 271 | M28443 | *AMY2A* | amylase, alpha 2A; pancreatic |
| 272 | M22324 | *ANPEP* | alanyl (membrane) aminopeptidase |
| 273 | Z11502 | *ANXA13* | annexin A13 |
| 274 | M82809 | *ANXA4* | annexin A4 |
| 275 | D00097 | *APCS* | amyloid P component, serum |
| 276 | M30704 | *AREG* | amphiregulin (schwannoma-derived growth factor) |
| 277 | AB007884 | *ARHGEF9* | Cdc42 guanine exchange factor (GEF) 9 |
| 278 | AI147612 | *ARL7* | ADP-ribosylation factor-like 7 |
| 279 | X83573 | *ARSE* | arylsulfatase E (chondrodysplasia punctata 1) |
| 280 | L19871 | *ATF3* | activating transcription factor 3 |
| 281 | Y15724 | *ATP2A3* | ATPase, Ca++ transporting, ubiquitous |
| 282 | AI091372 | *AXUD1* | AXIN1 up-regulated |
| 283 | X83107 | *BMX* | BMX non-receptor tyrosine kinase |
| 284 | AA468538 | *BRPF3* | bromodomain and PHD finger containing, 3 |
| 285 | U03274 | *BTD* | biotinidase |
| 286 | D31716 | *BTEBI* | basic transcription element binding protein 1 |
| 287 | W45244 | *C3* | complement component 3 |
| 288 | J03037 | *CA2* | carbonic anhydrase II |
| 289 | U36448 | *CADPS* | Ca2+-dependent activator protein for secretion |
| 290 | AI085802 | *CAV2* | Caveolin 2 |
| 291 | J02988 | *CD28* | CD28 antigen (Tp44) |
| 292 | M55509 | *CES1* | carboxylesterase 1 (monocyte/macrophage serine esterase 1) |
| 293 | U91543 | *CHD3* | chromodomain helicase DNA binding protein 3 |
| 294 | AA417345 | *CHPI* | chord domain-containing protein 1 |
| 295 | U62431 | *CHRNA2* | cholinergic receptor, nicotinic, alpha polypeptide 2 (neuronal) |
| 296 | U89916 | *CLDN10* | claudin 10 |
| 297 | AA885961 | *CLDN2* | Claudin 2 |
| 298 | J02883 | *CLPS* | colipase, pancreatic |
| 299 | M64722 | *CLU* | clusterin |
| 300 | X67318 | *CPA1* | carboxypeptidase A1 (pancreatic) |
| 301 | U19977 | *CPA2* | carboxypeptidase A2 (pancreatic) |
| 302 | AA780301 | *CTSF* | cathepsin F |
| 303 | T84490 | *CUGBP2* | CUG triplet repeat, RNA-binding protein 2 |
| 304 | M22865 | *CYB5* | cytochrome b-5 |

(continued)

| PNC Assign ment | Accession No. | Symbol | Gene Name |
|---|---|---|---|
| 305 | Y00498 | *CYP2C8* | cytochrome P450, subfamily IIC (mephenytoin 4-hydroxylase), polypeptide 8 |
| 306 | J04813 | *CYP3A5* | cytochrome P450, subfamily IIIA (niphedipine oxidase), polypeptide 5 |
| 307 | D00408 | *CYP3A7* | cytochrome P450, subfamily IIIA, polypeptide 7 |
| 308 | AA316159 | *DC11* | DC 11 protein |
| 309 | AA640753 | *DDAH1* | dimethylarginine dimethylaminohydrolase 1 |
| 310 | X96484 | *DGCR6* | DiGeorge syndrome critical region gene 6 |
| 311 | W76197 | *DLC1* | Deleted in liver cancer 1 |
| 312 | X68277 | *DUSP1* | dual specificity phosphatase 1 |
| 313 | M62829 | *EGRI* | early growth response 1 |
| 314 | M16652 | *ELA1* | elastase 1, pancreatic |
| 315 | AA845162 | *ELA3* | elastase 3, pancreatic (protease E) |
| 316 | M81635 | *EPB72* | erythrocyte membrane protein band 7.2 (stomatin) |
| 317 | M16967 | *F5* | coagulation factor V (proaccelerin, labile factor) |
| 318 | AA573905 | *FCGBP* | Fc fragment of IgG binding protein |
| 319 | AA033657 | *FGFR2* | fibroblast growth factor receptor 2 |
| 320 | U20391 | *FOLRI* | folate receptor I (adult) |
| 321 | U50743 | *FXYD2* | FXYD domain-containing ion transport regulator 2 |
| 322 | M11321 | *GC* | mRNA for group supecific component (GC) |
| 323 | Y15409 | *G6PT1* | glucose-6-phosphatase, transport (glucose-6-phosphate) protein 1 |
| 324 | A279817 | *GADD45B* | growth arrest and DNA-damage-inducible, beta |
| 325 | L13720 | *GAS6* | growth arrest-specific 6 |
| 326 | S68805 | *GATM* | glycine amidinotransferase (L-arginine:glycine amidinotransferase) |
| 327 | M24903 | *GGTI* | gamma-glutamyltransferase 1 |
| 328 | AW008481 | *GLUD1* | glutamate dehydrogenase 1 |
| 329 | T79836 | *GPS2* | G protein pathway suppressor 2 |
| 330 | D86962 | *GRB10* | growth factor receptor-bound protein 10 |
| 331 | L76687 | *GRB14* | growth factor receptor-bound protein 14 |
| 332 | D49742 | *HABP2* | hyaluronan-binding protein 2 |
| 333 | W37916 | *HCF-2* | host cell factor 2 |
| 334 | U63008 | *HGD* | homogentisate 1,2-dioxygenase (homogentisate oxidase) |
| 335 | W95267 | *HIBADH* | 3-hydroxyisobutyrate dehydrogenase |
| 336 | K01505 | *HLA-DQA1* | DC classII histocompatibility antigen alpha-chain |
| 337 | M81141 | *HLA-DQB1* | major histocompatibility complex, class II, DQ beta 1 |
| 338 | J03048 | *HPX* | hemopexin |
| 339 | T55714 | *HS3ST1* | heparan sulfate (glucosamine) 3-O-sulfotransferase 1 |
| 340 | AA206625 | *HS6ST* | heparan sulfate 6-O-sulfotransferase |
| 341 | U14631 | *HSD11B2* | hydroxysteroid (11-beta) dehydrogenase 2 |
| 342 | M11717 | *HSPA1A* | heat shock 70kD protein 1A |
| 343 | D49547 | *HSPF1* | heat shock 40kD protein 1 |
| 344 | AA885758 | *HTATIP* | HIV Tat interactive protein, 60 kDa |
| 345 | M27492 | *IL1R1* | interleukin 1 receptor, type I |
| 346 | AF014398 | *IMPA2* | inositol(myo)(or 4)-monophosphatase 2 |
| 347 | U84400 | *INPP5D* | inositol polyphosphate-5-phosphatase, 145kD |
| 348 | AA345854 | *ITGA3* | integrin, alpha 3 (antigen CD49C, alpha 3 subunit of VLA-3 receptor) |

(continued)

| PNC Assign ment | Accession No. | Symbol | Gene Name |
|---|---|---|---|
| 349 | AA845511 | *KCNJ16* | potassium inwardly-rectifying channel, subfamily J, member 16 |
| 350 | AI025297 | *KLF7* | Kruppel-like factor 7 (ubiquitous) |
| 351 | X79683 | *LAMB2* | laminin, beta 2 (laminin S) |
| 352 | X77196 | *LAMP2* | lysosomal-associated membrane protein 2 |
| 353 | M87842 | *LGALS2* | lectin, galactoside-binding, soluble, 2 (galectin 2) |
| 354 | AI160184 | *LOC51673* | brain specific protein |
| 355 | AI093595 | *LOC55895* | 22kDa peroxisomal membrane protein-like |
| 356 | AA347844 | *LOC56908* | Meis (mouse) homolog 2 |
| 357 | AK025620 | *LOC56990* | non-kinase Cdc42 effector protein SPEC2 |
| 358 | AA461526 | *LRRFIP2* | leucine rich repeat (in FLII) interacting protein 2 |
| 359 | H17536 | *LSM4* | U6 snRNA-associated Sm-like protein |
| 360 | AI092885 | *LSM6* | Sm protein F |
| 361 | AA157731 | *MAPIALC3* | Microtubule-associated proteins 1A and 1B, light chain 3 |
| 362 | X69078 | *MATIA* | methionine adenosyltransferase 1 alpha |
| 363 | X63380 | *MEF2B* | MADS box transcription enhancer factor 2, polypeptide B (myocyte enhancer factor 2B) |
| 364 | L08895 | *MEF2C* | MADS box transcription enhancer factor 2, polypeptide C (myocyte enhancer factor 2C) |
| 365 | X56741 | *MEL* | mel transforming oncogene (derived from cell line NK14)-RAB8 homolog |
| 366 | AI037890 | *MMP1* | matrix metalloproteinase 1 (interstitial collagenase) |
| 367 | R59292 | *MS4A8B* | Membrane-spanning 4-domains, subfamily A, member 8B |
| 368 | AL022315 | *MSE55* | serum constituent protein |
| 369 | D49441 | *MSLN* | mesothelin |
| 370 | M74178 | *MST1* | macrophage stimulating 1 |
| 371 | U35113 | *MTA1* | metastasis associated 1 |
| 372 | Y09788 | *MUC5B* | mucin 5, subtype B, tracheobronchial |
| 373 | AI745345 | *MVP* | major vault protein |
| 374 | X69090 | *MYOM1* | myomesin 1 (skelemin) (185kD) |
| 375 | AA497062 | *NFIC* | nuclear factor I/C (CCAAT-binding transcription factor) |
| 376 | AI309212 | *NLGN1* | neuroligin 1 |
| 377 | AJ005282 | *NPR2* | natriuretic peptide receptor B/guanylate cyclase B (atrionatriuretic peptide receptor B) |
| 378 | AA340728 | *NR2F2* | nuclear receptor subfamily 2, group F, member 2 |
| 379 | L13740 | *NR4A1* | nuclear receptor subfamily 4, group A, member 1 |
| 380 | X75918 | *NR4A2* | nuclear receptor subfamily 4, group A, member 2 |
| 381 | AB002341 | *NRCAM* | neuronal cell adhesion molecule |
| 382 | AA435678 | *P28* | dynein, axonemal, light intermediate polypeptide |
| 383 | AA576089 | *p53DINP1* | P53-inducible p53DINP1 |
| 384 | L15533 | *PAP* | pancreatitis-associated protein |
| 385 | T56982 | *PDE7A* | phosphodiesterase 7A |
| 386 | C05229 | *PDK4* | pyruvate dehydrogenase kinase, isoenzyme 4 |
| 387 | N47861 | *PDP* | pyruvate dehydrogenase phosphatase |
| 388 | AF012281 | *PDZK1* | PDZ domain containing 1 |
| 389 | AA220941 | *PHB* | prohibitin |
| 390 | D38616 | *PHKA2* | phosphorylase kinase, alpha 2 (liver) |
| 391 | L47738 | *PIR121* | p53 inducible protein |

(continued)

| PNC Assign ment | Accession No. | Symbol | Gene Name |
|---|---|---|---|
| 392 | X98654 | *PITPNM* | phosphatidylinositol transfer protein, membrane-associated |
| 393 | W19216 | *PKIG* | protein kinase (cAMP-dependent, catalytic) inhibitor gamma |
| 394 | AF064594 | *PLA2G6* | phospholipase A2, group VI (cytosolic, calcium-independent) |
| 395 | AF038440 | *PLD2* | phospholipase D2 |
| 396 | D87810 | *PMM1* | phosphomannomutase 1 |
| 397 | J05125 | *PNLIP* | pancreatic lipase |
| 398 | Z11898 | *POU5F1* | POU domain, class 5, transcription factor 1 |
| 399 | AI343963 | *PP2135* | PP2135 protein |
| 400 | U57961 | *13CDNA73* | putative gene product |
| 401 | AI094447 | *PP5395* | hypothetical protein PP5395 |
| 402 | S74349 | *PPARA* | peroxisome proliferative activated receptor, alpha |
| 403 | AB007851 | *PRPSAP2* | phosphoribosyl pyrophosphate synthetase-associated protein 2 |
| 404 | AA845165 | *PRSS1* | protease, serine, 1 (trypsin 1) |
| 405 | D88378 | *PSMF1* | proteasome (prosome, macropain) inhibitor subunit 1 (PI31) |
| 406 | U68142 | *RAB2L* | RAB2, member RAS oncogene family-like |
| 407 | AI277086 | *RAGB* | GTP-binding protein ragB |
| 408 | AA972852 | *RBF1* | retinol-binding protein 1, cellular |
| 409 | X00129 | *RBP4* | retinol-binding protein 4, interstitial |
| 410 | AA807607 | *RDGBB* | retinal degeneration B beta |
| 411 | AA428540 | *REC8* | Rec8p |
| 412 | M18963 | *REG1A* | regenerating islet-derived 1 alpha (pancreatic stone protein, pancreatic thread protein) |
| 413 | AC004003 | *RIPK2* | receptor-interacting serine-threonine kinase 2 |
| 414 | AI341482 | *RNB6* | RNB6 |
| 415 | AW510670 | *RNF3* | ring finger protein 3 |
| 416 | U38894 | *RORI* | receptor tyrosine kinase-like orphan receptor 1 |
| 417 | X65463 | *RXRB* | retinoid X receptor, beta |
| 418 | U72355 | *SAFB* | scaffold attachment factor B |
| 419 | AI338007 | *SCDGF-B* | Spinal cord-derived growth factor-B |
| 420 | AA911283 | *SCMH1* | sex comb on midleg homolog 1 |
| 421 | U84487 | *SCYD1* | small inducible cytokine subfamily D (Cys-X3-Cys), member 1 (fractalkine, neurotactin) |
| 422 | W73992 | *SDCCAG43* | serologically defined colon cancer antigen 43 |
| 423 | U28369 | *SEMA3B* | sema domain, immunoglobulin domain (Ig), short basic domain, secreted, (semaphorin) 3B |
| 424 | U38276 | *SEMA3F* | sema domain, immunoglobulin domain (Ig), short basic domain, secreted, (semaphorin) 3F |
| 425 | AI026695 | *SENP1* | Sentrin/SUMO-specific protease |
| 426 | Z11793 | *SEPP1* | selenoprotein P, plasma, 1 |
| 427 | H89783 | *SERPINA4* | serine (or cysteine) proteinase inhibitor, clade A (alpha antiproteinase, antitrypsin), member 4 |
| 428 | J02943 | *SERPINA6* | serine (or cysteine) proteinase inhibitor, clade A (alpha antiproteinase, antitrypsin), member 6 |
| 429 | M13690 | *SERPING1* | serine (or cysteine) proteinase inhibitor, clade G (C1 inhibitor), member 1 |
| 430 | AF017988 | *SFRP5* | secreted frizzled-related protein 5 |

(continued)

| PNC Assign ment | Accession No. | Symbol | Gene Name |
|---|---|---|---|
| 431 | N56912 | *SFTPC* | surfactant, pulmonary-associated protein C |
| 432 | Y10032 | *SGK* | serum/glucocorticoid regulated kinase |
| 433 | AI198522 | *SLC11A3* | solute carrier family 11, member 3 |
| 434 | U59299 | *SLC16A5* | solute carrier family 16, member 5 |
| 435 | AA243675 | *SLC1A1* | solute carrier family 1, member 1 |
| 436 | AA435777 | *SLC25A1* | Solute carrier family 25 (mitochondrial carrier; citrate transporter), member 1 |
| 437 | NM_000340 | *SLC2A2* | solute carrier family 2 (facilitated glucose transporter), member 2 |
| 438 | M95548 | *SLC3A1* | solute carrier family 3 (cystine, dibasic and neutral amino acid transporters, activator of cystine, dibasic and neutral amino acid transport), member 1 |
| 439 | AF007216 | *SLC4A4* | solute carrier family 4, sodium bicarbonate cotransporter, member 4 |
| 440 | M24847 | *SLC5A1* | solute carrier family 5 (sodium/glucose cotransporter), member 1 |
| 441 | AA902273 | *SMARCD3* | SWI/SNF related, matrix associated, actin dependent regulator of chromatin, member 3 |
| 442 | U41303 | *SNRPN* | small nuclear ribonucleoprotein polypeptide N |
| 443 | AA604446 | *SPINK5* | serine protease inhibitor, Kazal type, 5 |
| 444 | J04765 | *SPPI* | secreted phosphoprotein 1 (osteopontin, bone sialoprotein I, early T-lymphocyte activation 1) |
| 445 | L14865 | *SSTR5* | somatostatin receptor 5 |
| 446 | R60028 | *TAB1* | transforming growth factor beta-activated kinase-binding protein 1 |
| 447 | X58840 | *TCF2* | transcription factor 2, hepatic; LF-B3; variant hepatic nuclear factor |
| 448 | J05068 | *TCN1* | transcobalamin I (vitamin B12 binding protein, R binder family) |
| 449 | L15203 | *TFF3* | trefoil factor 3 (intestinal) |
| 450 | D29992 | *TFPI2* | tissue factor pathway inhibitor 2 |
| 451 | AA403273 | *TLE1* | transducin-like enhancer of split 1, homolog of Drosophila E(spl) |
| 452 | U31449 | *TM4SF4* | transmembrane 4 superfamily member 4 |
| 453 | AA131918 | *TMEM3* | transmembrane protein 3 |
| 454 | U70321 | *TNFRSF14* | tumor necrosis factor receptor superfamily, member 14 (herpesvirus entry mediator) |
| 455 | L21715 | *TNNI2* | troponin I, skeletal, fast |
| 456 | AI091425 | *TONDU* | TONDU |
| 457 | U54831 | *TOP2B* | topoisomerase (DNA) II beta (180kD) |
| 458 | U44427 | *TPD52L1* | tumor protein D52-like 1 |
| 459 | M10605 | *TTR* | transthyretin (prealbumin, amyloidosis type I) |
| 460 | AI090567 | *TUBB2* | tubulin, beta, 2 |
| 461 | L13852 | *UBE1L* | ubiquitin-activating enzyme E1-like |
| 462 | X63359 | *UGT2B10* | UDP glycosyltransferase 2 family, polypeptide B10 |
| 463 | J05428 | *UGT2B7* | UDP glycosyltransferase 2 family, polypeptide B7 |
| 464 | AA446913 | *USP11* | ubiquitin specific protease 11 |
| 465 | L13288 | *VIPR1* | vasoactive intestinal peptide receptor 1 |
| 466 | D78298 | *VLCAD* | very-long-chain acyl-CoA dehydrogenase |
| 467 | AA769424 | *VNN2* | vanin 2 |
| 468 | AF039022 | *XPOT* | exportin, tRNA (nuclear export receptor for tRNAs) |

(continued)

| PNC Assign ment | Accession No. | Symbol | Gene Name |
|---|---|---|---|
| 469 | D83407 | *ZAKI4* | Down syndrome critical region gene 1-like 1. |
| 470 | Z19002 | *ZNF145* | zinc finger protein 145 (Kruppel-like, expressed in promyelocytic leukemia) |
| 471 | M58297 | *ZNF42* | zinc finger protein 42 (myeloid-specific retinoic acid-responsive) |
| 472 | N24911 | *C11ORF2* | chromosome 11 open reading frame2 |
| 473 | AI186263 | *C21ORF11* | chromosome 21 open reading frame 11 |
| 474 | Y11392 | *C21ORF2* | chromosome 21 open reading frame 2 |
| 475 | H16793 | *C80RF4* | chromosome 8 open reading frame 4 |
| 476 | AI160590 | *DKFZp434G 0522* | hypothetical protein DKFZp434G0522 |
| 477 | T65389 | *DKFZP434J 214* | DKFZP434J214 protein |
| 478 | H61870 | *DKFZP564F 1123* | DKFZP564F1123 protein |
| 479 | AI218000 | *DKFZP564K 1964* | DKFZP564K1964 protein |
| 480 | AI306435 | *DKFZP586A 0522* | DKFZP586A0522 protein |
| 481 | W05570 | *DKFZP586B 0621* | DKFZP586B0621 protein |
| 482 | N92489 | *FLJ10103* | hypothetical protein FLJ10103 |
| 483 | AA933772 | *FLJ10252* | hypothetical protein FLJ10252 |
| 484 | AA452368 | *FIJ10582* | hypothetical protein FLJ10582 |
| 485 | AA481246 | *FLJ12287* | hypothetical protein FLJ12287 similar to semaphorins |
| 486 | AI042204 | *FLJ12895* | hypothetical protein FLJ12895 |
| 487 | AI342612 | *FLJ20011* | hypothetical protein FLJ20011 |
| 488 | AA708532 | *FLJ20041* | hypothetical protein FLJ20041 |
| 489 | AI016890 | *FLJ20542* | hypothetical protein FLJ20542 |
| 490 | AA593701 | *FLJ21817* | hypothetical protein FLJ21817 similar to Rhoip2 |
| 491 | NM_022493 | *FL121988* | hypothetical protein FLJ21988 |
| 492 | N30915 | *FLJ22649* | hypothetical protein FLJ22649 similar to signal peptidase SPC22/23 |
| 493 | AA650281 | *FLJ23153* | Likely ortholog of mouse tumor necrosis-alpha-induced adipose-related protein |
| 494 | AA522448 | *FLJ23239* | hypothetical protein FLJ23239 |
| 495 | AA403120 | *HT014* | HT014 |
| 496 | D31884 | *KIAA0063* | KIAA0063 gene product |
| 497 | D87465 | *KIAA0275* | KIAA0275 gene product |
| 498 | AI190847 | *KIAA0397* | KIAA0397 gene product |
| 499 | AB011115 | *KIAA0543* | KIAA0543 protein |
| 500 | AA910738 | *KIAA0579* | KIAA0579 protein |
| 501 | AA156717 | *KIAA0668* | KIAA0668 protein |
| 502 | W56303 | *KIAA0802* | KIAA0802 protein |
| 503 | AA127777 | *KIAA1071* | KIAA 1071 protein |
| 504 | AI148832 | *KIAA1209* | KIAA1209 protein |
| 505 | AA573892 | *KIAA1359* | KIAA1359 protein |
| 506 | N54300 | *KIAA1500* | KIAA1500 protein |
| 507 | N36929 | *KIAA1954* | KIAA 1954 protein |
| 508 | AA477232 | *LOC56997* | hypothetical protein, clone Telethon(Italy_B41)_ |
| 509 | AF001550 | *LOC57146* | Strait02270_FL142 hypothetical protein from clone 24796 |
| 510 | AA303231 | *LOC64744* | hypothetical protein AL133206 |
| 511 | AA044186 | | Homo sapiens cDNA FLJ11410 fis, clone HEMBA1000852 |
| 512 | D62873 | | Homo sapiens cDNA FLJ12900 fis, clone NT2RP2004321 |

(continued)

| PNC Assign ment | Accession No. | Symbol | Gene Name |
|---|---|---|---|
| 513 | AA858162 | | Homo sapiens cDNA FLJ13005 fis, clone NT2RP3000441 |
| 514 | AA327291 | | Homo sapiens cDNA FLJ13322 fis, clone OVARC1001713 |
| 515 | A1096874 | | Homo sapiens cDNA FLJ14115 fis, clone MAMMA1001760 |
| 516 | H28758 | | Homo sapiens cDNA: FLJ20925 fis, clone ADSE00963 |
| 517 | T04932 | | Homo sapiens cDNA: FLJ21545 fis, clone COL06195 |
| 518 | AK025906 | | Homo sapiens cDNA: FLJ22253 fis, clone HRC02763 |
| 519 | AI344138 | | Homo sapiens cDNA: FLJ22288 fis, clone HRC04157 |
| 520 | AA206578 | | Homo sapiens cDNA: FLJ22316 fis, clone HRC05262. |
| 521 | R89624 | | Homo sapiens cDNA: FLJ22386 fis, clone HRC07619 |
| 522 | AA404225 | | Homo sapiens cDNA: FLJ22418 fis, clone HRC08590 |
| 523 | AI089485 | | Homo sapiens cDNA: FLJ22479 fis, clone HRC10831 |
| 524 | AA505312 | | Homo sapiens cDNA: FLJ22648 fis, clone HS107329 |
| 525 | R72460 | | Homo sapiens cDNA: FLJ22807 fis, clone KAIA2887 |
| 526 | AA019961 | | Homo sapiens cDNA: FLJ22811 fis, clone KAIA2944 |
| 527 | N46856 | | Homo sapiens cDNA: FLJ23091 fis, clone LNG07220 |
| 528 | AA321321 | | Homo sapiens cDNA: FLJ23091 fis, clone LNG07220 |
| 529 | AI084531 | | Homo sapiens cDNA: FLJ23093 fis, clone LNG07264 |
| 530 | AA543086 | | Homo sapiens cDNA: FLJ23270 fis, clone COL10309 |
| 531 | AA741042 | | Homo sapiens cDNA: FLJ23527 fis, clone LNG05966 |
| 532 | AF009314 | | Homo sapiens clone TUA8 Cri-du-chat region mRNA |
| 533 | AA293837 | | Homo sapiens GKAP42 (FKSG21) mRNA, complete cds |
| 534 | AA195740 | | Homo sapiens mRNA full length insert cDNA clone EUROIMAGE 41832 |
| 535 | AA829835 | | Homo sapiens mRNA; cDNA DKFZp434M229 (from clone DKFZp434M229) |
| 536 | AA985007 | | Homo sapiens mRNA; cDNA DKFZp564A026 (from clone DKFZp564A026) |
| 537 | AA938345 | | Homo sapiens mRNA; cDNA DKFZp564N1116 (from clone DKFZp564N1116) |
| 538 | AA129758 | | Homo sapiens mRNA; cDNA DKFZp761K2024 (from clone DKFZp761K2024) |
| 539 | AI276126 | | Human DNA sequence from clone RP4-756G23 on chromosome 22q13.313.33 |
| 540 | AI301241 | | ESTs, Highly similar to AF 172268 1 Traf2 and NCK interacting kinase, splice variant 5 |
| 541 | AI291118 | | ESTs, Highly similar to AF2191401 1 gastric cancer-related protein GCYS-20 [H.sapiens] |
| 542 | AA143060 | | ESTs, Highly similar to 13 8945 melanoma ubiquitous mutated protein [H.sapiens] |
| 543 | AI304351 | | ESTs, Moderately similar to NFY-C [H.sapiens] |
| 544 | AA923049 | | ESTs, Weakly similar to cytokine receptor-like factor 2; cytokine receptor CRL2 precusor |
| 545 | AA604003 | | ESTs, Weakly similar to CTL1 protein [H.sapiens] |
| 546 | AA847242 | | ESTs, Weakly similar to G786_HUMAN PROTEIN GS3786 [H.sapiens] |
| 547 | AI274179. | | ESTs, Weakly similar to LIV protein [H.sapiens] |
| 548 | R87741 | | ESTs, Weakly similar to RAB8_HUMAN RAS-RELATED PROTEIN RAB-8 [H.sapiens] |

(continued)

| PNC Assign ment | Accession No. | Symbol | Gene Name |
|---|---|---|---|
| 549 | AA465193 | | ESTs, Weakly similar to unnamed protein product [H.sapiens] |
| 550 | AI266124 | | ESTs, Weakly similar to unnamed protein product [H.sapiens] |
| 551 | AA777360 | *KIAA1002* | ESTs |
| 552 | AA358397 | | ESTs |
| 553 | AA129817 | | ESTs |
| 554 | F06091 | | ESTs |
| 555 | H42099 | | ESTs |
| 556 | AI090386 | | ESTs |
| 557 | AA449335 | | ESTs |
| 558 | AI243456 | | ESTs |
| 559 | AI355928 | | ESTs |
| 560 | R45502 | | ESTs |
| 561 | AA630642 | | ESTs |
| 562 | AA781393 | | ESTs |
| 563 | AA528243 | | ESTs |
| 564 | AA430699 | | ESTs |
| 565 | AA528190 | | ESTs |
| 566 | AA369905 | | ESTs |
| 567 | AI201894 | | ESTs |
| 568 | AI342469 | | ESTs |
| 569 | AA313152 | | ESTs |
| 570 | AI299327 | | ESTs |
| 571 | AI341332 | | ESTs |
| 572 | N33189 | | ESTs. |
| 573 | W37776 | | ESTs |
| 574 | AI023557 | | ESTs |
| 575 | AA418448 | | ESTs |
| 576 | AA458558 | | ESTs |
| 577 | H52704 | | ESTs |
| 578 | AA142875 | | ESTs |
| 579 | AI366443 | | ESTs |
| 580 | H96559 | | ESTs |
| 581 | H98777 | | ESTs |
| 582 | AA989233 | | ESTs |
| 583 | AI032354 | | ESTs |
| 584 | W93000 | | ESTs |
| 585 | AA446184 | | ESTs |
| 586 | AI291207 | | ESTs |
| 587 | AA699359 | | ESTs |
| 588 | AA447217 | | ESTs |
| 589 | AA769604 | | ESTs |
| 590 | AI208970 | | ESTs |
| 591 | N93057 | | ESTs |
| 592 | AI225224 | | ESTs |
| 593 | W67193 | | ESTs |
| 594 | AI022649 | | ESTs |
| 595 | AA625553 | | ESTs |
| 596 | AA446064 | | ESTs |

(continued)

| PNC Assign ment | Accession No. | Symbol | Gene Name |
|---|---|---|---|
| 597 | D61466 | | ESTs |
| 598 | H05777 | | ESTs |
| 599 | N30923 | | ESTs |
| 600 | AA135406 | | ESTs |
| 601 | AA661636 | | ESTs |
| 602 | H98796 | | ESTs |
| 603 | AI927063 | | ESTs |
| 604 | AA687594 | | ESTs |
| 605 | AA879280 | | ESTs |

**Table 5** Representative up-regulated genes with known function in pancreatic cancers

| PNC Assignment | Accession No. | Symbol | Gene Name |
|---|---|---|---|
| genes involved in signal transduction pathway | | | |
| 12 | AA916826 | *APP* | amyloid beta (A4) precursor protein (protease nexin-II, |
| 13 | L20688 | *ARHGDIB* | Alzheimer disease) Rho GDP dissociation inhibitor (GDI) beta |
| 59 | L36645 | *EPHA4* | EphA4 |
| 69 | J03260 | *GNAZ* | guanine nucleotide binding protein (G protein), alpha z |
| 100 | AA574178 | *KAI1* | polypeptide Kangai 1 |
| 119 | AA458825 | *MTIF2* | mitochondrial translational initiation factor 2 |
| 130 | M80482 | *PACE4* | paired basic amino acid cleaving system 4 |
| 135 | M16750 | *PIMI* | pim oncogene |
| 151 | X62006 | *PTB* | polypyrimidine tract binding protein (heterogeneous |
| 154 | AA346311 | *RAI3* | nuclear ribonucleoprotein I) retinoic acid induced 3 |
| 156 | S45545 | *RCV1* | recoverin |
| 163 | D38583 | *S100A11* | S100 calcium-binding protein A11 (calgizzarin) |
| 164 | AA308062 | *S100P* | S100 calcium-binding protein P |
| 169 | AF029082 | *SFN* | stratifin |
| 177 | J03040 | *SPARC* | secreted protein, acidic, cysteine-rich (osteonectin) |
| transcriptional factors | | | |
| 8 | AF047002 | *ALY* | transcriptional coactivator |
| 44 | AA905901 | *CRSP3* | cofactor required for Sp1 transcriptional activation, |
| 63 | L16783 | *FOXM1* | subunit 3 (130kD) forkhead box M1 |
| 66 | AA418167 | *GATA3* | GATA-binding protein 3 |
| 80 | X92518 | *HMGIC* | high-mobility group (nonhistone chromosomal) protein |
| 83 | M16937 | *HOXB7* | isoform I-C homeo box B7 |
| 108 | U24576 | *LMO4* | LIM domain only 4 |
| 120 | X13293 | *MYBL2* | v-myb avian myeloblastosis viral oncogene homolog-like 2 |
| 155 | X64652 | *RBMS1* | RNA binding motif, single stranded interacting protein 1 |
| 180 | M81601 | *TCEA1* | transcription elongation factor A (SII), 1 |
| cell adhesion and cytoskeleton | | | |
| 14 | AF006086 | *ARPC3* | actin related protein 2/3 complex, subunit 3 (21 kD) |
| 28 | AA557142. | *CD2AP* | CD2-associated protein |
| 32 | X63629 | *CDH3* | cadherin 3, type 1, P-cadherin (placental) |
| 38 | AA977821 | *COL1A1* | collagen, type I, alpha 1 |

(continued)

| | | | |
|---|---|---|---|
| cell adhesion and cytoskeleton | | | |
| 39 | J03464 | *COL1A2* | collagen, type I, alpha 2 |
| 40 | X14420 | *COL3A1* | collagen, type III, alpha 1 (Ehlers-Danlos syndrome type |
| 41 | AI140851 | *COL6A1* | IV, autosomal dominant) collagen, type VI, alpha 1 |
| 46 | U16306 | *CSPG2* | chondroitin sulfate proteoglycan 2 (versican) |
| 62 | X02761 | *FN1* | fibronectin 1 |
| 98 | M15395 | *ITGB2* | integrin, beta 2 (antigen CD18 (p95), lymphocyte function-associated antigen 1; macrophage antigen 1 (mac) beta |
| 104 | J00269 | *KRT6A* | subunit) keratin 6A |
| 131 | L11370 | *PCDHI* | protocadherin 1 (cadherin-like 1) |
| 136 | AA234962 | *PKP3* | plakophilin 3 |
| cell cycle | | | |
| 35 | X54941 | *CKS1* | CDC28 protein kinase 1 |
| 63 | L16783 | *FOXM1* | forkhead box M1 |
| 102 | U63743 | *KNSL6* | kinesin-like 6 (mitotic centromere-associated kinesin) |
| 143 | AF044588 | *PRC1* | protein regulator of cytokinesis 1 |
| 184 | K02581 | *TK1* | thymidine kinase 1, soluble |

Comparison of clinicopathological parameters with the expression profiles indicated that altered expression of 76 genes was associated with lymph-node metastasis and that of 168 genes with liver metastasis. In addition, expression levels of 84 genes were related to the recurrence of disease. These genome-wide expression profiles should provide useful information for finding candidate genes whose products might serve as specific tumor markers and/or as molecular targets for treatment of patients with pancreatic cancer.

Materials and Methods

*Identification of genes responsible for clinicopathological data*

Genes associated with clinicopathological features, such as lymph-node-positive (r) and -negative (n), liver metastasis-positive (r) and -negative (n), and early-recurrence (r) and late-recurrence (n), were chosen according to the these two criteria; (i) signal intensities are higher than the cut-off value in at least 80% of the cases; (ii)$|Med_r - Med_n|$>=0.5, where Med indicates the median derived from log-transformed relative expression ratios in two groups. Genes were selected as candidates when they met the criteria with a permutation p-value of smaller than 0.05 in each clinicopathological status.

First, we applied a random permutation test to identify genes that were expressed differently in following two groups. The mean ($\mu$) and standard deviation ($\sigma$) were calculated from the log-transformed relative expression ratios of each gene in node-positive (r) and node-negative (n) cases, liver-metastasis-positive (r) and -negative (n), and early-recurrence (r) and late-recurrence (n), respectively. A discrimination score (DS) for each gene was defined as follows:

$$DS = (\mu_r - \mu_n) / (\sigma_r + \sigma_n)$$

We carried out permutation tests to estimate the ability of individual genes to distinguish with two groups; samples were randomly permutated between the two classes 10,000 times. Since the DS dataset of each gene showed a normal distribution, we calculated a P value for the user-defined grouping (Golub et al., 1999). For this analysis, we applied the expression data of 13 cases consisting of 4 lymph-node-positive and 9 negative cases, those of 11 cases consisting of 5 liver metastasis-positive and 6 negative cases, and those of 13 cases consisting of 7 early-recurrent cases and 6 late-recurrent cases. For these analyses were performed by using only StageIV cases according to UICC TNM classification.

*Calculation of prediction score*

We further calculated the prediction score of recurrence according to procedures described previously (Golub et al., 1999). Each gene ($g_i$) votes for either early-recurrent cases or late-recurrent cases depending on whether the

expression level ($x_i$) in the sample is closer to the mean expression level of early-recurrent cases or late-recurrent cases in reference samples. The magnitude of the vote ($v_i$) reflects the deviation of the expression level in the sample from the average of the two classes:

$$V_i = | x_i - (\mu_r + \mu_n) /2 |$$

We summed the votes to obtain total votes for the early-recurrent cases ($V_r$) and late-recurrent cases ($V_n$), and calculated PS values as follows:

$$PS = ((V_r - V_n) / (V_r + V_n)) x100$$

reflecting the margin of victory in the direction of either early-recurrent cases or late-recurrent cases. PS values range from -100 to 100; a higher absolute value of PS reflects a stronger prediction.

*Evaluation of classification and leave-one-out test*

We calculated the classification score (CS) by using the prediction score of early-recurrent ($PS_r$) and late-recurrent cases ($PS_n$) in each gene set, as follows:

$$CS = (\mu_{PSr} - \mu_{PSn}) / (\sigma_{PSr} + \sigma_{PSn})$$

A larger value of CS indicates better separation of the two groups by the predictive-scoring system. For the leave-one-out test, one sample is withheld, the permutation p-value and mean expression levels are calculated using remaining samples, and the class of the withheld sample is subsequently evaluated by calculating its prediction score. We repeated this procedure for each of the 13 samples.

Results

*Identifcation of genes correlated with clinicopathological features*

*Lymph-node metastasis and liver metastasis*

In order to investigate relations between gene expression profiles and clinicopathological parameters, we searched genes that were possibly associated with lymph-node metastasis and liver metastasis that are important determining factors of patients' prognosis. We first examined the expression profiles and the status of lymph-node metastasis using nine lymph-node-positive and four node-negative cases, and identified 76 genes that were associated with lymph node status by a random permutation (p-value <0.05) (Table 6). Of those, 35 genes were relatively up-regulated, and 41 genes were down-regulated in node-positive tumors (Figure 3) comparing with node-negative tumors as control. In addition, we compared expression profiles of 5 cases with predominant recurrence in liver with those of 6 cases with metastasis to other sites (local, peritoneal and chest). We identified 168 genes that showed altered expression patterns uniquely in cases that had liver metastasis (Table 7), and 60 of them were relatively up-regulated in tumors (Figure 4). These genes included some key factors which had been proposed to play crucial roles in tumor cell proliferation, invasion and metastasis: integrin, beta 4 (*ITGB4*) (Shaw et al., 1997), colony stimulating factor 1 (*CSF1*) (Chambers et al., 1997), basigin (*BSG*) (Guo et al., 2000), and kinesin-like 6 (*KNSL6*) (Scanlan MJ et al., 2002). Hierarchical clustering analysis using these identified gene sets was also able to clearly classify the groups with regard to lymph node status or those with liver metastasis, respectively (Figure 3, 4).

*Prognosis*

To further investigate genes that might be associated with prognosis, we compared expression profiles of 7 cases who had recurrence within 12 months after surgery (disease free interval <12 months; median 6.4 months) with those of 6 cases who had >12 months of disease free interval (median 17.0 months). As shown in Figure 5A, we identified 84 genes that were expressed differently between these two groups using a random permutation method ($p < 0.05$).

In attempt on establishment of a predictive scoring system using gene expression pattern for recurrence after surgery, we rank-ordered above prognostic 84 candidate genes on the basis of the magnitude of their permutation p-values (Table 8) and calculated the prediction score by the leave-one-out test for cross-validation using top 5, 10, 15, 20, 25, 30, 35, 40, 45, 50, 55, 60, 65, 70, 75, 80 and 84 genes on the rank-ordered list. To determine the number of discriminating genes giving the best separation of the two groups, we calculated a classification score (CS) for each gene set (Figure 5B). As show in Figure 5C, the best separation was obtained when we used 30 genes consisting of top 17 genes of up-regulated in late recurrence cases genes and top 13 genes of up-regulated in early recurrence cases genes in our candidate list for scores calculation.

Discussion

Pancreatic cancer is characterized by very aggressive progression and rapid recurrence after surgical treatment. It has been reported that the cumulative 1-, 3-, and 5-year disease free survival rate were 66%, 7%, and 3% respectively, and median disease-free survival time was the only 8 months (Sperti et al., 1997). Most common recurrent sites are the local region and the liver, and distant metastases appear in the peritoneal cavity. However, since the relationships between tumor characteristics and the recurrence patterns are still little understood, we compared the expression profiles to lymph-node status or liver metastasis. We identified 76 genes that might be associated with lymph-node status, and 168 genes with liver metastasis. These genes included some key molecules whose possible roles in tumor progression had been reported previously; *ITGB4* and *BSG* were up-regulated in lymph-node positive cases, and *KNSL6* and *KRT8* were relatively up-regulated in liver metastasis cases. *ITGB4* was reported to promote carcinoma invasion through a preferential and localized targeting of phosphoinositide-3 OH kinase activity (Shaw et al., 1997), supporting the possible involvement of *ITGB4* in lymph-node metastasis. *KNSL6,* a member of the kinesin family of motor proteins, is known to be involved in chromosome segregation during mitosis (Maney T et al., 1998). The transcript of *KNSL6* was highly expressed in colon cancer, and was identified as cancer antigens associated with a cancer-related serum IgG response (Scanlan MJ et al., 2002). Thus, this antigen could be a biological marker for diagnosis and for monitoring of recurrence site.

In addition, we identified 84 genes possibly associated with tumor recurrence of pancreatic cancers. Expression levels of a subset of 30 genes selected from these 84 genes would be useful for predicting the disease free interval after surgical operation (Figure 5). These results might be useful for selection of patients for active adjuvant therapy although larger-scale study will be required to further evaluate our prediction system.

**Table6 A** list of 76 Candidate Genes for lymph-node metastasis

| PNC Assign ment | GenBank ID | Symbol | Gene Name |
|---|---|---|---|
| **UP-REGULATED GENES** | | | |
| 606 | D16480 | *HADHA* | hydroxyacy dehydrogenase, subunitA |
| 607 | AF015767 | *BRE* | brain and reproductive organ-expressed (TNFRSF1A modulator) |
| 608 | D49742 | *HABP2* | hyaluronan-binding protein 2 |
| 609 | M37400 | *GOT1* | glutamic-oxaloacetic transaminase 1 |
| 610 | Z11502 | *ANXA13* | annexin A13 |
| 611 | D32050 | *AARS* | alanyl-tRNA synthetase |
| 612 | U42376 | *LY6E* | lymphocyte antigen 6 complex, locus E |
| 613 | U68019 | *MADH3* | MAD (mothers against decapentaplegic, Drosophila) homolog 3 |
| 614 | AI248620 | *AP3D1* | adaptor-related protein complex 3, delta 1 subunit |
| 615 | U24183 | *PFKM* | phosphofructokinase, muscle |
| 616 | AA193416 | | ESTs |
| 617 | AA911109 | *FLJ20254* | hypothetical protein FLJ20254 |
| 618 | AF070616 | *HPCAL1* | hippocalcin-like 1 |
| 619 | AI143127 | | Dynactin 4 |
| 620 | AA412250 | *PYGB* | phosphorylase, glycogen; brain |
| 621 | D45131 | *BSG* | basigin |
| 622 | AB010427 | *WDRI* | WD repeat domain 1 |
| 623 | H20386 | *MYG1* | MYG1 protein |

(continued)

| PNC Assign ment | GenBank ID | Symbol | Gene Name |
|---|---|---|---|
| **UP-REGULATED GENES** | | | |
| 624 | AA371593 | *GCNILI* | GCN1 (general control of amino-acid synthesis 1, yeast)-like 1 |
| 625 | L31581 | *CCR7* | chemokine (C-C motif) receptor 7 |
| 626 | AA922357 | | DKFZp586A0618 |
| 627 | U07424 | *FARSL* | phenylalanine-tRNA synthetase-like |
| 628 | AI248327 | | FLJ22233 |
| 629 | AF055022 | *DKFZP727 M231* | DKFZP727M231 protein |
| 630 | M37435 | *CSF1* | colony stimulating factor 1 (macrophage) |
| 631 | U34683 | *GSS* | glutathione synthetase |
| 632 | L41351 | *PRSS8* | protease, serine, 8 (prostasin) |
| 633 | X52186 | *ITGB4* | integrin, beta 4 |
| 634 | R52161 | | DKFZp434A2410 |
| 635 | U23028 | *EIF2B5* | eukaryotic translation initiation factor 2B, subunit 5 (epsilon, 82kD) |
| 636 | AI336230 | *RPS8* | ribosomal protein S8 |
| 637 | AI268861 | | EST |
| 638 | U73036 | *IRF7* | interferon regulatory factor 7 |
| 639 | AI097058 | | FLJ23538 |
| 640 | L36151 | *PIK4CA* | phosphatidylinositol 4-kinase, catalytic, alpha polypeptide |
| **DOWN-REGULATED GENES** | | | |
| 641 | AA747290 | *RPS15A* | ribosomal protein S15a |
| 642 | AA641744 | *RPA2* | replication protein A2 (32kD) |
| 643 | AI188196 | *USP22* | ubiquitin specific protease 22 |
| 644 | AI222007 | | ESTs |
| 645 | AA192445 | *TMEPAI* | transmembrane, prostate androgen induced RNA |
| 646 | AW069055 | *FLJ10773* | Likely ortholog of mouse NPC derived proline rich protein 1 |
| 647 | AI365733 | | ESTs |
| 648 | AF017418 | *MEIS2* | Meis (mouse) homolog 2 |
| 649 | AF024714 | *AIM2* | absent in melanoma 2 |
| 650 | AU155489 | *MMP7* | matrix metalloproteinase 7 (matrilysin, uterine) |
| 651 | AW779142 | *HUMAGCG B* | chromosome 3p21.1 gene sequence |
| 652 | AA487669 | *GSTM1* | glutathione-S-transferase M1 |
| 653 | AA601564 | *DLG5* | discs, large (Drosophila) homolog 5 |
| 654 | AI042204 | *FLJ12895* | hypothetical protein FLJ12895 |
| 655 | D14662 | *KIAA0106* | anti-oxidant protein 2 |
| 656 | BF059178 | *NONO* | non-POU-domain-containing, octamer-binding |
| 657 | U70063 | *ASAH* | N-acylsphingosine amidohydrolase (acid ceramidase) |
| 658 | AA091553 | *UBE2H* | ubiquitin-conjugating enzyme E2H (homologous to yeast UBC8) |
| 659 | L12350 | *THBS2* | thrombospondin 2 |
| 660 | AA324335 | *ERF.* | Ets2 repressor factor |
| 661 | AI626007 | *NTRK1* | neurotrophic tyrosine kinase, receptor, type 1 |
| 662 | AI261382 | *SH120* | putative G-protein coupled receptor |
| 663 | AF046024 | *UBE1C* | ubiquitin-activating enzyme E1C (homologous to yeast UBA3) |
| 664 | AI299911 | *PPP3CA* | protein phosphatase 3 (formerly 2B), catalytic subunit, alpha isoform |

(continued)

| | | | |
|---|---|---|---|
| **DOWN-REGULATED GENES** | | | |
| 665 | X07979 | *ITGBI* | integrin, beta 1 |
| 666 | W45244 | C3 | complement component 3 |
| 667 | AI245516 | | EST |
| 668 | AA907519 | *C3ORF4* | chromosome 3 open reading frame 4 |
| 669 | D42041 | *KIAA0088* | KIAA0088 protein |
| 670 | AI300002 | *CCNI* | cyclin I |
| 671 | AI338165 | *HEF1* | enhancer of filamentation 1 (cas-like docking; Crk-associated substrate related) |
| 672 | AI312689 | *HE1* | epididymal secretory protein (19.5kD) |
| 673 | NM_00607 7 | *CBARA1* | calcium binding atopy-related autoantigen 1 |
| 674 | AF131847 | *MRG15* | MORF-related gene 15 |
| 675 | AA676585 | *NPM1* | nucleophosmin (nucleolar phosphoprotein B23, numatrin) |
| 676 | U85658 | *TFAP2C* | transcription factor AP-2 gamma (activating enhancer-binding protein 2 gamma) |
| 677 | AB011090 | *KIAA0518* | Max-interacting protein |
| 678 | U93867 | *RPC62* | polymerase (RNA) III (DNA directed) (62kD) |
| 679 | Z111531 | *EEF1G* | eukaryotic translation elongation factor 1 gamma |
| 680 | AA676322 | *MTF1* | metal-regulatory transcription factor 1 |
| 681 | AI339006 | | DKFZp586L1121 |

**Table 7** A list of 168 Candidate Genes for liver metastasis

| PNC Assign ment | GenBank ID | Symbol | Gene Name |
|---|---|---|---|
| **UP-REGULATED GENES** | | | |
| 682 | U63743 | *KNSL6* | kinesin-like 6 (mitotic centromere-associated kinesin) |
| 683 | U12707 | *WAS* | Wiskott-Aldrich syndrome (eczema-thrombocytopenia) |
| 684 | AA904028 | *PAPPA* | pregnancy-associated plasma protein A |
| 685 | T69711 | | EST |
| 686 | AI338282 | *TIGA1* | Homo sapiens mRNA; cDNA DKFZp566L203 (from clone DKFZp566L203) |
| 687 | AA843756 | *ID2* | inhibitor of DNA binding 2, dominant negative helix-loop-helix protein |
| 688 | AF076483 | *PGLYRP* | peptidoglycan recognition protein |
| 689 | AA447852 | *PC326* | PC326 protein |
| 690 | L13939 | *AP1B1* | adaptor-related protein complex 1, beta 1 subunit |
| 691 | AI344213 | *CCS* | copper chaperone for superoxide dismutase |
| 692 | X74929 | *KRT8* | keratin 8 |
| 693 | U92459 | *GRM8* | glutamate receptor, metabotropic 8 |
| 694 | AA078295 | | ESTs |
| 695 | AA084871 | *YKT6* | SNARE protein |
| 696 | M26252 | *PKM2* | pyruvate kinase, muscle |
| 697 | AI280555 | *KIAA0860* | KIAA0860 protein |
| 698 | U09278 | *FAP* | fibroblast activation protein, alpha |
| 699 | AA989386 | | EST |
| 700 | U01184 | *FLII* | flightless I (Drosophila) homolog |
| 701 | NM_0164 01 | *HSPC138* | hypothetical protein |
| 702 | AW24510 1 | *E2IG3* | putative nucleotide binding protein, estradiol-induced |
| 703 | U47025 | *PYGB* | phosphorylase, glycogen; brain |
| 704 | Z21507 | *EEF1D* | eukaryotic translation elongation factor 1 delta |

(continued)

| PNC Assign ment | GenBank ID | Symbol | Gene Name |
|---|---|---|---|
| **UP-REGULATED GENES** | | | |
| 705 | U38320 | *MMP19* | matrix metalloproteinase 19 |
| 706 | AA233644 | *PPP1CC* | protein phosphatase 1, catalytic subunit, gamma isoform |
| 707 | L40401 | *ZAP128* | peroxisomal long-chain acyl-coA thioesterase ; putative protein |
| 708 | AI365683 | | Homo sapiens PAC clone RP4-751H13 from 7q35-qter |
| 709 | AF039690 | *SDCCAG8* | serologically defined colon cancer antigen 8 |
| 710 | L19067 | *RELA* | v-rel avian reticuloendotheliosis viral oncogene homolog A |
| 711 | U48734 | *ACTN4* | actinin, alpha 4 |
| 712 | M22324 | *ANPEP* | alanyl (membrane) aminopeptidase |
| 713 | AA921921 | *KIAA0414* | KIAA0414 protein |
| 714 | X97630 | *EMK1* | ELKL motif kinase |
| 715 | AJ002308 | *SYNGR2* | synaptogyrin 2 |
| 716 | AA447019 | *MAN1B1* | mannosidase, alpha, class 1B, member 1 |
| 717 | M98252 | *PLOD* | procollagen-lysine, 2-oxoglutarate 5-dioxygenase |
| 718 | H48649 | *FGG* | fibrinogen, gamma polypeptide |
| 719 | AI139231 | *FBL* | fibrillarin |
| 720 | AA249454 | | ESTs, Weakly similar to KIAA0227 [H.sapiens] |
| 721 | U89278 | *EDR2* | early development regulator 2 (homolog of polyhomeotic 2) |
| 722 | M24398 | *PTMS* | parathymosin |
| 723 | L41668 | *GALE* | galactose-4-epimerase, UDP- |
| 724 | D78298 | *VLCAD* | very-long-chain acyl-CoA dehydrogenase |
| 725 | X89602 | *HSRTSBET A* | rTS beta protein |
| 726 | M91029 | *AMPD2* | adenosine monophosphate deaminase 2 (isoform L) |
| 727 | X73478 | *PPP2R4* | protein phosphatase 2A, regulatory subunit B' (PR 53) |
| 728 | AI189477 | *IDH2* | isocitrate dehydrogenase 2 (NADP+), mitochondrial |
| 729 | D30612 | *ZNF282* | zinc finger protein 282 |
| 730 | AA506972 | *KIAA0668* | KIAA0668 protein |
| 731 | AA404724 | *GPRK7* | G protein-coupled receptor kinase 7 |
| 732 | AB001451 | *SLI* | neuronal Shc adaptor homolog |
| 733 | AL120683 | *LASS2* | LAG 1 longevity assurance homolog 2 (S. cerevisiae) |
| 734 | H20386 | *MYG1* | MYG1 protein |
| 735 | AA47862 | *KIAA0974* | KIAA0974 protein |
| 736 | AF075590 | *BZRP* | benzodiazapine receptor (peripheral) |
| 737 | AA748421 | *TFR2* | transferrin receptor 2 |
| 738 | AA639771 | *MMP12* | matrix metalloproteinase 12 (macrophage elastase) |
| 739 | AI218495 | | ESTs, Moderately similar to integral inner nuclear membrane protein MAN1 |
| 740 | N80334 | *DKFZP586 00223* | hypothetical protein |
| 741 | AA847660 | *HEXA* | hexosaminidase A (alpha polypeptide) |
| **DOWN-REGULATED GENES** | | | |
| 742 | S74678 | *HNRPK* | heterogeneous nuclear ribonucleoprotein K |
| 743 | D56784 | *DEK* | DEK oncogene (DNA binding) |
| 744 | U31383 | *GNG10* | guanine nucleotide binding protein 10 |
| 745 | H06970 | *STK24* | serine/threonine kinase 24 (Ste20, yeast homolog) |
| 746 | AF038954 | *ATP6J* | ATPase, H+ transporting, lysosomal (vacuolar proton pump), member J |

(continued)

**DOWN-REGULATED GENES**

| | | | |
|---|---|---|---|
| 747 | W19984 | *DREV1* | CGI-81 protein |
| 748 | AA282650 | *SAC1* | Suppressor of actin 1 |
| 749 | U16738 | *RPL14* | ribosomal protein L14 |
| 750 | AA614311 | *VCP* | valosin-containing protein |
| 751 | AF006088 | *ARPC5* | actin related protein 2/3 complex, subunit 5 (16 kD) |
| 752 | AF007871 | *DYT1* | dystonia 1, torsion (autosomal dominant; torsin A) |
| 753 | D21090 | *RAD23B* | RAD23 (S. cerevisiae) homolog B |
| 754 | AA910279 | *STAU* | staufen (Drosophila, RNA-binding protein) |
| 755 | AA226073 | *ITM2C* | integral membrane protein 2C |
| 756 | AA583455 | *RNF7* | ring finger protein 7 |
| 757 | AA731151 | *KIAA1085* | KIAA1085 protein |
| 758 | U14575 | *PPP1R8* | protein phosphatase 1, regulatory (inhibitor) subunit 8 |
| 759 | M81637 | *GCL* | grancalcin |
| 760 | L37368 | *RNPS1* | RNA-binding protein S1, serine-rich domain |
| 761 | AK000403 | *FLJ20396* | hypothetical protein FLJ20396 |
| 762 | D13315 | *GLO1* | glyoxalase I |
| 763 | U66818 | *UBE2I* | ubiquitin-conjugating enzyme E2I (homologous to yeast UBC9) |
| 764 | X56351 | *ALAS1* | aminolevulinate, delta-, synthase 1 |
| 765 | L08424 | *ASCL1* | achaete-scute complex (Drosophila) homolog-like 1 |
| 766 | X15187 | *TRA1* | tumor rejection antigen (gp96) 1 |
| 767 | U33286 | *CSE1L* | chromosome segregation 1 (yeast homolog)-like |
| 768 | AA747290 | *RPS15A* | ribosomal protein S15a |
| 769 | AI148832 | *KIAA1209* | KIAA1209 protein |
| 770 | S65738 | *ADF* | destrin (actin depolymerizing factor) |
| 771 | X53586 | *ITGA6* | integrin, alpha 6 |
| 772 | U31906 | *GOLGA4* | golgi autoantigen, golgin subfamily a, 4 |
| 773 | AA664213 | *DKC1* | dyskeratosis congenita 1, dyskerin enhancer of filamentation 1 (cas-like docking; Crk-associated |
| 774 | AI338165 | *HEF1* | substrate related) |
| 775 | W74416 | *LOC51126* | N-terminal acetyltransferase complex ard1 subunit |
| 776 | AI125978 | *SNX2* | sorting nexin 2 |
| 777 | H96478 | | EST |
| 778 | U46570 | *TTC1* | tetratricopeptide repeat domain 1 |
| 779 | U21242 | *GTF2A2* | general transcription factor IIA, 2 (12kD subunit) |
| 780 | W95089 | *HSPC033* | HSPC033 protein |
| 781 | D55654 | *MDH1* | malate dehydrogenase 1, NAD (soluble) |
| 782 | AF072860 | *PRKRA* | protein kinase, interferon-inducible double stranded RNA dependent activator |
| 783 | AF042081 | *SH3BGRL* | SH3 domain binding glutamic acid-rich protein like |
| 784 | D63881 | *KIAA0160* | KIAA0160 protein |
| 785 | AA195740 | | Homo sapiens mRNA full length insert cDNA clone EUROIMAGE 41832 |
| 786 | M36341 | *ARF4* | ADP-ribosylation factor 4 |
| 787 | C06051 | *JAK1* | Janus kinase 1 (a protein tyrosine kinase) |
| 788 | D28473 | *LARS* | isoleucine-tRNA synthetase |
| 789 | R23830 | | ESTs |
| 790 | U51166 | *TDG* | thymine-DNA glycosylase |
| 791 | AA128470 | *DSP* | desmoplakin (DPI, DPII) |
| 792 | M77698 | *YY1* | YY 1 transcription factor |

(continued)

| DOWN-REGULATED GENES | | | |
|---|---|---|---|
| 793 | AI72932 | *BAG5* | BCL2-associated athanogene 5 |
| 794 | U45879 | *BIRC2* | baculoviral IAP repeat-containing 2 |
| 795 | Z35491 | *BAG1* | BCL2-associated athanogene |
| 796 | AF016507 | *CTBP2* | C-terminal binding protein 2 |
| 797 | X89478 | *HRB* | HIV Rev binding protein |
| 798 | X06323 | *MRPL3* | mitochondrial ribosomal protein L3 |
| 799 | M29065 | *HNRPA2B1* | heterogeneous nuclear ribonucleoprotein A2/B1 |
| 800 | AA431846 | *LOC51187* | 60S ribosomal protein L30 isolog |
| 801 | E02628 | | polypeptide chain elongation factor 1 alpha |
| 802 | AI349804 | | EST |
| 803 | X99584 | *SMT3H1* | SMT3 (suppressor of mif two 3, yeast) homolog 1 |
| 804 | D13630 | *KIAA0005* | KIAA0005 gene product |
| 805 | U24223 | *PCBP1* | poly(rC)-binding protein 1 |
| 806 | AA315729 | | FLJ23197 |
| 807 | AA401318 | *DKFZP566 D193* | DKFZP566D193 protein |
| 808 | AA524350 | *LOC51719* | MO25 protein |
| 809 | AB004857 | *SLC11A2* | solute carrier family 11, member 2 |
| 810 | AA379042 | *PUM2* | Pumilio (Drosophila) homolog 2 |
| 811 | AW77914 2 | *HUMAGCG B* | chromosome 3p21.1 gene sequence |
| 812 | R39044 | | Homo sapiens clone 25194 mRNA sequence |
| 813 | M58458 | *RPS4X* | ribosomal protein S4, X-linked |
| 814 | H89110 | | ESTs |
| 815 | U47077 | *PRKDC* | protein kinase, DNA-activated, catalytic polypeptide |
| 816 | AA236252 | *ASH2L* | ash2 (absent, small, or homeotic, Drosophila, homolog)-like |
| 817 | D50683 | *TGFBR2* | transforming growth factor, beta receptor II (70-80kD) |
| 818 | M61199 | *SSFA2* | sperm specific antigen 2 |
| 819 | U56637 | *CAPZA1* | capping protein (actin filament) muscle Z-line, alpha 1 |
| 820 | AA514818 | *KIAA0068* | KIAA0068 protein |
| 821 | N45298 | *ARHGEF12* | Rho guanine exchange factor (GEF) 12 |
| 822 | X76104 | *DAPK1* | death-associated protein kinase 1 |
| 823 | D14812 | *KIAA0026* | MORF-related gene X |
| 824 | AA357508 | | Homo sapiens clone 24711 mRNA sequence |
| 825 | U96915 | *SAP18* | sin3-associated polypeptide, 18kD |
| 826 | D10522 | *MACS* | myristoylated alanine-rich protein kinase C substrate (MARCKS, 80K-L) |
| 827 | N46856 | | Homo sapiens cDNA: FLJ23091 fis, clone LNG07220 |
| 828 | D26125 | *AKR1C4* | aldo-keto reductase family 1, member C4 |
| 829 | AI085802 | *CAV2* | Caveolin 2 |
| 830 | AI289407 | *ZNF207* | zinc finger protein 207 |
| 831 | U54831 | *TOP2B* | topoisomerase (DNA) II beta (180kD) |
| 832 | AA281115 | *UBQLN1* | ubiquilin 1 |
| 833 | N41902 | *CLTH* | Clathrin assembly lymphoid-myeloid leukemia gene |
| 834 | AA432312 | *TSPYL* | TSPY-like |
| 835 | AF006516 | *SSH3BP1* | spectrin SH3 domain binding protein 1 |
| 836 | AA706503 | *EEF1A1* | eukaryotic translation elongation factor 1 alpha 1 |
| 837 | N95414 | | ESTs |
| 838 | M20472 | *CLTA* | clathrin, light polypeptide (Lca) |
| 839 | AI078833 | *TAX1BP1* | Tax1 (human T-cell leukemia virus type I) binding protein 1 |

(continued)

| | | | |
|---|---|---|---|
| **DOWN-REGULATED GENES** | | | |
| 840 | U09953 | *RPL9* | ribosomal protein L9 |
| 841 | U44772 | *PPT1* | palmitoyl-protein thioesterase 1 |
| 842 | AA973853 | | Homo sapiens cDNA FLJ20532 fis, clone KAT10877 |
| 843 | U81504 | *AP3B1* | adaptor-related protein complex 3, beta 1 subunit |
| 844 | AA634090 | *HNRPA1* | heterogeneous nuclear ribonucleoprotein A1 |
| 845 | U83463 | *SDCBP* | syndecan binding protein (syntenin) |
| 846 | AI092703 | *FBXW1B* | f-box and WD-40 domain protein 1B |
| 847 | AF052113 | *Rab14* | GTPase Rab 14 |
| 848 | AF007216 | *SLC4A4* | solute carrier family 4, sodium bicarbonate cotransporter, member 4 |
| 849 | AA809819 | *CREG* | cellular repressor of E1A-stimulated genes |

**Table 8** A list of 84 Candidate Genes for prognosis

| PNC Assign ment | GenBank ID | Symbol | Gene Name |
|---|---|---|---|
| up-regulated in late recurrence cases | | | |
| 850 | AF049884 | *ARGBP2* | Arg/Abl-interacting protein ArgBP2 |
| 851 | NM_0060 77 | *CBARA1* | calcium binding atopy-related autoantigen 1 |
| 852 | Z11531 | *EEF1G* | eukaryotic translation elongation factor 1 gamma |
| 853 | AW15720 3 | *LCAT* | lecithin-cholesterol acyltransferase |
| 854 | AI123363 | *RPL23A* | ribosomal protein L23a |
| 855 | X53777 | *RPL17* | ribosomal protein L17 |
| 856 | U16798 | *ATP1A1* | ATPase, Na+/K+ transporting, alpha 1 polypeptide |
| 857 | X76013 | *QARS* | glutaminyl-tRNA synthetase |
| 858 | AF075590 | *BZRP* | benzodiazapine receptor (peripheral) |
| 859 | L38995 | *TUFM* | Tu translation elongation factor, mitochondrial |
| 860 | H89783 | *SERPINA4* | serine (or cysteine) proteinase inhibitor, clade A , member 4 |
| 861 | D83782 | *SCAP* | SREBP CLEAVAGE-ACTIVATING PROTEIN |
| 862 | M75126 | *HK1* | hexokinase 1 |
| 863 | AA936173 | *RPS11* | ribosomal protein S 11 |
| 864 | AA488766 | *SYNGR2* | synaptogyrin 2 |
| 865 | M60922 | *FLOT2* | flotillin 2 |
| 866 | D26600 | *PSMB4* | proteasome (prosome, macropain) subunit, beta type, 4 |
| 867 | L19711 | *DAG1* | dystroglycan 1 (dystrophin-associated glycoprotein 1) |
| 868 | AI148194 | | Novel human gene mapping to chomosome 22 |
| 869 | X57398 | *PM5* | pM5 protein |
| 870 | M17886 | *RPLP1* | ribosomal protein, large, P1 |
| 871 | L14778 | *PPP3CA* | protein phosphatase 3 (formerly 2B), catalytic subunit, alpha isoform (calcineurin A alpha) |
| 872 | AA156481 | *RPL13A* | ribosomal protein L 13 a |
| 873 | AA083406 | *EIF3S8* | eukaryotic translation initiation factor 3, subunit 8 (110kD) |
| 874 | AF000984 | *DBY* | DEAD/H (Asp-Glu-Ala-Asp/His) box polypeptide, Y chromosome |
| 875 | X17206 | *RPS2* | ribosomal protein S2 |
| 876 | W45522 | *LOC51189* | ATPase inhibitor precursor |
| 877 | X83218 | *ATP50* | ATP synthase, H+ transporting, mitochondrial F1 complex, O subunit |
| 878 | AI246699 | *CATX-8* | CATX-8 protein |
| 879 | AA029875 | *CASP4* | caspase 4, apoptosis-related cysteine protease |
| 880 | AI366139 | *MAC30* | hypothetical protein |

(continued)

| PNC Assign ment | GenBank ID | Symbol | Gene Name |
|---|---|---|---|
| up-regulated in late recurrence cases | | | |
| 881 | U46191 | *RAGE* | renal tumor antigen |
| 882 | AA487669 | *GSTM1* | glutathione S-transferase M1 |
| 883 | AI131289 | *RPLP2* | ribosomal protein, large P2 |
| 884 | AI299327 | | ESTs |
| 885 | AA922716 | *PRKACB* | protein kinase, cAMP-dependent, catalytic, beta |
| 886 | AA845165 | *PRSS1* | protease, serine, 1 (trypsin 1) |
| 887 | AA877534 | *GPRC5C* | G protein-coupled receptor, family C, group 5, member C |
| 888 | C01335 | | ESTs, Weakly similar to FLDED [H.sapiens] |
| 889 | Z26876 | *RPL38* | ribosomal protein L38 |
| 890 | AI080640 | *AGR2* | anterior gradient 2 (Xenepus laevis) homolog |
| 891 | X04588 | *TPM3* | 2.5kb mRNA for cytoskeletal tropomyosin TM30 |
| 892 | D30949 | | Homo sapiens cDNA FLJ12750 fis, clone NT2RP2001168, weakly similar to VERPROLIN |
| 893 | Z11559 | *ACO1* | aconitase 1, soluble |
| up-regulated in early recurrence cases | | | |
| 894 | AA700379 | *MTMR1* | myotubularin related protein 1 |
| 895 | AI340331 | *HT010* | uncharacterized hypothalamus protein HT010 |
| 896 | AA459167 | *NPD002* | NPD002 protein |
| 897 | AI014395 | *YME1L1* | YME1 (S.cerevisiae)-like 1 |
| 898 | M94083 | *CCT6A* | chaperonin containing TCP1, subunit 6A (zeta 1) |
| 899 | M22382 | *HSPD1* | heat shock 60kD protein 1 (chaperonin) |
| 900 | AA150867 | *TIMM9* | translocase of inner mitochondrial membrane 9 (yeast) homolog |
| 901 | L76687 | *GRB14* | growth factor receptor-bound protein 14 |
| 902 | T70782 | *FLJ10803* | hypothetical protein FLJ10803 |
| 903 | AI018632 | *LAMP1* | lysosomal-associated membrane protein 1 |
| 904 | AA531437 | *MLLT4* | myeloid/lymphoid or mixed-lineage leukemia translocated to, 4 |
| 905 | AI075048 | *CTSB* | cathepsin B |
| 906 | AL031668 | *RALY* | RNA-binding protein (autoantigenic) |
| 907 | AI357601 | *RPL37A* | ribosomal protein L37a |
| 908 | U51586 | *SIAHBP1* | siah binding protein 1 |
| 909 | AF004430 | *TPD52L2* | tumor protein D52-like 2 |
| 910 | AI279562 | *KIAA0469* | KIAA0469 gene product |
| 911 | M11717 | *HSPA1A* | heat shock 70kD protein 1A |
| 912 | AF015767 | *BRE* | brain and reproductive organ-expressed (TNFRSF1A modulator) |
| 913 | X06323 | *MRPL3* | mitochondrial ribosomal protein L3 |
| 914 | AI305234 | | ESTs |
| 915 | W24533 | *GRB10* | growth factor receptor-bound protein 10 |
| 916 | AA504081 | *CSH2* | chorionic somatomammotropin hormone 2 |
| 917 | AA778572 | *HSPC164* | hypothetical protein |
| 918 | D11999 | *AARS* | glutaminase |
| 919 | D32050 | *AARS* | alanyl-tRNA synthetase |
| 920 | D63997 | *GOLGA3* | golgi autoantigen, golgin subfamily a, 3 |
| 921 | R64726 | | Homo sapiens cDNA: FLJ23591 fis, clone LNG14729 |
| 922 | M61715 | *WARS* | tryptophanyl-tRNA synthetase |
| 923 | AI090753 | *SHMT2* | serine hydroxymethyltransferase 2 (mitochondrial) |
| 924 | AI289991 | *DKFZP761C 169* | hypothetical protein DKFZp761C169 |

(continued)

| PNC Assign ment | GenBank ID | Symbol | Gene Name |
|---|---|---|---|
| up-regulated in late recurrence cases | | | |
| 925 | AA345061 | *KIAA0903* | KIAA0903 protein |
| 926 | AA255699 | | Human DNA sequence from clone RP3-324O17 on chromosome 20 |
| 927 | H73961 | *ARPC3* | actin related protein 2/3 complex, subunit 3 (21 kD) |
| 928 | D87666 | *GPI* | glucose phosphate isomerase |
| 929 | A1075943 | *SENP2* | sentrin-specific protease |
| 930 | D87989 | *UGTREL1* | UDP-galactose transporter related |
| 931 | D86956 | *HSP105B* | heat shock 105kD |
| 932 | L13740 | *NR4A1* | nuclear receptor subfamily 4, group A, member 1 |
| 933 | AA320379 | *POH1* | 26S proteasome-associated pad1 homolog |

Industrial Applicability

The gene-expression analysis of pancreatic cancer described herein, obtained through a combination of laser-capture dissection and genome-wide cDNA microarray, has identified specific genes as targets for cancer prevention and therapy. Based on the expression of a subset of these differentially expressed genes, the present invention provides a molecular diagnostic markers for identifying or detecting pancreatic ductal adenocarcinoma.

The methods described herein are also useful in the identification of additional molecular targets for prevention, diagnosis and treatment of pancreatic ductal adenocarcinoma. The data reported herein add to a comprehensive understanding of pancreatic cancer, facilitate development of novel diagnostic strategies, and provide clues for identification of molecular targets for therapeutic drugs and preventative agents. Such information contributes to a more profound understanding of pancreatic tumorigenesis, and provide indicators for developing novel strategies for diagnosis, treatment, and ultimately prevention of pancreatic ductal adenocarcinoma.

**REFERENCES**

1. Greenlee, R. T., Hill-Harmon, M. B., Murray; T., and Thun, M. Cancer statistics, 2001. CA Cancer J Clin, *51*: 15-36, 2001.

2. Klinkenbijl, J. H., Jeekel, J., Sahmoud, T., van Pel, R., Couvreur, M. L., Veenhof, C. H., Arnaud, J. P., Gonzalez, D. G., de Wit, L. T., Hennipman, A., and Wils, J. Adjuvant radiotherapy and 5-fluorouracil after curative resection of cancer of the pancreas and periampullary region: phase III trial of the EORTC gastrointestinal tract cancer cooperative group. Ann Surg, *230*: 776-782; discussion 782-774, 1999.

3. Ishiguro, H., Shimokawa, T., Tsunoda, T., Tanaka, T., Fujii, Y., Nakamura, Y., and Furukawa, Y. Isolation of HELAD1, a novel human helicase gene up-regulated in colorectal carcinomas. Oncogene, 21: 6387-6394, 2002.

4. Yagyu, R., Hamamoto, R., Furukawa, Y., Okabe, H., Yamamura, T., and Nakamura, Y. Isolation and characterization of a novel human gene, VANGL1, as a therapeutic target for hepatocellular carcinoma. Int J Oncol, 20: 1173-1178, 2002.

5. Iacobuzio-Donahue, C. A., Maitra, A., Shen-Ong, G. L., van Heek, T., Ashfaq, R., Meyer, R., Walter, K., Berg, K., Hollingsworth, M. A., Cameron, J. L., Yeo, C. J., Kern, S. E., Goggins, M., and Hruban, R. H. Discovery of novel tumor markers of PNC using global gene expression technology. Am J Pathol, 160: 1239-1249, 2002.

6. Han, H., Bearss, D. J., Browne, L. W., Calaluce, R., Nagle, R. B., and Von Hoff, D. D. Identification of differentially expressed genes in PNC cells using cDNA microarray. Cancer Res, 62: 2890-2896, 2002.

7. Bockman, D. E., Boydston, W. R., and Parsa, I. Architecture of human pancreas: implications for early changes in pancreatic disease. Gastroenterology, 85: 55-61, 1983.

8. Hruban, R. H., Wilentz, R. E., and Kern, S. E. Genetic progression in the pancreatic ducts. Am J Pathol,156: 1821-1825, 2000.

9. Kitahara, O., Furukawa, Y., Tanaka, T., Kihara, C., Ono, K., Yanagawa, R., Nita, M. E., Takagi, T., Nakamura, Y., and Tsunoda, T. Alterations of gene expression during colorectal carcinogenesis revealed by cDNA microarrays after laser-capture microdissection of tumor tissues and normal epithelia. Cancer Res, 61: 3544-3549, 2001.

10. Gjerdrum, L. M., Lielpetere, I., Rasmussen, L. M., Bendix, K., and Hamilton-Dutoit, S. Laser-assisted microdissection of membrane-mounted paraffin sections for polymerase chain reaction analysis: identification of cell populations using immunohistochemistry and in situ hybridization. J Mol Diagn, 3: 105-110, 2001.
11. Ono, K., Tanaka, T., Tsunoda, T., Kitahara, O., Kihara, C., Okamoto, A., Ochiai, K., Takagi, T., and Nakamura, Y. Identification by cDNA microarray of genes involved in ovarian carcinogenesis. Cancer Res, 60: 5007-5011, 2000.
12. Saito-Hisaminato, A., Katagiri, T., Kakiuchi, S., Nakamura, T., Tsunoda, T., and Nakamura, Y. Genome-wide profiling of gene expression in 29 normal human tissues with a cDNA microarray. DNA Res, 9: 35-45, 2002.
13. DiMagno EP, Reber HA, Tempero MA. AGA technical review on the epidemiology, diagnosis, and treatment of pancreatic ductal adenocarcinoma. American Gastroenterological Association. Gastroenterology. 1999 Dec;117 (6):1464-84. Review.
14. Brentnall TA, Bronner MP, Byrd DR, Haggitt RC, Kimmey MB. Early diagnosis and treatment of pancreatic dysplasia in patients with a family history of pancreatic cancer. Ann Intern Med. 1999 Aug 17;131(4):247-55.
15. Rosenberg L. Pancreatic cancer: a review of emerging therapies. Drugs. 2000 May;59(5):1071-89. Review.
16. Hao D, Rowinsky EK. Inhibiting signal transduction: recent advances in the development of receptor tyrosine kinase and Ras inhibitors. Cancer Invest. 2002;20(3):387-404. Review.
17. Laheru D, Biedrzycki B, Jaffee EM. Immunologic approaches to the management of pancreatic cancer. Cancer J. 2001 Jul-Aug;7(4):324-37. Review.
18. Crnogorac-Jurcevic T, Efthimiou E, Nielsen T, Loader J, Terris B, Stamp G, Baron A, Scarpa A, Lemoine NR. Expression profiling of microdissected pancreatic adenocarcinomas. Oncogene. 2002 Jul 4;21 (29):4587-94.
19. Ciardiello F, Tortora G. A novel approach in the treatment of cancer: targeting the epidermal growth factor receptor. Clin Cancer Res. 2001 Oct;7(10):2958-70. Review.
20. Slamon DJ, Leyland-Jones B, Shak S, Fuchs H, Paton V, Bajamonde A, Fleming T, Eiermann W, Wolter J, Pegram M, Baselga J, Norton L. Use of chemotherapy plus a monoclonal antibody against HER2 for metastatic breast cancer that overexpresses HER2. N Engl J Med. 2001 Mar 15;344(11):783-92.
21. Rehwald U, Schulz H, Reiser M, Sieber M, Staak JO, Morschhauser F, Driessen C, Rudiger T, Muller-Hermelink K, Diehl V, Engert A. Treatment of relapsed CD20+ Hodgkin lymphoma with the monoclonal antibody rituximab is effective and well tolerated: results of a phase 2 trial of the German Hodgkin Lymphoma Study Group. Blood. 2003 Jan 15;101(2):420-424.
22. Violette S, Festor E, Pandrea-Vasile I, Mitchell V, Adida C, Dussaulx E, Laçorte JM, Chambaz J, Lacasa M, Lesuffleur T. Reg IV, a new member of the regenerating gene family, is overexpressed in colorectal carcinomas. Int J Cancer. 2003 Jan 10;103(2):185-93.
23. Kullander K, Mather NK, Diella F, Dottori M, Boyd AW, Klein R. Kinase-dependent and kinase-independent functions of EphA4 receptors in major axon tract formation in vivo. Neuron. 2001 Jan;29(1):73-84.
24. Rozenblum E, Schutte M, Goggins M, Hahn SA, Panzer S, Zahurak M, Goodman SN, Sohn TA, Hruban RH, Yeo CJ, Kem SE. Tumor-suppressive pathways in pancreatic carcinoma. Cancer Res. 1997 May 1;57(9):1731-4.
25. Goggins M, Hruban RH, Kern SE. BRCA2 is inactivated late in the development of pancreatic intraepithelial neoplasia: evidence and implications. Am J Pathol. 2000 May;156(5):1767-71.
26. Ishiguro H, Tsunoda T, Tanaka, T, Fujii Y, Nakamura Y, Furukawa Y. Identification of AXUD1, a novel human gene induced by AXIN1 and its reduced expression in human carcinomas of the lung, liver, colon and kidney. Oncogene. 2001 Aug 16;20(36):5062-6.
27. Satoh S, Daigo Y, Furukawa Y, Kato T, Miwa N, Nishiwaki T, Kawasoe T, Ishiguro H, Fujita M, Tokino T, Sasaki Y, Imaoka S, Murata M, Shimano T, Yamaoka Y, Nakamura Y. AXIN1 mutations in hepatocellular carcinomas, and growth suppression in cancer cells by virus-mediated transfer of AXIN1. Nat Genet. 2000 Mar;24(3):245-50.
28. Yuan BZ, Miller MJ, Keck CL, Zimonjic DB, Thorgeirsson SS, Popescu NC. Cloning, characterization, and chromosomal localization of a gene frequently deleted in human liver cancer (DLC-1) homologous to rat RhoGAP. Cancer Res. 1998 May 15;58(10):2196-9.
29. Ng IO, Liang ZD, Cao L, Lee TK. DLC-1 is deleted in primary hepatocellular carcinoma and exerts inhibitory effects on the proliferation of hepatoma cell lines with deleted DLC-1. Cancer Res. 2000 Dec 1;60(23):6581-4.
30. Fang G, Kim CN, Perkins CL, Ramadevi N, Winton E, Wittmann S and Bhalla KN. (2000). Blood, 96, 2246-2253.
31. Gianni L. (2002). Oncology, 63 Suppl 1, 47-56.
32. Klejman A, Rushen L, Morrione A, Slupianek A and Skorski T. (2002). Oncogene, 21, 5868-5876.

## Claims

**1.** A method of diagnosing pancreatic ductal adenocarcinoma in a subject, comprising determining the level of expression of the PNC-associated gene PNC157 (REG IV) in a patient-derived biological sample comprising a pancreatic ductal epithelial cell, wherein an increase of said level of expression compared to the normal control level indicates that said subject suffers from pancreatic ductal adenocarcinoma, wherein said level of expression is determined by detecting the mRNA of PNC157 or detecting the protein encoded by PNC157.

**2.** The method of claim 1, wherein said increase is at least 10% greater than said normal control level.

**3.** The method of claim 1, wherein said level of expression is determined by detecting hybridization of a PNC157 (REG IV) probe to a gene transcript of the patient-derived biological sample.

**4.** The method of claim 3, wherein said hybridization step is carried out on a DNA array.

**5.** An in vitro method of screening for a compound binding to a polypeptide encoded by PNC157 (REG IV) useful in the treatment or prevention of pancreatic ductal adenocarcinoma, said method comprising the steps of:

(a) contacting a test compound with a polypeptide encoded by PNC157;
(b) detecting the binding activity between the polypeptide and the test compound; and
(c) selecting a compound that binds to a polypeptide encoded by PNC157.

**6.** An in vitro method of screening for a compound reducing the expression level of PNC157 (REG IV)useful in the treatment or prevention of pancreatic ductal adenocarcinoma, said method comprising the steps of:

(a) contacting a candidate compound with a cell expressing PNC157; and

(b) selecting a compound that reduces the expression level of PNC157.

7. The method of claim 6, wherein said cell comprises a pancreatic ductal adenocarcinoma cell.

8. Use of

(a) an antisense composition, said composition comprising a nucleotide sequence complementary to a coding sequence of PNC157 (REG IV);
(b) a siRNA composition, wherein said composition reduces the expression of PNC 157 (REG IV); or
(c) an antibody or fragment thereof that binds to a protein encoded by PNC157 for the preparation of a pharmaceutical composition for treating or preventing pancreatic ductal adenocarcinoma in a subject.

9. A pharmaceutical composition for treating or preventing pancreatic ductal adenocarcinoma in a subject comprising

(a) an antisense polynucleotide or small interfering RNA against PNC157 (REG IV); or
(b) an antibody or fragment thereof that binds to a protein encoded by PNC157 (REG IV),
and a pharmaceutically acceptable carrier.

## Patentansprüche

1. Verfahren zum Diagnostizieren von duktalem Adenokarzinom des Pankreas in einem Individuum, umfassend das Bestimmen des Expressionsspiegels des PNCassoziierten Gens PNC157 (REG IV) in einer von einem Patienten stammenden biologischen Probe, welche eine duktale Epithelzelle des Pankreas umfasst, wobei ein Anstieg des Expressionsspiegels verglichen mit dem normalen Kontrollspiegel anzeigt, dass das Individuum an duktalem Adenokarzinom des Pankreas leidet, wobei der Expressionsspiegel durch Nachweis der mRNA von PNC157 oder des von PNC157 codierten Proteins bestimmt wird.

2. Verfahren nach Anspruch 1, wobei der Anstieg mindestens 10% größer ist als der normale Kontrollspiegel.

3. Verfahren nach Anspruch 1, wobei der Expressionsspiegel durch Nachweisen der Hybridisierung einer PNC157 (REG IV)-Sonde an ein Gentranskript in der von dem Patienten stammenden biologischen Probe bestimmt wird.

4. Verfahren nach Anpruch 3, wobei der Hybridisierungsschritt auf einem DNA-Array ausgeführt wird.

5. In vitro-Verfahren zum Screenen auf eine Verbindung, welche an ein von PNC157 (REG IV) codiertes Polypeptid bindet, welches für die Behandlung oder Prävention von duktalem Adenokarzinom des Pankreas nützlich ist, wobei das Verfahren die Schritte umfasst:

(a) Inkontaktbringen einer Testverbindung mit einem Polypeptid, welches von PNC157 codiert wird;
(b) Nachweisen der Bindungsaktivität zwischen dem Polypeptid und der Testverbindung; und
(c) Auswählen einer Verbindung, welche an ein Polypeptid bindet, welches von PNC157 codiert wird.

6. In vitro-Verfahren zum Screenen auf eine Verbindung, welche den Expressionsspiegel von PNC157 (REG IV) vermindert, und welche für die Behandlung oder Prävention von duktalem Adenokarzinom des Pankreas nützlich ist, wobei das Verfahren die Schritte umfasst:

(a) Inkontaktbringen einer Testverbindung mit einer Zelle, welche PNC157 exprimiert; und
(b) Auswählen einer Verbindung, welche den Expressionsspiegel von PNC157 vermindert.

7. Verfahren nach Anspruch 6, wobei die Zelle eine Zelle eines duktalen Adenokarzinoms des Pankreas umfasst.

8. Verwendung

(a) einer Antisense-Zusammensetzung, welche eine Nucleotidsequenz umfasst, welche komplementär zu einer codierenden Sequenz von PNC157 (REG IV) ist;
(b) einer siRNA-Zusammensetzung, wobei die Zusammensetzung die Expression von PNC157 (REG IV) vermindert; oder

(c) eines Antikörpers oder eines Fragments davon, welcher an ein Protein bindet, welches von PNC157 codiert wird
für die Herstellung eines Arzneimittels zur Behandlung oder Prävention von duktalem Adenokarzinom des Pankreas in einem Individuum.

**9.** Arzneimittel für die Behandlung oder Prävention von duktalem Adenokarzinom des Pankreas in einem Individuum, umfassend

(a) ein Antisense-Polynucleotid oder eine siRNA gegen PNC157 (REG IV); oder
(b) einen Antikörper oder ein Fragment davon, welcher an ein Protein bindet, welches von PNC157 (REG IV) codiert wird,
und einen pharmazeutisch verträglichen Träger.

## Revendications

**1.** Méthode pour diagnostiquer un adénocarcinome canalaire pancréatique chez un sujet, comprenant la détermination du niveau d'expression du gène associé au PNC, PNC157 (REG IV), dans un échantillon biologique dérivé du patient comprenant une cellule épithéliale canalaire pancréatique, dans laquelle une augmentation dudit niveau d'expression en comparaison du niveau contrôle normal indique que ledit sujet souffre d'un adénocarcinome canalaire pancréatique, et dans laquelle ledit niveau d'expression est déterminé en détectant l'ARNm de PNC157 ou en détectant la protéine codée par PNC157.

**2.** Méthode selon la revendication 1, dans laquelle ladite augmentation est au moins 10 % de plus que ledit niveau contrôle normal.

**3.** Méthode selon la revendication 1, dans laquelle ledit niveau d'expression est déterminé en détectant l'hybridation d'une sonde PNC157 (REG IV) à un transcrit du gène de l'échantillon biologique dérivé du patient.

**4.** Méthode selon la revendication 3, dans laquelle ladite étape d'hybridation est effectuée sur une puce à ADN.

**5.** Méthode in vitro pour cribler un composé se liant au polypeptide codé par PNC157 (REG IV) utile pour le traitement ou la prévention d'un adénocarcinome canalaire pancréatique, ladite méthode comprenant les étapes de :

(a) mise en contact d'un composé test avec un polypeptide codé par PNC157 ;
(b) détection de l'activité de liaison entre le polypeptide et le composé test ; et,
(c) sélection d'un composé qui se lie au polypeptide codé par PNC157.

**6.** Méthode in vitro pour cribler un composé diminuant le niveau d'expression de PNC157 (REG IV) utile pour le traitement ou la prévention d'un adénocarcinome canalaire pancréatique, ladite méthode comprenant les étapes de :

(a) mise en contact d'un composé candidat avec une cellule exprimant PNC157 ; et,
(b) sélection d'un composé qui diminue le niveau d'expression de PNC157.

**7.** Méthode selon la revendication 6, dans laquelle ladite cellule comprend une cellule d'adénocarcinome canalaire pancréatique.

**8.** Utilisation d'

(a) une composition d'antisens, ladite composition comprenant une séquence nucléotidique complémentaire d'une séquence codante de PNC157 (REG IV) ;
(b) une composition d'ARNsi, ladite composition diminuant l'expression de PNC157 (REG IV); ou
(c) un anticorps ou un fragment de celui-ci qui se lie à une protéine codée par PNC157
pour la préparation d'une composition pharmaceutique destinée au traitement ou à la prévention d'un adénocarcinome canalaire pancréatique chez un sujet.

**9.** Composition pharmaceutique destinée au traitement ou à la prévention d'un adénocarcinome canalaire pancréatique chez un sujet comprenant

(a) un polynucléotide antisens ou un petit ARN interférant dirigé contre PNC157 (REG IV) ; ou
(b) un anticorps ou un fragment de celui-ci qui se lie à une protéine codée par PNC157 (REG IV),
et un support pharmaceutiquement acceptable.

FIG.1

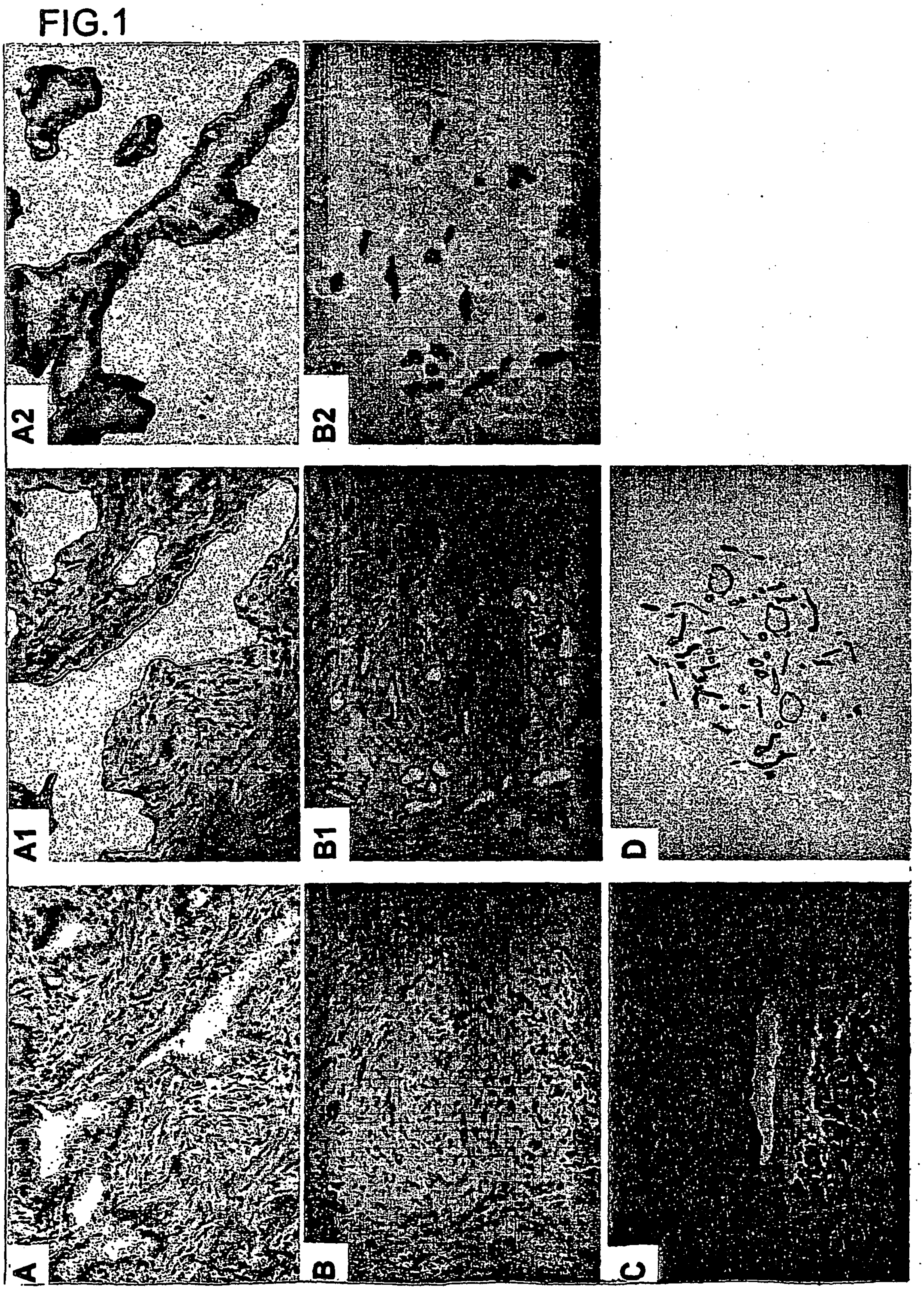

FIG.2-1

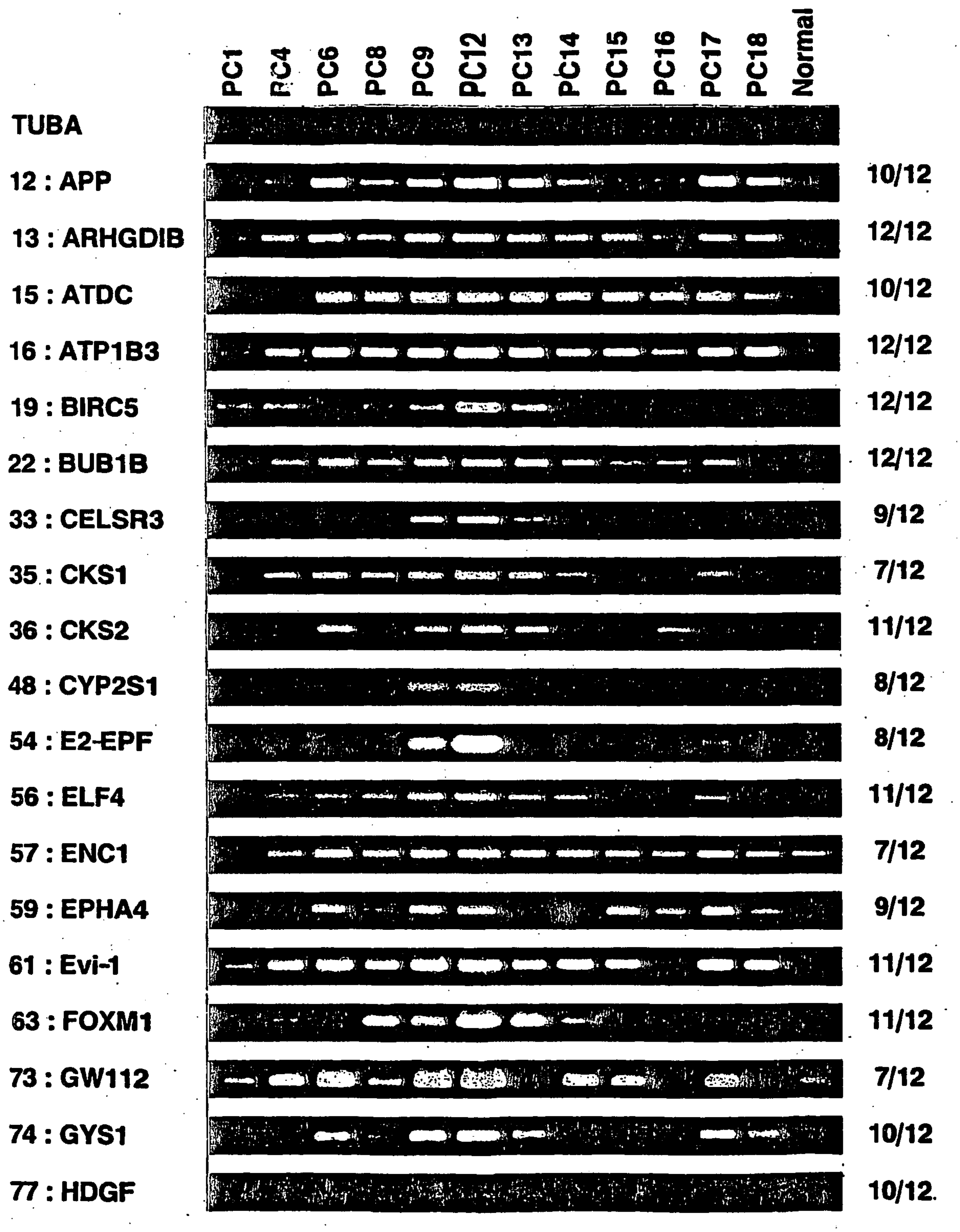
PC1
PC4
PC6
PC8
PC9
PC12
PC13
PC14
PC15
PC16
PC17
PC18
Normal
TUBA
12 : APP 10/12
13 : ARHGDIB 12/12
15 : ATDC 10/12
16 : ATP1B3 12/12
19 : BIRC5 12/12
22 : BUB1B 12/12
33 : CELSR3 9/12
35 : CKS1 7/12
36 : CKS2 11/12
48 : CYP2S1 8/12
54 : E2-EPF 8/12
56 : ELF4 11/12
57 : ENC1 7/12
59 : EPHA4 9/12
61 : Evi-1 11/12
63 : FOXM1 11/12
73 : GW112 7/12
74 : GYS1 10/12
77 : HDGF 10/12

FIG.2-2

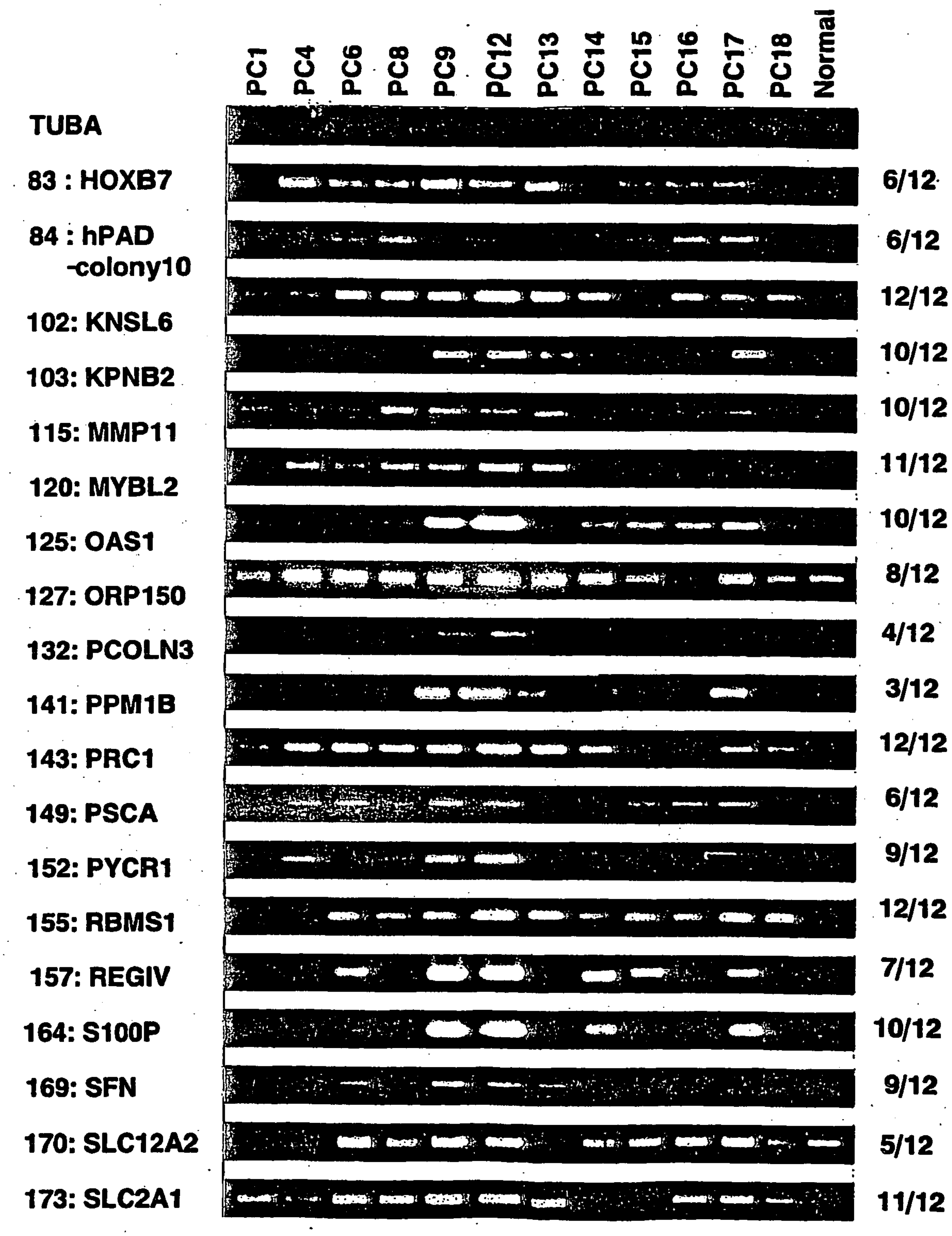
PC1
PC4
PC6
PC8
PC9
PC12
PC13
PC14
PC15
PC16
PC17
PC18
Normal
TUBA
83 : HOXB7 6/12
84 : hPAD -colony10 6/12
102: KNSL6 12/12
103: KPNB2 10/12
115: MMP11 10/12
120: MYBL2 11/12
125: OAS1 10/12
127: ORP150 8/12
132: PCOLN3 4/12
141: PPM1B 3/12
143: PRC1 12/12
149: PSCA 6/12
152: PYCR1 9/12
155: RBMS1 12/12
157: REGIV 7/12
164: S100P 10/12
169: SFN 9/12
170: SLC12A2 5/12
173: SLC2A1 11/12

FIG.2-3

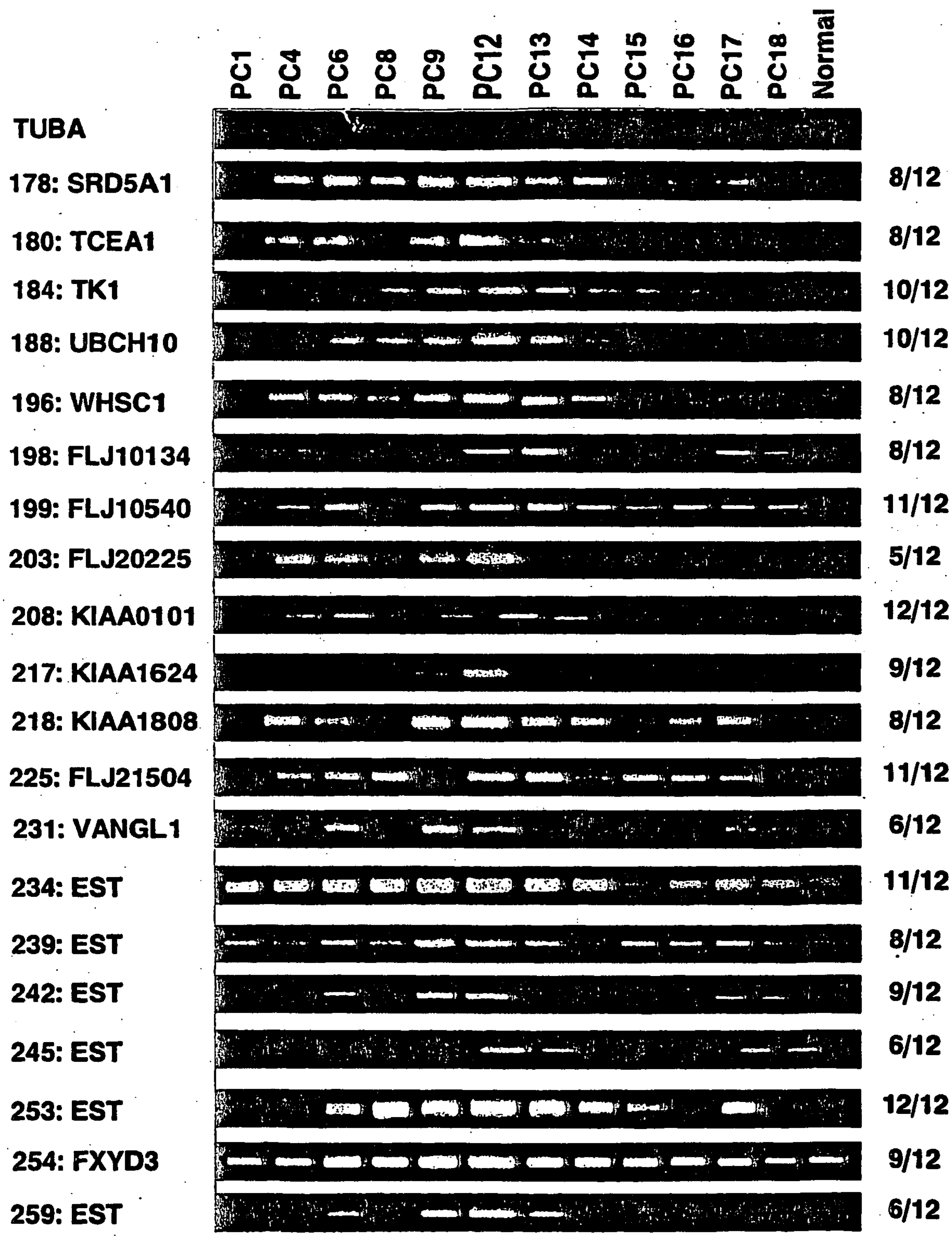
PC1
PC4
PC6
PC8
PC9
PC12
PC13
PC14
PC15
PC16
PC17
PC18
Normal
TUBA
178: SRD5A1
8/12
180: TCEA1
8/12
184: TK1
10/12
188: UBCH10
10/12
196: WHSC1
8/12
198: FLJ10134
8/12
199: FLJ10540
11/12
203: FLJ20225
5/12
208: KIAA0101
12/12
217: KIAA1624
9/12
218: KIAA1808
8/12
225: FLJ21504
11/12
231: VANGL1
6/12
234: EST
11/12
239: EST
8/12
242: EST
9/12
245: EST
6/12
253: EST
12/12
254: FXYD3
9/12
259: EST
6/12

FIG.3

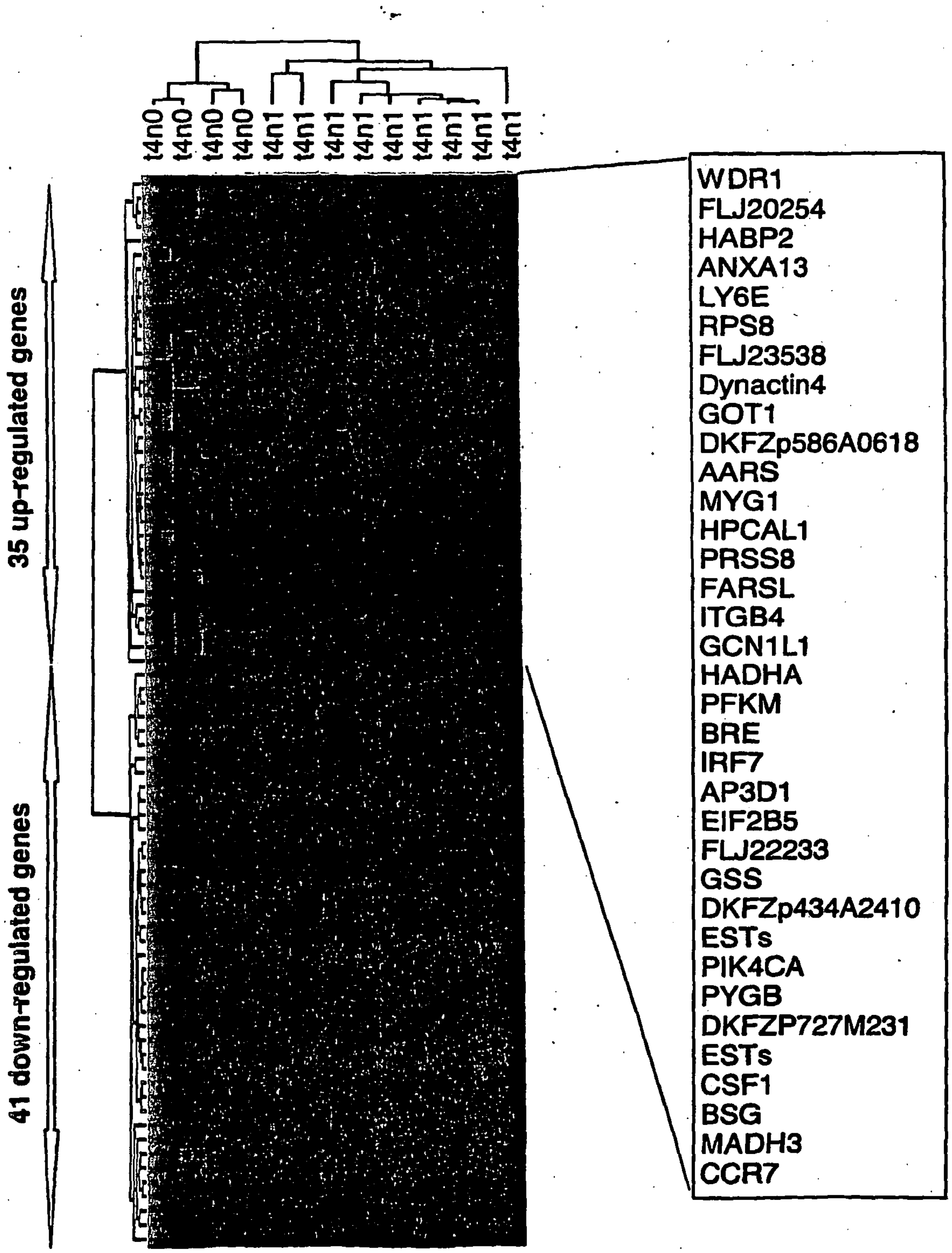
t4n0
t4n0
t4n0
t4n0
t4n1
t4n1
t4n1
t4n1
t4n1
t4n1
t4n1
t4n1
t4n1
35 up-regulated genes
41 down-regulated genes
WDR1
FLJ20254
HABP2
ANXA13
LY6E
RPS8
FLJ23538
Dynactin4
GOT1
DKFZp586A0618
AARS
MYG1
HPCAL1
PRSS8
FARSL
ITGB4
GCN1L1
HADHA
PFKM
BRE
IRF7
AP3D1
EIF2B5
FLJ22233
GSS
DKFZp434A2410
ESTs
PIK4CA
PYGB
DKFZP727M231
ESTs
CSF1
BSG
MADH3
CCR7

FIG.4

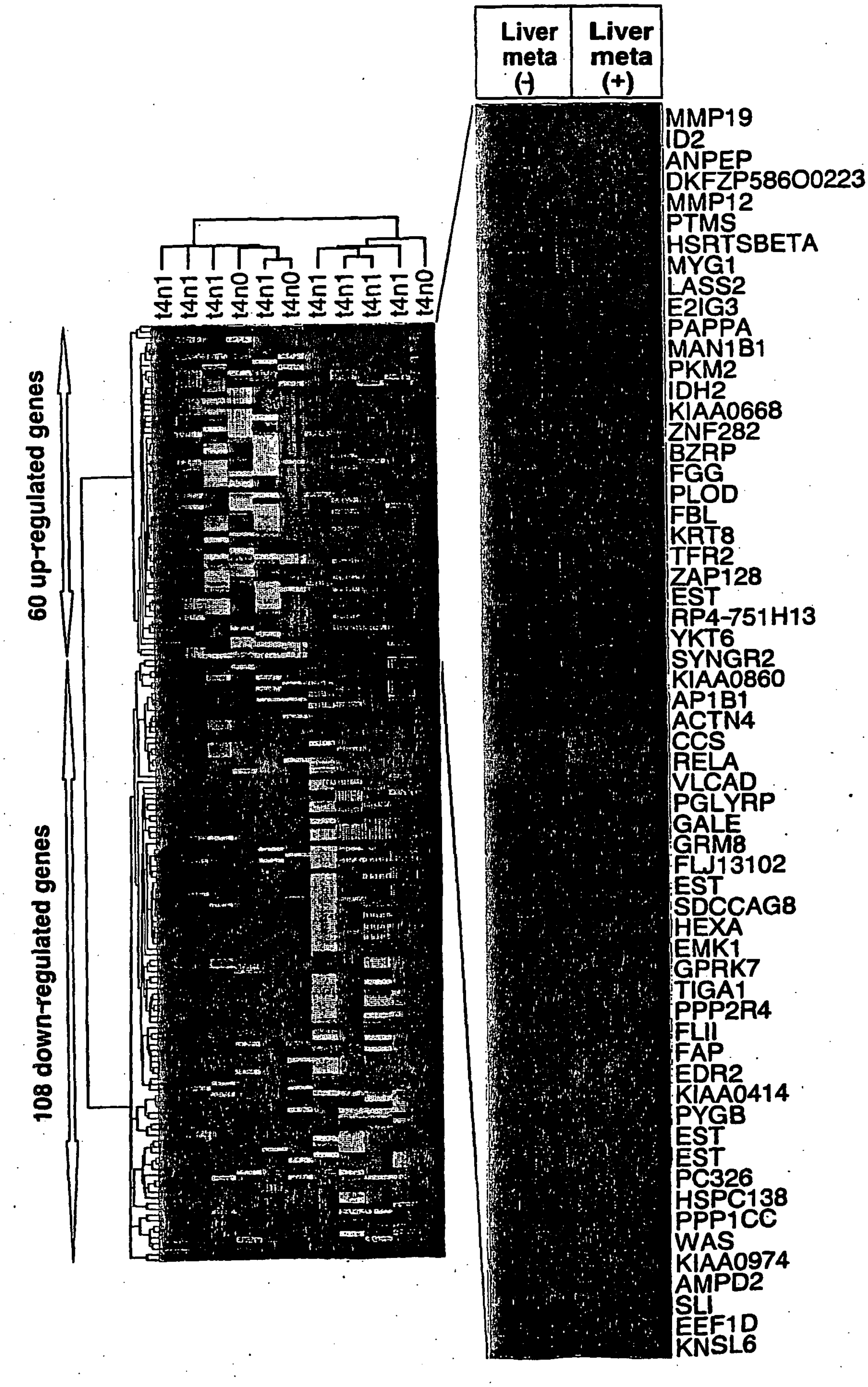
Liver meta (-)
Liver meta (+)
t4n1
t4n1
t4n1
t4n0
t4n1
t4n0
t4n1
t4n1
t4n1
t4n1
t4n0
60 up-regulated genes
108 down-regulated genes
MMP19
ID2
ANPEP
DKFZP586O0223
MMP12
PTMS
HSRTSBETA
MYG1
LASS2
E2IG3
PAPPA
MAN1B1
PKM2
IDH2
KIAA0668
ZNF282
BZRP
FGG
PLOD
FBL
KRT8
TFR2
ZAP128
EST
RP4-751H13
YKT6
SYNGR2
KIAA0860
AP1B1
ACTN4
CCS
RELA
VLCAD
PGLYRP
GALE
GRM8
FLJ13102
EST
SDCCAG8
HEXA
EMK1
GPRK7
TIGA1
PPP2R4
FLII
FAP
EDR2
KIAA0414
PYGB
EST
EST
PC326
HSPC138
PPP1CC
WAS
KIAA0974
AMPD2
SLI
EEF1D
KNSL6

FIG.5

(A-1)

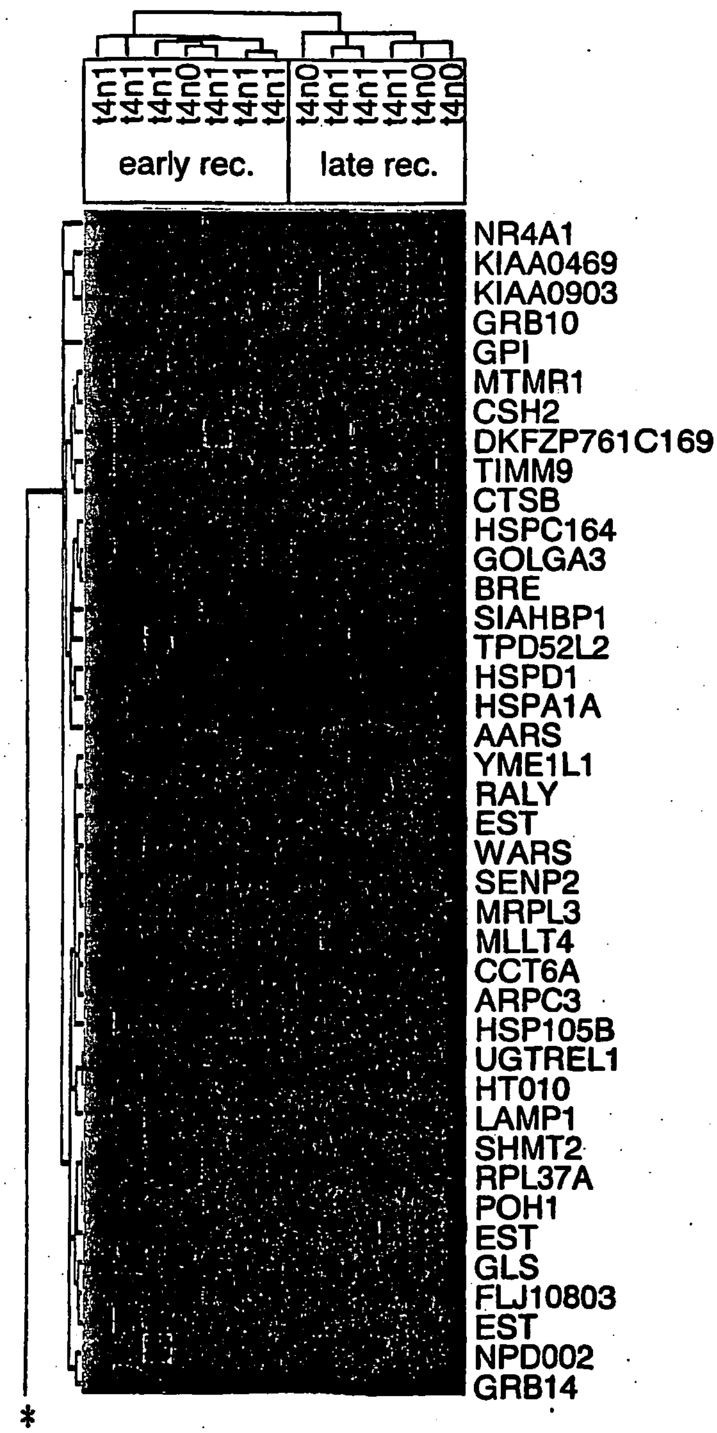
t4n1
t4n1
t4n1
t4n0
t4n1
t4n1
t4n1
t4n0
t4n1
t4n1
t4n1
t4n0
t4n0
early rec.
late rec.
NR4A1
KIAA0469
KIAA0903
GRB10
GPI
MTMR1
CSH2
DKFZP761C169
TIMM9
CTSB
HSPC164
GOLGA3
BRE
SIAHBP1
TPD52L2
HSPD1
HSPA1A
AARS
YME1L1
RALY
EST
WARS
SENP2
MRPL3
MLLT4
CCT6A
ARPC3
HSP105B
UGTREL1
HT010
LAMP1
SHMT2
RPL37A
POH1
EST
GLS
FLJ10803
EST
NPD002
GRB14
*

FIG.5

(A-2)

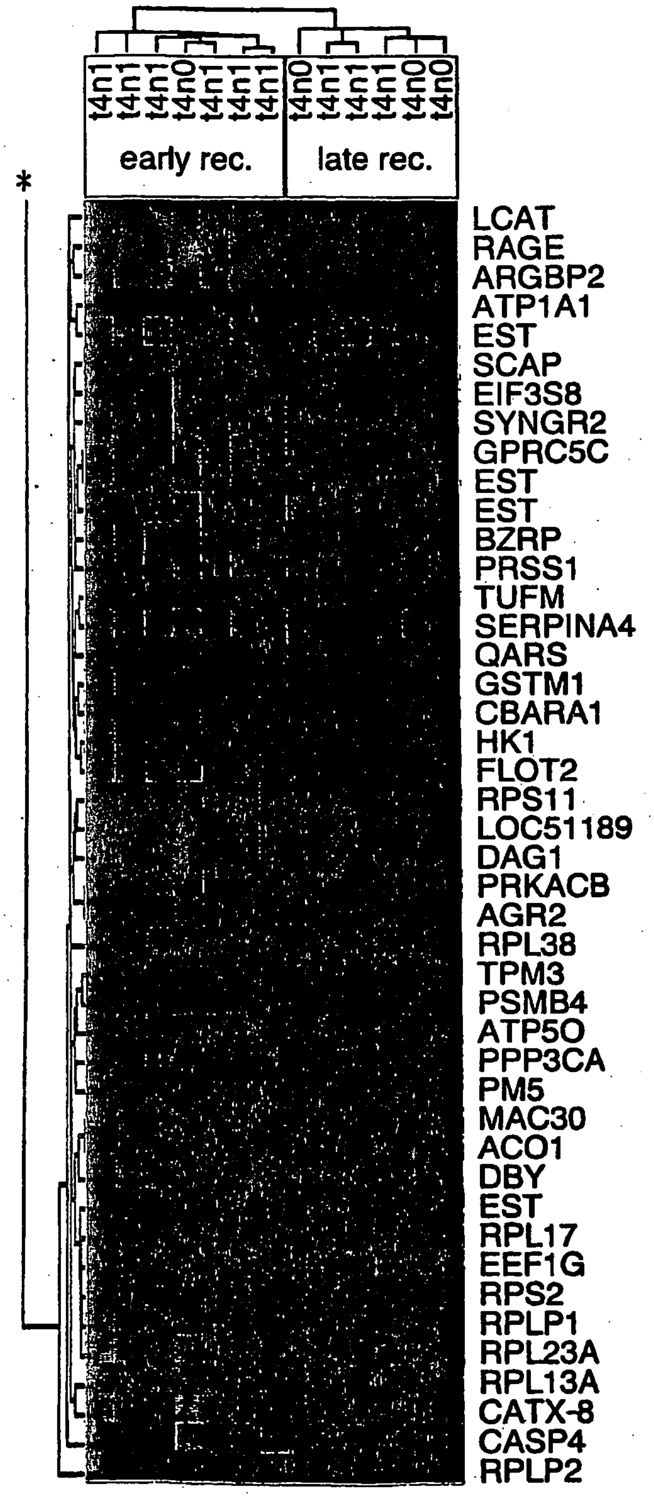
t4n1
t4n1
t4n1
t4n0
t4n1
t4n1
t4n1
t4n0
t4n1
t4n1
t4n1
t4n0
t4n0
early rec.
late rec.
*
LCAT
RAGE
ARGBP2
ATP1A1
EST
SCAP
EIF3S8
SYNGR2
GPRC5C
EST
EST
BZRP
PRSS1
TUFM
SERPINA4
QARS
GSTM1
CBARA1
HK1
FLOT2
RPS11
LOC51189
DAG1
PRKACB
AGR2
RPL38
TPM3
PSMB4
ATP5O
PPP3CA
PM5
MAC30
ACO1
DBY
EST
RPL17
EEF1G
RPS2
RPLP1
RPL23A
RPL13A
CATX-8
CASP4
RPLP2

FIG.5

(B)

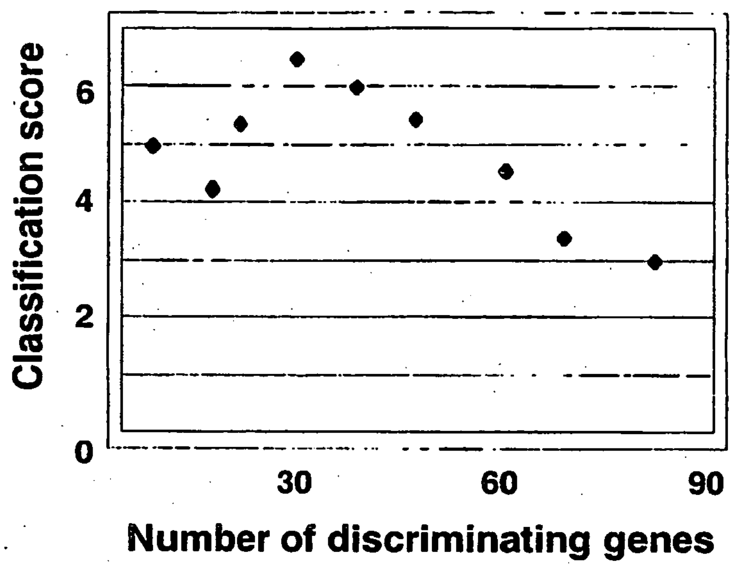
Classification score
0
2
4
6
30
60
90
Number of discriminating genes

(C)

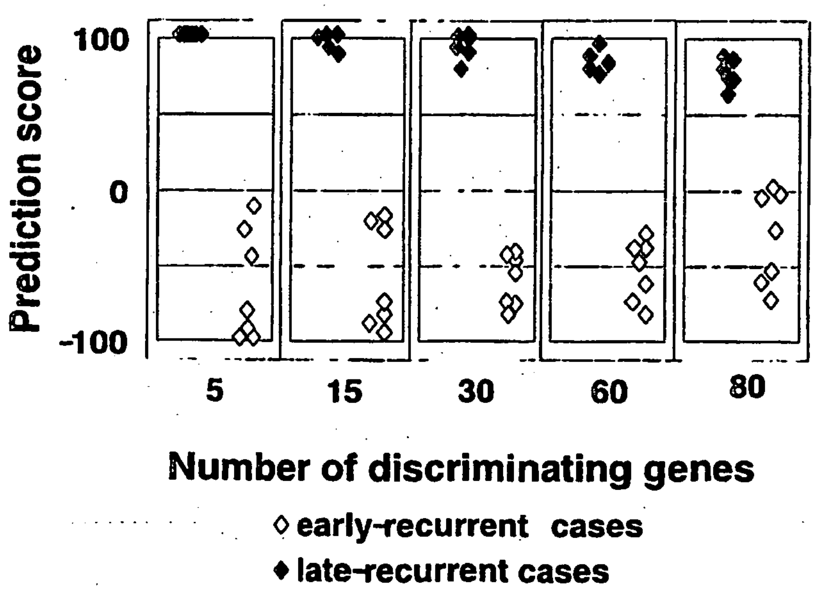
Prediction score
100
0
-100
5
15
30
60
80
Number of discriminating genes
◇ early-recurrent cases
◆ late-recurrent cases

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 60414872 B
- US 60450889 B
- EP 1241269 A
- WO 02060317 A
- US 5744305 A


### Non-patent literature cited in the description


- **OKABE et al.** *Cancer Res,* 2001, vol. 61, 2129-37
- **KITAHARA et al.** *Cancer Res,* 2001, vol. 61, 3544-9
- **LIN et al.** *Oncogene,* 2002, vol. 21, 4120-8
- **HASEGAWA et al.** *Cancer Res,* 2002, vol. 62, 7412-7
- **BIENZ ; CLEVERS.** *Cell,* 2000, vol. 103, 311-20
- **HASEGAWA et al.** *Cancer Res,* 2002, vol. 62, 7012-7
- **HE et al.** *Cell,* 1999, vol. 99, 335-45
- **LIN et al.** *Cancer Res,* 2001, vol. 61, 6345-9
- **FUJITA et al.** *Cancer Res,* 2001, vol. 61, 7722-6
- **BOON.** *Int J Cancer,* 1993, vol. 54, 177-80
- **BOON ; VAN DER BRUGGEN.** *J Exp Med,* 1996, vol. 183, 725-9
- **VAN DER BRUGGEN et al.** *Science,* 1991, vol. 254, 1643-7
- **BRICHARD et al.** *J Exp Med,* 1993, vol. 178, 489-95
- **KAWAKAMI et al.** *J Exp Med,* 1994, vol. 180, 347-52
- **SHICHIJO et al.** *J Exp Med,* 1998, vol. 187, 277-88
- **CHEN et al.** *Proc Natl Acad Sci USA,* 1997, vol. 94, 1914-8
- **UMANO et al.** *Brit J Cancer,* 2001, vol. 84, 1052-7
- **TANAKA et al.** *Brit J Cancer,* 2001, vol. 84, 94-9
- **NUKAYA et al.** *Int J Cancer,* 1999, vol. 80, 92-7
- **ROSENBEG et al.** *Nature Med,* 1998, vol. 4, 321-7
- **MUKHERJI et al.** *Proc Natl Acad Sci USA,* 1995, vol. 92, 8078-82
- **HU et al.** *Cancer Res,* 1996, vol. 56, 2479-83
- **BOON ; CAN DER BRUGGEN.** *J Exp Med,* 1996, vol. 183, 725-9
- **HARRIES.** *J Natl Cancer Inst,* 1996, vol. 88, 1442-5
- **BUTTERFIELD et al.** *Cancer Res,* 1999, vol. 59, 3134-42
- **VISSERS et al.** *Cancer Res,* 1999, vol. 59, 5554-9
- **VAN DER BURG et al.** *J Immunol,* 1996, vol. 156, 3308-14
- **TANAKA et al.** *Cancer Res,* 1997, vol. 57, 4465-8
- **FUJIE et al.** *Int J Cancer,* 1999, vol. 80, 169-72
- **KIKUCHI et al.** *Int J Cancer,* 1999, vol. 81, 459-66
- **OISO et al.** *Int J Cancer,* 1999, vol. 81, 387-94
- **KAWANO et al.** *Cance Res,* 2000, vol. 60, 3550-8
- **NISHIZAKA et al.** *Cancer Res,* 2000, vol. 60, 4830-7
- **TAMURA et al.** *Jpn J Cancer Res,* 2001, vol. 92, 762-7
- **DATE et al.** *Tissue Antigens,* 1996, vol. 47, 93-101
- **KONDO et al.** *J Immunol,* 1995, vol. 155, 4307-12
- **KUBO et al.** *J Immunol,* 1994, vol. 152, 3913-24
- **IMANISHI et al.** Proceeding of the eleventh International Hictocompatibility Workshop and Conference. Oxford University Press, 1992, 1065
- **WILLIAMS et al.** *Tissue Antigen,* 1997, vol. 49, 129
- **ALEXANDER-MILLER et al.** *Proc Natl Acad Sci USA,* 1996, vol. 93, 4102-7
- **T.R. GOLUB et al.** *Science,* 1999, vol. 286, 531-7
- **T.J. MACDONALD et al.** *Nat. Genet,* 2001, vol. 29, 143-52
- **HUSTON J. S. et al.** *Proc. Natl. Acad. Sci. U.S.A.,* 1988, vol. 85, 5879-5883
- **CO M. S. et al.** *J. Immunol.,* 1994, vol. 152, 2968-2976

- **BETTER M. ; HORWITZ A. H.** *Methods Enzymol.,* 1989, vol. 178, 476-496
- **PLUCKTHUN A. ; SKERRA A.** *Methods Enzymol.,* 1989, vol. 178, 497-515
- **LAMOYI E.** *Methods Enzymol.,* 1986, vol. 121, 652-663
- **ROUSSEAUX J. et al.** *Methods Enzymol.,* 1986, vol. 121, 663-669
- **BIRD R. E. ; WALKER B. W.** *Trends Biotechnol.,* 1991, vol. 9, 132-137
- **CIARDIELLO F ; TORTORA G.** A novel approach in the treatment of cancer: targeting the epidermal growth factor receptor. *Clin Cancer Res.,* October 2001, vol. 7 (10), 2958-70
- **SLAMON DJ ; LEYLAND-JONES B ; SHAK S ; FUCHS H ; PATON V ; BAJAMONDE A ; FLEMING T ; EIERMANN W ; WOLTER J ; PEGRAM M.** Use of chemotherapy plus a monoclonal antibody against HER2 for metastatic breast cancer that overexpresses HER2. *N Engl J Med.,* 15 March 2001, vol. 344 (11), 783-92
- **REHWALD U ; SCHULZ H ; REISER M ; SIEBER M ; STAAK JO ; MORSCHHAUSER F ; DRIESSEN C ; RUDIGER T ; MULLER-HERMELINK K ; DIEHL V.** Treatment of relapsed CD20+ Hodgkin lymphoma with the monoclonal antibody rituximab is effective and well tolerated: results of a phase 2 trial of the German Hodgkin Lymphoma Study Group. *Blood.,* 15 January 2003, vol. 101 (2), 420-424
- **FANG G ; KIM CN ; PERKINS CL ; RAMADEVI N ; WINTON E ; WITTMANN S ; BHALLA KN.** *Blood,* 2000, vol. 96, 2246-2253
- **GIANNI L.** *Oncology,* 2002, vol. 63 (1), 47-56
- **KLEJMAN A ; RUSHEN L ; MORRIONE A ; SLUPIANEK A ; SKORSKI T.** *Oncogene,* 2002, vol. 21, 5868-5876
- **CAPECCHI.** *Science,* 1989, vol. 244, 1288-1292
- **ISHIGURO, H. ; SHIMOKAWA, T. ; TSUNODA, T. ; TANAKA, T. ; FUJII, Y. ; NAKAMURA, Y. ; FURUKAWA, Y.** Isolation of HELAD1, a novel human helicase gene up-regulated in colorectal carcinomas. *Oncogene,* 2002, vol. 21, 6387-6394
- **YAGYU, R. ; HAMAMOTO, R. ; FURUKAWA, Y. ; OKABE, H. ; YAMAMURA, T. ; NAKAMURA, Y.** Isolation and characterization of a novel human gene, VANGL1, as a therapeutic target for hepatocellular carcinoma. *Int J Oncol,* 2002, vol. 20, 1173-1178
- **IACOBUZIO-DONAHUE, C. A. ; MAITRA, A. ; SHEN-ONG, G. L. ; VAN HEEK, T. ; ASHFAQ, R. ; MEYER, R. ; WALTER, K. ; BERG, K. ; HOLLINGSWORTH, M. A. ; CAMERON, J. L.** Discovery of novel tumor markers of PNC using global gene expression technology. *Am J Pathol,* vol. 160, 1239-1249
- **HAN, H. ; BEARSS, D. J. ; BROWNE, L. W. ; CALALUCE, R. ; NAGLE, R. B. ; VON HOFF, D. D.** Identification of differentially expressed genes in PNC cells using cDNA microarray. *Cancer Res,* 2002, vol. 62, 2890-2896
- **BOCKMAN, D. E. ; BOYDSTON, W. R. ; PARSA, I.** Architecture of human pancreas: implications for early changes in pancreatic disease. *Gastroenterology,* 1983, vol. 85, 55-61
- **HRUBAN, R. H. ; WILENTZ, R. E. ; KERN, S. E.** Genetic progression in the pancreatic ducts. *Am J Pathol,* 2000, vol. 156, 1821-1825
- **KITAHARA, O. ; FURUKAWA, Y. ; TANAKA, T. ; KIHARA, C. ; ONO, K. ; YANAGAWA, R. ; NITA, M. E. ; TAKAGI, T. ; NAKAMURA, Y. ; TSUNODA, T.** Alterations of gene expression during colorectal carcinogenesis revealed by cDNA microarrays after laser-capture microdissection of tumor tissues and normal epithelia. *Cancer Res,* 2001, vol. 61, 3544-3549
- **GJERDRUM, L. M. ; LIELPETERE, I. ; RASMUSSEN, L. M. ; BENDIX, K. ; HAMILTON-DUTOIT, S.** Laser-assisted microdissection of membrane-mounted paraffin sections for polymerase chain reaction analysis: identification of cell populations using immunohistochemistry and in situ hybridization. *J Mol Diagn,* 2001, vol. 3, 105-110
- **ONO, K. ; TANAKA, T. ; TSUNODA, T. ; KITAHARA, O. ; KIHARA, C. ; OKAMOTO, A. ; OCHIAI, K. ; TAKAGI, T. ; NAKAMURA, Y.** Identification by cDNA microarray of genes involved in ovarian carcinogenesis. *Cancer Res,* 2000, vol. 60, 5007-5011
- **SAITO-HISAMINATO, A. ; KATAGIRI, T. ; KAKIUCHI, S. ; NAKAMURA, T. ; TSUNODA, T. ; NAKAMURA, Y.** Genome-wide profiling of gene expression in 29 normal human tissues with a cDNA microarray. *DNA Res,* 2002, vol. 9, 35-45
- **DIMAGNO EP ; REBER HA ; TEMPERO MA.** AGA technical review on the epidemiology, diagnosis, and treatment of pancreatic ductal adenocarcinoma. *American Gastroenterological Association. Gastroenterology,* December 1999, vol. 117 (6), 1464-84
- **BRENTNALL TA ; BRONNER MP ; BYRD DR ; HAGGITT RC ; KIMMEY MB.** Early diagnosis and treatment of pancreatic dysplasia in patients with a family history of pancreatic cancer. *Ann Intern Med.,* 17 August 1999, vol. 131 (4), 247-55
- **ROSENBERG L.** Pancreatic cancer: a review of emerging therapies. *Drugs,* May 2000, vol. 59 (5), 1071-89
- **HAO D ; ROWINSKY EK.** Inhibiting signal transduction: recent advances in the development of receptor tyrosine kinase and Ras inhibitors. *Cancer Invest.,* 2002, vol. 20 (3), 387-404

- **LAHERU D ; BIEDRZYCKI B ; JAFFEE EM.** Immunologic approaches to the management of pancreatic cancer. *Cancer J.,* July 2001, vol. 7 (4), 324-37
- **CRNOGORAC-JURCEVIC T ; EFTHIMIOU E ; NIELSEN T ; LOADER J ; TERRIS B ; STAMP G ; BARON A ; SCARPA A ; LEMOINE NR.** Expression profiling of microdissected pancreatic adenocarcinomas. *Oncogene,* 04 July 2002, vol. 21 (29), 4587-94
- **VIOLETTE S ; FESTOR E ; PANDREA-VASILE I ; MITCHELL V ; ADIDA C ; DUSSAULX E ; LAÇORTE JM ; CHAMBAZ J ; LACASA M ; LESUFFLEUR T.** Reg IV, a new member of the regenerating gene family, is overexpressed in colorectal carcinomas. *Int J Cancer.,* 10 January 2003, vol. 103 (2), 185-93
- **KULLANDER K ; MATHER NK ; DIELLA F ; DOTTORI M ; BOYD AW ; KLEIN R.** Kinase-dependent and kinase-independent functions of EphA4 receptors in major axon tract formation in vivo. *Neuron,* January 2001, vol. 29 (1), 73-84
- **ROZENBLUM E ; SCHUTTE M ; GOGGINS M ; HAHN SA ; PANZER S ; ZAHURAK M ; GOODMAN SN ; SOHN TA ; HRUBAN RH ; YEO CJ.** Tumor-suppressive pathways in pancreatic carcinoma. *Cancer Res.,* 01 May 1997, vol. 57 (9), 1731-4
- **GOGGINS M ; HRUBAN RH ; KERN SE.** BRCA2 is inactivated late in the development of pancreatic intraepithelial neoplasia: evidence and implications. *Am J Pathol.,* May 2000, vol. 156 (5), 1767-71
- **ISHIGURO H ; TSUNODA T ; TANAKA, T ; FUJII Y ; NAKAMURA Y ; FURUKAWA Y.** Identification of AXUD1, a novel human gene induced by AXIN1 and its reduced expression in human carcinomas of the lung, liver, colon and kidney. *Oncogene,* 16 August 2001, vol. 20 (36), 5062-6
- **SATOH S ; DAIGO Y ; FURUKAWA Y ; KATO T ; MIWA N ; NISHIWAKI T ; KAWASOE T ; ISHIGURO H ; FUJITA M ; TOKINO T.** AXIN1 mutations in hepatocellular carcinomas, and growth suppression in cancer cells by virus-mediated transfer of AXIN1. *Nat Genet.,* March 2000, vol. 24 (3), 245-50
- **YUAN BZ ; MILLER MJ ; KECK CL ; ZIMONJIC DB ; THORGEIRSSON SS ; POPESCU NC.** Cloning, characterization, and chromosomal localization of a gene frequently deleted in human liver cancer (DLC-1) homologous to rat RhoGAP. *Cancer Res.,* 15 May 1998, vol. 58 (10), 2196-9
- **NG IO ; LIANG ZD ; CAO L ; LEE TK.** DLC-1 is deleted in primary hepatocellular carcinoma and exerts inhibitory effects on the proliferation of hepatoma cell lines with deleted DLC-1. *Cancer Res.,* 01 December 2000, vol. 60 (23), 6581-4

SEQUENCE LISTING

<110> ONCOTHERAPY SCIENCE, INC.
JAPAN AS REPRESENTED BY THE PRESIDENT OF THE UNIVERSITY OF TOKYO

<120> METHOD FOR DIAGNOSING PANCREATIC CANCER

<130> ONC-A0217Y1P1

<150> US 60/414,872
<151> 2002-09-30

<150> US 60/450,889
<151> 2003-02-28

<160> 118

<170> PatentIn version 3.1

<210> 1
<211> 22
<212> DNA
<213> Artificial

<220>

<223> Artificially synthesized primer sequence for RT-PCR

<400> 1
ctgctggtct tcaattacca ag 22

<210> 2
<211> 23
<212> DNA
<213> Artificial

<220>

<223> Artificially synthesized primer sequence for RT-PCR

<400> 2
ctcatcccct tatattgcca ctt 23

<210> 3
<2U> 21
<212> DNA
<213> Artificial

<220>

<223> Artificially synthesized primer sequence for RT-PCR

<400> 3
ctccctctga tcctccatca g 21

<210> 4
<211> 25
<212> DNA
<213> Artificial

<220>

<223> Artificially synthesized primer sequence for RT-PCR

<400> 4

tcttgttctc ttgtgtcgtt tacag 25

<210> 5
<211> 22
<212> DNA
<213> Artificial

<220>

<223> Artificially synthesized primer sequence for RT-PCR

<400> 5
cattctctct ggcgatggag tg 22

<210> 6
<211> 22
<212> DNA
<213> Artificial

<220>

<223> Artificially synthesized primer sequence for RT-PCR

<400> 6
accaatggtt tattccaaag gg 22

<210> 7
<211> 21
<212> DNA
<213> Artificial

<220>

<223> Artificially synthesized primer sequence for RT-PCR

<400> 7
cagtgtacag tcgccagata g 21

<210> 8
<211> 24
<212> DNA
<213> Artificial

<220>

<223> Artificially synthesized primer sequence for RT-PCR

<400> 8
tcctcacata cagaacttct ccac 24

<210> 9
<211> 21
<212> DNA
<213> Artificial

<220>

<223> Artificially synthesized primer sequence for RT-PCR

<400> 9
ctccctcaga aaaaggcagt g 21

<210> 10
<211> 22
<212> DNA
<213> Artificial

<220>

<223> Artificially synthesized primer sequence for RT-PCR

<400> 10
gaagctgtaa caatccaccc tg 22

<210> 11
<211> 21
<212> DNA
<213> Artificial

<220>

<223> Artificially synthesized primer sequence for RT-PCR

<400> 11
agctaggcaa tcaagtctca c 21

<210> 12
<211> 23
<212> DNA
<213> Artificial

<220>

<223> Artificially synthesized primer sequence for RT-PCR

<400> 12
agggaaaagt agagacaaat ggg 23

<210> 13
<211> 20
<212> DNA
<213> Artificial

<220>

<223> Artificially synthesized primer sequence for RT-PCR

<400> 13
aagcagcttc ctgggagatt 20

<210> 14
<211> 23
<212> DNA
<213> Artificial

<220>

<223> Artificially synthesized primer sequence for RT-PCR

<400> 14
acggaacaat ttacacagac agg 23

<210> 15
<211> 23
<212> DNA
<213> Artificial

<220>

<223> Artificially synthesized primer sequence for RT-PCR

<400> 15
actattcgga caaatacgac gac 23

<210> 16
<211> 23
<212> DNA
<213> Artificial

<220>

<223> Artificially synthesized primer sequence for RT-PCR

<400> 16
cactgtttga atgtgctggt aac 23

<210> 17
<211> 24
<212> DNA
<213> Artificial

<220>

<223> Artificially synthesized primer sequence for RT-PCR

<400> 17
caagcagatc tactactcgg acaa 24

<210> 18
<211> 24
<212> DNA
<213> Artificial

<220>

<223> Artificially synthesized primer sequence for RT-PCR

<400> 18
cagtaaccta cttgcagttg catt 24

<210> 19
<211> 21
<212> DNA
<213> Artificial

<220>

<223> Artificially synthesized primer sequence for RT-PCR

<400> 19
caccctgatt ctaccaaatg c 21

<210> 20
<211> 23
<212> DNA
<213> Artificial

<220>

<223> Artificially synthesized primer sequence for RT-PCR

<400> 20
ccttaagtca caaggaacgt cag 23

<210> 21
<211> 20
<212> DNA
<213> Artificial

<220>

<223> Artificially synthesized primer sequence for RT-PCR

<400> 21
tctgctcaca gagatccacg 20

<210> 22
<211> 23
<212> DNA
<213> Artificial

<220>

<223> Artificially synthesized primer sequence for RT-PCR

<400> 22
ttagagacag agttggaggg agg 23

<210> 23
<211> 21
<212> DNA
<213> Artificial

<220>

<223> Artificially synthesized primer sequence for RT-PCR

<400> 23
agaaatgtca gccacggaaa c 21

<210> 24
<211> 22
<212> DNA

<213> Artificial

<220>

<223> Artificially synthesized primer sequence for RT-PCR

<400> 24
aaaggcactt taatgccaac tg 22

<210> 25
<211> 22
<212> DNA
<213> Artificial

<220>

<223> Artificially synthesized primer sequence for RT-PCR

<400> 25
cgatataggc atttggtctc ac 22

<210> 26
<211> 25
<212> DNA
<213> Artificial

<220>

<223> Artificially synthesized primer sequence for RT-PCR

<400> 26
tttctcttca ttagacttgg cctct 25

<210> 27
<211> 23
<212> DNA
<213> Artificial

<220>

<223> Artificially synthesized primer sequence for RT-PCR

<400> 27
gaaggcgtgg tcactaaatg taa 23

<210> 28
<211> 23
<212> DNA
<213> Artificial

<220>

<223> Artificially synthesized primer sequence for RT-PCR

<400> 28
ctttaatttc agagggcgaa gac 23

<210> 29

<211> 22
<212> DNA
<213> Artificial

<220>

<223> Artificially synthesized primer sequence for RT-PCR

<400> 29
gcaagcttgt gcgatgttat gt 22

<210> 30
<211> 23
<212> DNA
<213> Artificial

<220>

<223> Artificially synthesized primer sequence for RT-PCR .

<400> 30
ctcctcccat agtaatgcac tga 23

<210> 31
<211> 22
<212> DNA
<213> Artificial

<220>

<223> Artificially synthesized primer sequence for RT-PCR

<400> 31
gatggatgca actgaagcag ag 22

<210> 32
<211> 23
<212> DNA
<213> Artificial

<220>

<223> Artificially synthesized primer sequence for RT-PCR

<400> 32
gtccaccttc gcttttattg agt 23

<210> 33
<211> 22
<212> DNA
<213> Artificial

<220>

<223> Artificially synthesized primer sequence for RT-PCR

<400> 33
gaaaatctga tggcagtgac aa 22

<210> 34
<211> 23
<212> DNA
<213> Artificial

<220>

<223> Artificially synthesized primer sequence for RT-PCR

<400> 34
aaggtttcca actactgcac tga 23

<210> 35
<211> 21
<212> DNA
<213> Artificial

<220>

<223> Artificially synthesized primer sequence for RT-PCR

<400> 35
tgcccactgt gaaaccacta g 21

<210> 36
<211> 21
<212> DNA
<213> Artificial

<220>

<223> Artificially synthesized primer sequence for RT-PCR

<400> 36
catctcatct ccggacacac t 21

<210> 37
<211> 20
<212> DNA
<213> Artificial

<220>

<223> Artificially synthesized primer sequence for RT-PCR

<400> 37
tatcccagct gcctagattc 20

<210> 38
<211> 23
<212> DNA
<213> Artificial

<220>

<223> Artificially synthesized primer sequence for RT-PCR

<400> 38

gagtcttccc aagcatccta ttt 23

<210> 39
<211> 21
<212> DNA
<213> Artificial

<220>

<223> Artificially synthesized primer sequence for RT-PCR

<400> 39
gtacctatag gaaagtctgt c 21

<210> 40
<211> 21
<212> DNA
<213> Artificial

<220>

<223> Artificially synthesized primer sequence for RT-PCR

<400> 40
aacacgcgag tggtaggttt t 21

<210> 41
<211> 23
<212> DNA
<213> Artificial

<220>

<223> Artificially synthesized primer sequence for RT-PCR

<400> 41
cactgagcca actactgtca ctg 23

<210> 42
<211> 23
<212> DNA
<213> Artificial

<220>

<223> Artificially synthesized primer sequence for RT-PCR

<400> 42
cttcctaccc acagctcttt ctc 23

<210> 43
<211> 23
<212> DNA
<213> Artificial

<220>

<223> Artificially synthesized primer sequence for RT-PCR

<400> 43
actctaggac ttgcatgatt gcc 23

<210> 44
<211> 23
<212> DNA
<213> Artificial

<220>

<223> Artificially synthesized primer sequence for RT-PCR

<400> 44
tcctctagga ctctagggag aca 23

<210> 45
<211> 22
<212> DNA
<213> Artificial

<220>

<223> Artificially synthesized primer sequence for RT-PCR

<400> 45
tcttggagac tataagggag cc 22

<210> 46
<211> 22
<212> DNA
<213> Artificial

<220>

<223> Artificially synthesized primer sequence for RT-PCR

<400> 46
ttttgcttct tcacatccac tg 22

<210> 47
<211> 20
<212> DNA
<213> Artificial

<220>

<223> Artificially synthesized primer sequence for RT-PCR

<400> 47
gcactgaagc aagggtgctg 20

<210> 48
<211> 23
<212> DNA
<213> Artificial

<220>

<223> Artificially synthesized primer sequence for RT-PCR

<400> 48
gacaggattg aggtatgttg cag 23

<210> 49
<211> 21
<212> DNA
<213> Artificial

<220>

<223> Artificially synthesized primer sequence for RT-PCR

<400> 49
tcctgaggtg ttgagggtgt c 21

<210> 50
<211> 22
<212> DNA
<213> Artificial

<220>

<223> Artificially synthesized primer sequence for RT-PCR

<400> 50
atcctaagca gggtctgaga tg 22

<210> 51
<211> 22
<212> DNA
<213> Artificial

<220>

<223> Artificially synthesized primer sequence for RT-PCR

<400> 51
tttcaggatc agttaaatcg cc 22

<210> 52
<211> 21
<212> DNA
<213> Artificial

<220>

<223> Artificially synthesized primer sequence for RT-PCR

<400> 52
ggcctggctg aattacccat g 21

<210> 53
<211> 20
<212> DNA
<213> Artificial

<220>

<223> Artificially synthesized primer sequence for RT-PCR

<400> 53
gttctgctcc tcccagacag 20

<210> 54
<211> 20
<212> DNA
<213> Artificial

<220>

<223> Artificially synthesized primer sequence for RT-PCR

<400> 54
gccctagctc ctgctacaga 20

<210> 55
<211> 20
<212> DNA
<213> Artificial

<220>

<223> Artificially synthesized primer sequence for RT-PCR

<400> 55
gctcactgcg tttggttttc 20

<210> 56
<211> 23
<212> DNA
<213> Artificial

<220>

<223> Artificially synthesized primer sequence for RT-PCR

<400> 56
cagcattcta ggagaaaggt gaa 23

<210> 57
<211> 23
<212> DNA
<213> Artificial

<220>

<223> Artificially synthesized primer sequence for RT-PCR

<400> 57
ctgtaacgtt ttcctgaagc tgt 23

<210> 58
<211> 23
<212> DNA

<213> Artificial

<220>

<223> Artificially synthesized primer sequence for RT-PCR

<400> 58
tcagtacagg gttggatcag agt 23

<210> 59
<211> 21
<212> DNA
<213> Artificial

<220>

<223> Artificially synthesized primer sequence for RT-PCR

<400> 59
gtgcctactt tgcctgagtt c 21

<210> 60
<211> 25
<212> DNA
<213> Artificial

<220>

<223> Artificially synthesized primer sequence for RT-PCR

<400> 60
caggacacgt actgtatgag gtaaa 25

<210> 61
<211> 20
<212> DNA
<213> Artificial

<220>

<223> Artificially synthesized primer sequence for RT-PCR

<400> 61
gaccatgtat gtttgcaccc 20

<210> 62
<211> 23
<212> DNA
<213> Artificial

<220>

<223> Artificially synthesized primer sequence for RT-PCR

<400> 62
aactcacgtc aactcttgtc ctc 23

<210> 63

<211> 20
<212> DNA
<213> Artificial

<220>

<223> Artificially synthesized primer sequence for RT-PCR,

<400> 63
atcccaagtc cagcgtgaag 20

<210> 64
<211> 23
<212> DNA
<213> Artificial

<220>

<223> Artificially synthesized primer sequence for RT-PCR

<400> 64
tccactattc cacccacagt aac 23

<210> 65
<211> 21
<212> DNA
<213> Artificial

<220>

<223> Artificially synthesized primer sequence for RT-PCR

<400> 65
ctgtcgagac gtctaatgac c 21

<210> 66
<211> 25
<212> DNA
<213> Artificial

<220>

<223>. Artificially synthesized primer sequence for RT-PCR

<400> 66
ttactaaaat aaacctgttc ggggg 25

<210> 67
<211> 20
<212> DNA
<213> Artificial

<220>

<223> Artificially synthesized primer sequence for RT-PCR

<400> 67
ccagtagtgg cttctagctc 20

<210> 68
<211> 23
<212> DNA
<213> Artificial

<220>

<223> Artificially synthesized primer sequence for RT-PCR

<400> 68
gaaaaacaag caggagttga gtg 23

<210> 69
<211> 23
<212> DNA
<213> Artificial

<220>

<223> Artificially synthesized primer sequence for RT-PCR

<400> 69
gcatgatcat agacgtcttt tcc 23

<210> 70
<211> 23
<212> DNA
<213> Artificial

<220>

<223> Artificially synthesized primer sequence for RT-PCR

<400> 70
gatgaactca ctgaagtcca cct 23

<210> 71

<211> 21
<212> DNA
<213> Artificial

<220>

<223> Artificially synthesized primer sequence for RT-PCR

<400> 71
gagcgcacct aaccactggt c 21

<210> 72
<211> 23
<212> DNA
<213> Artificial

<220>

<223> Artificially synthesized primer sequence for RT-PCR

<400> 72
tgagtgtcac aggggaactt tat 23

<210> 73
<211> 20
<212> DNA
<213> Artificial

<220>

<223> Artificially synthesized primer sequence for RT-PCR

<400> 73
aaccgaagtc tccatacacg 20

<210> 74
<211> 20
<212> DNA
<213> Artificial

<220>

<223> Artificially synthesized primer sequence for RT-PCR

<400> 74
gttcgtggga atcatcagag 20

<210> 75
<211> 20
<212> DNA
<213> Artificial

<220>

<223> Artificially synthesized primer sequence for RT-PCR

<400> 75
aaccgaagtc tccatacacg 20

<210> 76
<211> 20
<212> DNA
<213> Artificial

<220>

(223) Artificially synthesized primer sequence for RT-PCR

<400> 76
gttcgtggga atcatcagag 20

<210> 77
<211> 20
<212> DNA
<213> Artificial

<220>

<223> Artificially synthesized primer sequence for RT-PCR

<400> 77
tctgtaacaa taacaagacc 20

<210> 78
<211> 23
<212> DNA
<213> Artificial

<220>

<223> Artificially synthesized primer sequence for RT-PCR

<400> 78
ccagatgaga tgataaggca aag 23

<210> 79
<211> 22
<212> DNA
<213> Artificial

<220>

<223> Artificially synthesized primer sequence for RT-PCR

<400> 79
tgtcccaagt cttatttgct ga 22

<210> 80
<211> 23
<212> DNA
<213> Artificial

<220>

<223> Artificially synthesized primer sequence for RT-PCR

<400> 80
gcaacagtgg cctttaaagt atg 23

<210> 81
<211> 21
<212> DNA
<213> Artificial

<220>

<223> Artificially synthesized primer sequence for RT-PCR

<400> 81
gtaattgtgg ctgcactgga t 21

<210> 82
<211> 23
<212> DNA
<213> Artificial

<220>

<223> Artificially synthesized primer sequence for RT-PCR

<400> 82
atttcataag ctacagcaga ggc 23

<210> 83
<211> 20
<212> DNA
<213> Artificial

<220>

<223> Artificially synthesized primer sequence for RT-PCR

<400> 83
acacacatgc tgccgagctc 20

<210> 84
<211> 23
<212> DNA
<213> Artificial

<220>

<223> Artificially synthesized primer sequence for RT-PCR

<400> 84
taatatacaa gggctcaacc gag 23

<210> 85
<211> 21
<212> DNA
<213> Artificial

<220>

<223> Artificially synthesized primer sequence for RT-PCR

<400> 85
cctatgagtg tagttgatga c 21

<210> 86
<211> 23
<212> DNA
<213> Artificial

<220>

<223> Artificially synthesized primer sequence for RT-PCR

<400> 86
caactggcaa gtctcaactc tct 23

<210> 87
<211> 23
<212> DNA

<213> Artificial

<220>

<223> Artificially synthesized primer sequence for RT-PCR

<400> 87
tccagatgga tttgtcctgt atc 23

<210> 88
<211> 23
<212> DNA
<213> Artificial

<220>

<223> Artificially synthesized primer sequence for RT-PCR

<400> 88
tagtagcaag cccagtaacc ttg 23

<210> 89
<211> 23
<212> DNA
<213> Artificial

<220>

<223> Artificially synthesized primer sequence for RT-PCR

<400> 89
gcttaccatt gaaacttaac ccc 23

<210> 90
<211> 23
<212> DNA
<213> Artificial

<220>

<223> Artificially synthesized primer sequence for RT-PCR

<400> 90
ctcatttaca gtagcccagt ggt 23

<210> 91
<211> 23
<212> DNA
<213> Artificial

<220>

<223> Artificially synthesized primer sequence for RT-PCR

<400> 91
gacttccaca atgaacaggg taa 23

<210> 92

<211> 23
<212> DNA
<213> Artificial

<220>

<223> Artificially synthesized primer sequence for RT-PCR

<400> 92
attggaataa gaggaacagg age 23

<210> 93
<211> 23
<212> DNA
<213> Artificial

<220>

<223> Artificially synthesized primer sequence for RT-PCR

<400> 93
ccaattagct ttgttgaaca ggc 23

<210> 94
<211> 23
<212> DNA
<213> Artificial

<220>

<223> Artificially synthesized primer sequence for RT-PCR

<400> 94
ggcagcagta caacaatcta agc 23

<210> 95
<211> 21
<212> DNA
<213> Artificial

<220>

<223> Artificially synthesized primer sequence for RT-PCR

<400> 95
cagtgctaca cccacttctt g 21

<210> 96
<211> 23
<212> DNA
<213> Artificial

<220>

<223> Artificially synthesized primer sequence for RT-PCR

<400> 96
ataccaccaa tggttctgct atg 23

<210> 97
<211> 20
<212> DNA
<213> Artificial

<220>

<223> Artificially synthesized primer sequence for RT-PCR

<400> 97
ctcatctttg aagccagcag 20

<210> 98
<211> 20
<212> DNA
<213> Artificial

<220>

<223> Artificially synthesized primer sequence for RT-PCR

<400> 98
gactcacagg caggaacatc 20

<210> 99
<211> 23
<212> DNA
<213> Artificial

<220>

<223> Artificially synthesized primer sequence for RT-PCR

<400> 99
ggatagctgg ggcatttgtc tag 23

<210> 100
<211> 22
<212> DNA
<213> Artificial

<220>

<223> Artificially synthesized primer sequence for RT-PCR

<400> 100
tccataaaag agtttggcag tc 22

<210> 101
<211> 23
<212> DNA
<213> Artificial

<220>

<223> Artificially synthesized primer sequence for RT-PCR

<400> 101

gagttgtatt atgaagaggc cga 23

<210> 102
<211> 23
<212> DNA
<213> Artificial

<220>

<223> Artificially synthesized primer sequence for RT-PCR

<400> 102
atgtctcaga ctgtaagcga agg 23

<210> 103
<211> 23
<212> DNA
<213> Artificial

<220>

<223> Artificially synthesized primer sequence for RT-PCR

<400> 103
gtagatgtgg ggacaacaga gag 23

<210> 104
<211> 23
<212> DNA
<213> Artificial

<220>

<223> Artificially synthesized primer sequence for RT-PCR

<400> 104
tttaaagtca ccttaggttg ggg 23

<210> 105
<211> 23
<212> DNA
<213> Artificial

<220>

<223> Artificially synthesized primer sequence for RT-PCR

<400> 105
cacctatccc tattacctga ccc 23

<210> 106
<211> 23
<212> DNA
<213> Artificial

<220>

<223> Artificially synthesized primer sequence for RT-PCR

<400> 106
tctgagggtt tacattgacg act 23

<210> 107
<211> 24
<212> DNA
<213> Artificial

<220>

<223> Artificially synthesized primer sequence for RT-PCR

<400> 107
gagtccaggt aagtgaatct gtcc 24

<210> 108
<211> 22
<212> DNA
<213> Artificial

<220>

<223> Artificially synthesized primer sequence for RT-PCR

<400> 108
atttccaccg agacctctca tc 22

<210> 109
<211> 23
<212> DNA
<213> Artificial

<220>

<223> Artificially synthesized primer sequence for RT-PCR

<400> 109
gtctatctgt gctggaacct gag 23

<210> 110
<211> 22
<212> DNA
<213> Artificial

<220>

<223> Artificially synthesized primer sequence for RT-PCR

<400> 110
gtgtaggtga gtgctttctc ca 22

<210> 111
<211> 23
<212> DNA
<213> Artificial

<220>

<223> Artificially synthesized primer sequence for RT-PCR

<400> 111
actcccgagt aaatcataga gcc 23

<210> 112
<211> 23
<212> DNA
<213> Artificial

<220>

<223> Artificially synthesized primer sequence for RT-PCR

<400> 112
gactgtttct actccagagg ggt 23

<210> 113
<211> 22
<212> DNA
<213> Artificial

<220>

<223> Artificially synthesized primer sequence for RT-PCR

<400> 113
aaagctgatg aggacagacc ag 22

<210> 114
<211> 22
<212> DNA
<213> Artificial

<220>

<223> Artificially synthesized primer sequence for RT-PCR

<400> 114
ggcagaggca caatcatttt ag 22

<210> 115
<211> 23
<212> DNA
<213> Artificial

<220>

<223> Artificially synthesized primer sequence for RT-PCR

<400> 115
tggtgtcttt ctaccattca agg 23

<210> 116
<211> 23
<212> DNA
<213> Artificial

<220>

<223> Artificially synthesized primer sequence for RT-PCR

<400> 116
aaaaggctag tccccttcta cct 23

<210> 117
<211> 23
<212> DNA
<213> Artificial

<220>

<223> Artificially synthesized primer sequence for RT-PCR

<400> 117
cttgggtctg taacaaagca ttc 23

<210> 118
<211> 23
<212> DNA
<213> Artificial

<220>

<223> Artificially synthesized primer sequence for RT-PCR

<400> 118
aaggattatg aggaggttgg tgt 23